# EUROPEAN PATENT APPLICATION

(11) **EP 1 151 990 A1**
(43) Date of publication of application: **07.11.2001**
(21) Application number: 01119663.1
(22) Date of filing: 16.07.1997
(51) Int. Cl.: C07C 255/58, A61K 31/275, C07C 225/20, C07C 233/41, A61K 31/16, A61P 9/12

(54) **Substituted N-arylmethylamino derivatives of cyclobutene-3, 4-diones**

(30) Priority: 17.07.1996 US 678430; 17.07.1996 US 682207; 17.07.1996 US 684278; 17.07.1996 US 684279; 07.07.1997 US 889163; 07.07.1997 US 889164; 07.07.1997 US 889165; 07.07.1997 US 889166
(62) Divisional of application: 97936050.0
(71) Applicant: AMERICAN HOME PRODUCTS CORPORATION, Madison, New Jersey 07940-0874 (US)
(72) Inventor: Antane, Madelene Miyoko, Lawrenceville, New Jersey 08648 (US); Herbst, David Richard, Wayne, Pennsylvania 19087 (US); McFarlane, Geraldine Ruth, Monmouth Junction, New Jersey 08852 (US); Gundersen, Eric Gould, Plainsboro, New Jersey 08536 (US); Hirth, Bradford Hammond, Littleton, Massachusetts 01460 (US); Quagliato, Dominick Anthony, Bridgewater, New Jersey 08807 (US); Graceffa, Russell Francis, Plainsboro, New Jersey 08536 (US); Butera, John Anthony, Clarksburg, New Jersey 08510 (US); Gilbert, Adam Matthew, Valley Cottage, New York 10989 (US)
(74) Representative: Mannion, Sally Kim, Dr.

(57) **Abstract**

The compounds of the formula: 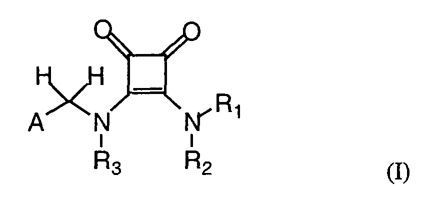 wherein R₁, R₂, R₃ and A are as defined herein are useful, via potassium channel modulation, in the treatment of disorders associated with smooth muscle contraction. Such disorders include, but are not limited to, urinary incontinence, hypertension, asthma, premature labor, irritable bowel syndrome, congestive heart failure, angina and cerebral vascular disease.

## Description

### Background of Invention

The present invention relates to novel 1, 2-diamino derivatives of cyclobutene 3,4-diones having pharmacological activity, to a process for their preparation, to pharmaceutical compositions containing them, and to their use, via potassium channel modulation, in the treatment of disorders associated with smooth muscle contraction. Such disorders include, but are not limited to, urinary incontinence, hypertension, asthma, premature labor, irritable bowel syndrome, congestive heart failure, angina and cerebral vascular disease.

Stemp et al. (EP-426379) disclose a class of amino substituted cyclobutenedione derivatives of chromans described as having blood pressure lowering activity and bronchodilatory activity . Takeno et al. (Public Patent Disclosure Bulletin No. 6-92915) report a series of diaminocyclobuten-3,4-diones Our own efforts in this area have been disclosed in the following US Patents: 5,464,867, 5,466,712, 5,403,853, 5,403,854, 5,397,790, and 5,401,753. Several series of 1-amino-2-phenylalkylamino-cyclobutene-3,4-diones are reported as H-2 receptor antagonists by Algieri et al. in US Patent 4,390,701. Several related 1-amino-2-phenoxyalkylamino derivatives are disclosed by Nohara et al. in US Patent 4,673,747. Additionally, US Patent 5,240,946 and EP-496561 disclose diaminocyclobuten-3,4-diones useful as NMDA antagonists.

The syntheses of variously substituted 1,2-diamino-cyclobutene-3,4-diones are described in the following publications: Tietze et al., Chem Ber. 1991, 124, 1215; Tietze et al., Bioconjugate Chem. 1991, 2, 148; Ehrhardt et al., Chem. Ber. 1977, 110, 2506, Neuse et al., Liebigs Ann. Chem. 1973, 619, Ried et al., Liebigs Ann. Chem. 1973, 619, Kinney et al., J. Med. Chem. 1992, 35, 4702.

### Description of The Invention

Accordingly, the present invention discloses compounds represented by formula (I): 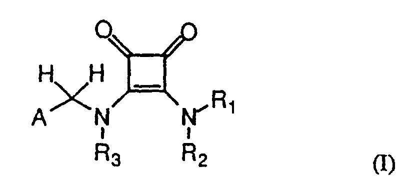 wherein:
R₁ is straight chain alkyl of 1 to 10 carbon atoms, branched chain alkyl of 3 to 10 carbon atoms, cycloalkyl of 3 to 10 carbon atoms, hydroxyalkyl of 2 to 10 carbon atoms, or fluoroalkyl of 1 to 10 carbon atoms;
R₂ and R₃ are, independently, hydrogen or an acyl substituent selected from the group consisting of formyl, alkanoyl of 2 to 7 carbon atoms, alkenoyl of 3 to 7 carbon atoms, straight chain alkoxycarbonyl of 2 to 11 carbon atoms, branched chain alkoxycarbonyl of 4 to 11 carbon atoms, cycloalkoxycarbonyl of 4 to 11 carbon atoms, alkenoxycarbonyl of 2 to 11 carbon atoms, aralkoxycarbonyl of 6 to 12 carbon atoms, alkylsulfonyl of 1 to 7 carbon atoms, aroyl of 7 to 12 carbon atoms, arylalkenoyl of 9 to 20 carbon atoms, arylsulfonyl of 6 to 12 carbon atoms, arylalkanoyl of 8 to 12 carbon atoms or arylalkylsulfonyl of 7 to 12 carbon atoms; with the proviso that when R₃ is straight chain alkoxycarbonyl of 2 to 11 carbon atoms, branched chain alkoxycarbonyl of 4 to 11 carbon atoms, cycloalkoxycarbonyl of 4 to 11 carbon atoms, alkenoxycarbonyl of 2 to 11 carbon atoms or aralkoxycarbonyl of 6 to 12 carbon atoms, R₂ must be hydrogen;
A is a substituted phenyl group of the following formula: 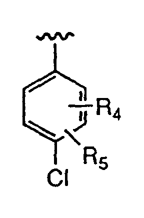 wherein:
   R₄ and R₅ are, independently, cyano, nitro, amino, alkyl of 1 to 6 carbon atoms, fluoroalkyl of 1 to 6 carbon atoms, alkoxy of 1 to 6 carbon atoms, fluoroalkoxy of 1 to 6 carbon atoms, amino, alkylamino of 1 to 6 carbon atoms, dialkylamino of 2 to 12 carbon atoms, sulfamyl, alkylsulfonamido of 1 to 6 carbon atoms, arylsulfonamido of 6 to 12 carbon atoms, carbamoyl, alkylcarbamoyl of 2 to 7 carbon atoms, dialkylcarbamoyl of 4 to 14 carbon atoms, alkylcarboxamido containing 2 to 7 carbon atoms, arylcarboxamido containing 7 to 13 carbon atoms, alkylsulfonyl of 1 to 6 carbon atoms, perfluoroalkylsulfonyl of 1 to 6 carbon atoms, arylsulfonyl of 6 to 12 carbon atoms, chloro, bromo, fluoro, iodo, 1-imidazolyl, carboxyl or hydrogen, with the proviso that R₄ and R₅ cannot both be hydrogen;
   or A is a substitued phenyl group of the following formula : 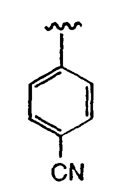 or A is a phenyl group with either two or three substituents of the following formula: 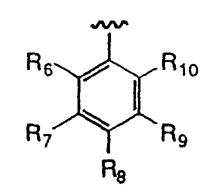 wherein:
   the positions of substitution are R₆,R₇-, R₆,R₈₋, R₆,R₉-, R₆,R₁₀-,
   R₇,R₈₋, R₇,R₉-, and R₆,R₈,R₁₀- and
   R₆ is methyl, ethyl, fluoro, chloro, methoxy or trifluoromethyl;
   R₇ is methyl, ethyl, fluoro, chloro, methoxy or trifluoromethyl;
   R₈ is methyl, fluoro, bromo, methoxy or cyano;
   R₉ is methyl, fluoro, chloro, methoxy, cyano or trifluoromethyl;
   R₁₀ is methyl, fluoro, chloro, or methoxy;
   or A is a substituted phenyl group of the following formula: 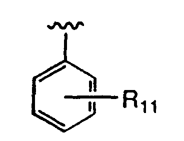 wherein:
   R₁₁ is F, perfluoroalkyl group of 1 to 10 carbon atoms or perfluoroalkoxy of 1 to 10 carbon atoms;
   or a pharmaceutically acceptable salt thereof.

When R₁ is fluoroalkyl it can be mono-, poly or per susbsituted fluoroalkyl. When R₄ and/or R₅ are fluoroalkyl or fluoroalkoxy, it can be mono-, poly or per susbsituted fluoroalkyl or fluoroalkoxy,

Preferred values of R₁ are straight chain alkyl of 1 to 10 carbon atoms, branched chain alkyl of 3 to 10 carbon atoms or fluoroalkyl of 1 to 10 carbon atoms. Particularly preferred values of R₁ are branched alkyl of 3 to 10 carbon atoms or fluoroalkyl of 1 to 10 carbon atoms.

Preferred values of R₂ and R₃ are, independently, hydrogen, alkanoyl of 2 to 7 carbon atoms, alkenoyl of 3 to 7 carbon atoms, aroyl of 7 to 12 carbon atoms, arylalkenoyl of 9 to 20 carbon atoms, straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms; with the proviso that when R₃ is straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms, R₂ must be hydrogen.

More preferred values of R₂ and R₃ are, independently, hydrogen, alkanoyl of 2 to 7 carbon atoms, alkenoyl of 3 to 7 carbon atoms, straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms; with the proviso that when R₃ is straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms, R₂ must be hydrogen.

Particularly preferred values of R₂ and R₃ are, independently, hydrogen, alkanoyl of 2 to 7 carbon atoms, alkenoyl of 3 to 7 carbon atoms, aroyl of 7 to 12 carbon atoms, or arylalkenoyl of 9 to 20 carbon atoms.

Preferred values of R₄ and R₅ are, independently, cyano, alkyl of 1 to 6 carbon atoms, fluoroalkyl of 1 to 6 carbon atoms, alkoxy of 1 to 6 carbon atoms, fluoroalkoxy of 1 to 6 carbon atoms, chloro, bromo, fluoro, iodo or hydrogen, with the proviso that R₄ and R₅ cannot both be hydrogen.

Particularly preferred values of R4 and R₅ are, independently, cyano, methyl, ethyl, trifluoromethyl, fluoroalkyl of 1 to 2 carbon atoms, methoxy, ethoxy, trifluoromethoxy, fluoroalkoxy of I to 2 carbon atoms, chloro, bromo, fluoro or hydrogen, with the proviso that R₄ and R₅ cannot both be hydrogen;.

Preferred values of R₆ to R₁₀ are where R₆ is methyl, ethyl or chloro; R₇ is methyl ethyl or chloro; R₈ is methyl, bromo or cyano; R₉ is cyano, chloro, or methyl; and R₁₀ is methyl or choro.
Preferred substitutional variations are at position combinations :
- R₆,R₈- or R₇,R₈- when R₆ is trifluoromethyl, fluoro or chloro; R₇ is fluoro or chloro; and R₈ is fluoro.
- R₆,R₉-, R₆,R₁₀- or R₇,R₉- where R₆ is methyl, fluoro or chloro; R₇ is fluoro; R₉ is fluoro or trifluoromethyl; and R₁₀ is fluoro;
- R₆,R₇-, R₆,R₈- or R₆,R₈,R₁₀- where R₆ is methyl, ethyl or chloro; R₇ is methyl or chloro; R₈ is methyl, bromo or cyano; R₁₀ is methyl or chloro.
- R₇,R₈-, R₇,R₉-, R₆,R₉-, or R₆,R₁₀-, where R₆ is methyl or chloro; R₇ is methyl, ethyl or chloro; R₈ is cyano or methyl; R₉ is cyano, chloro, or methyl; and R₁₀ is methyl or chloro;

Preferred values of R₁₁ are F, perfluoroalkyl group of 1 to 10 carbon atoms or perfluoroalkoxy of 1 to 10 carbon atoms.

A preferred aspect of this invention involves compounds of formula (I) wherein R₁ is straight chain alkyl of 1 to 10 carbon atoms, branched chain alkyl of 3 to 10 carbon atoms, fluoroalkyl of I to 10 carbon atoms; R₂ and R₃ are, independently, hydrogen, alkanoyl of 2 to 7 carbon atoms, alkenoyl of 3 to 7 carbon atoms, aroyl of 7 to 12 carbon atoms, or arylalkenoyl of 9 to 20 carbon atoms.

A more preferred aspect of this invention are those compounds of formula (I) wherein R₁ is most preferably branched alkyl of 3 to 10 carbon atoms or fluoroalkyl of 1 to 10 carbon atoms; R₂ and R₃ are, independently, hydrogen, alkanoyl of 2 to 7 carbon atoms or alkenoyl of 3 to 7 carbon atoms.

A preferred aspect of this invention involves compounds of formula (I) wherein A is 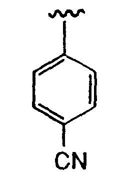 R₁ is straight chain alkyl of 1 to 10 carbon atoms, branched chain alkyl of 3 to 10 carbon atoms or fluoroalkyl of 1 to 10 carbon atoms; R₂ and R₃ are, independently, hydrogen, alkanoyl of 2 to 7 carbon atoms, alkenoyl of 3 to 7 carbon atoms, aroyl of 7 to 12 carbon atoms, arylalkenoyl of 9 to 20 carbon atoms, straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms; with the proviso that when R₃ is straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms, R₂ must be hydrogen; or a pharmaceutically acceptable salt thereof.

A particularly preferred aspect of this invention are those compounds of formula (I) wherein A is: 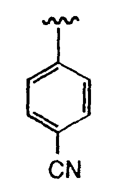 R₁ is branched alkyl of 3 to 10 carbon atoms or polyfluoroalkyl of 1 to 10 carbon atoms; R₂ and R₃ are, independently, hydrogen, alkanoyl of 2 to 7 carbon atoms, alkenoyl of 3 to 7 carbon atoms, straight chain alkoxycarbonyl of 3 or 5 carbon atoms, branched chain alkoxycarbonyl of 5 carbon atoms, alkenoxycarbonyl of 4 carbon atoms, or, aralkoxycarbonyl of 8 carbon atoms; with the proviso that when R₃ is straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms, R₂ must be hydrogen; or a pharmaceutically acceptable salt thereof.

Another preferred aspect of this invention are those compounds of formula (I) wherein A is 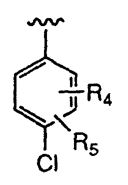
R₁ is straight chain alkyl of 1 to 10 carbon atoms, branched chain alkyl of 3 to 10 carbon atoms or fluoroalkyl of 1 to 10 carbon atoms;
R₂ and R₃ are, independently, hydrogen, alkanoyl of 2 to 7 carbon atoms, alkenoyl of 3 to 7 carbon atoms, aroyl of 7 to 12 carbon atoms, arylalkenoyl of 9 to 20 carbon atoms, straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms; with the proviso that when R₃ is straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms, R₂ must be hydrogen;
R₄ and R₅ are, independently, cyano, alkyl of 1 to 6 carbon atoms, fluoroalkyl of 1 to 6 carbon atoms, alkoxy of 1 to 6 carbon atoms, fluoroalkoxy of 1 to 6 carbon atoms, chloro, bromo, fluoro, iodo or hydrogen, with the proviso that R₄ and R₅ cannot both be hydrogen;
or a pharmaceutically acceptable salt thereof.

A particularly preferred aspect of this invention includes compounds of formula (I) wherein A is : 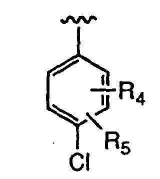
and R₁ is branched chain alkyl of 3 to 10 carbon atoms or fluoroalkyl of 1 to 10 carbon atoms;
R₂ and R₃ are, independently, hydrogen, alkanoyl of 2 to 7 carbon atoms, alkenoyl of 3 to 7 carbon atoms, straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms; with the proviso that when R₃ is straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms, R₂ must be hydrogen;
R₄ and R₅ are, independently, cyano, methyl, ethyl, trifluoromethyl, fluoroalkyl of 1 to 2 carbon atoms, methoxy, ethoxy, trifluoromethoxy, fluoroalkoxy of 1 to 2 carbon atoms, chloro, bromo, fluoro or hydrogen, with the proviso that R₄ and R₅ cannot both be hydrogen ; or a pharmaceutically acceptable salt thereof.

Another preferred aspect of this invention involves those compounds that exhibit activity at a concentration less than 30 µM in the relaxation of smooth muscle, represented by the formula (I) when A is: 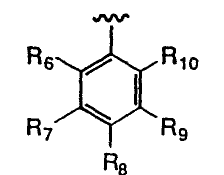 wherein R₁ is a straight chain alkyl of 1 to 10 carbon atoms, branched chain alkyl of 3 to 10 carbon atoms or fluoroalkyl of 1 to 10 carbon atoms; R₂ is hydrogen; R₃ is hydrogen, alkanoyl of 2 to 7 carbon atoms or alkenoyl of 3 to 7 carbon atoms, straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms and R₆ to R₁₀ are selected from the following preferred substitutional variations and position combinations:
- R₆,R₈- or R₇,R₈- when R₆ is trifluoromethyl, fluoro or chloro; R₇ is fluoro or chloro; and R₈ is fluoro.
- R₆,R₉-, R₆,R₁₀- or R₇,R₉- where R₆ is methyl, fluoro or chloro; R₇ is fluoro; R₉ is fluoro or trifluoromethyl; and R₁₀ is fluoro;
- R₆,R₇-, R₆,R₈- or R₆,R₈,R₁₀- where R₆ is methyl, ethyl or chloro; R₇ is methyl or chloro; R₈ is methyl, bromo or cyano; R₁₀ is methyl or chloro.
- R₇,R₈-, R₇,R₉-, R₆,R₉-, or R₆,R₁₀-, where R₆ is methyl or chloro; R₇ is methyl, ethyl or chloro; R₈ is cyano or methyl; R₉ is cyano, chloro, or methyl; and R₁₀ is methyl or chloro;

The most preferred compounds of formula I in which A is phenyl substituted with R₆ to R₁₀ are those compounds with activity at concentrations less than 10 µM in relaxation of smooth muscle, represented by formula (I) wherein R₁ is α,α-substituted branched chain alkyl of 4 to 10 carbon atoms; R₂ is hydrogen; R₃ is hydrogen, or alkanoyl of 2 to 4 carbon atoms; A is a phenyl group with either two or three substituents of the following formula: 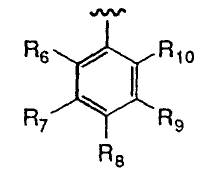 wherein the preferred substitution combinations are selected from the following:

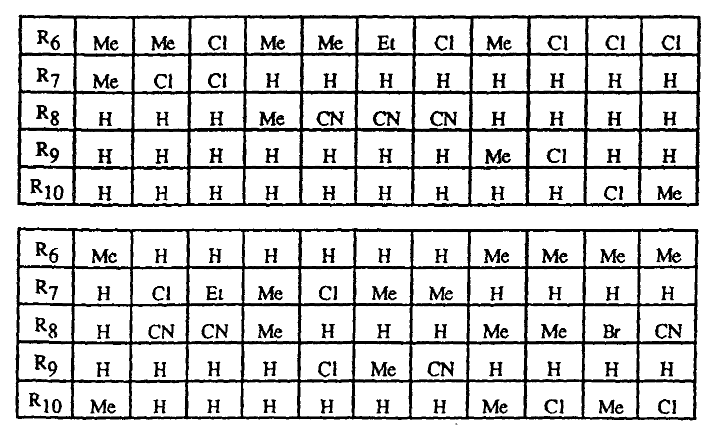

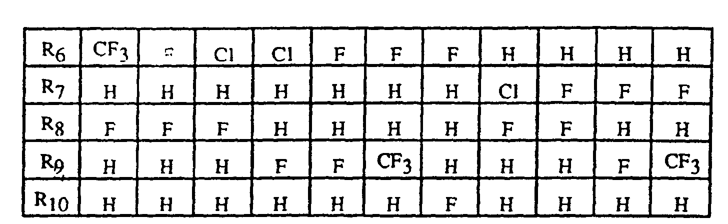

or a pharmaceutically acceptable salt thereof.

Particularly preferred compounds of the invention are :
4-{[3,4-dioxo-2-(1,2,2-trimethyl-propylamino)-cyclobut-1-enylamino]-methyl}-benzonitrile or a pharmaceutically acceptable salt thereof;
4-{[2-(1,2-dimethyl-propylamino)-3,4-dioxo-cyclobut-1-enylamino]-methyl}-benzonitrile or a pharmaceutically acceptable salt thereof;
4-[(2-isopropylamino-3,4-dioxo-cyclobut-1-enylamino)-methyl]-benzonitrile or a pharmaceutically acceptable salt thereof;
4-{[2-(1-ethyl-propylamino)-3,4-dioxo-cyclobut-1-enylamino]-methyl}-benzonitrile or a pharmaceutically acceptable salt thereof;
4-[(2-tert-butylamino-3,4-dioxo-cyclobut-1-enylamino)-methyl]-benzonitrile or a pharmaceutically acceptable salt thereof;
4-[(3,4-dioxo-2-propylamino-cyclobut-1-enylamino)-methyl]-benzonitrile or a pharmaceutically acceptable salt thereof;
4-{[2-(2-hydroxy-1,1-dimethyl-ethylamino)-3,4-dioxo-cyclobut-1-enylamino]-methyl}-benzonitrile or a pharmaceutically acceptable salt thereof;
4-[(2-butylamino-3,4-dioxo-cyclobut- 1-enylamino)-methyl]-benzonitrile or a pharmaceutically acceptable salt thereof;
4-[(2-cyclopentylamino-3,4-dioxo-cyclobut-1-enylamino)-methyl]-benzonitrile or a pharmaceutically acceptable salt thereof;
4-[(2-isobutylamino-3,4-dioxo-cyclobut-1-enylamino)-methyl]-benzonitrile or a pharmaceutically acceptable salt thereof;
4-[(2-methylamino-3,4-dioxo-cyclobut-1-enylamino)-methyl]-benzonitrile or a pharmaceutically acceptable salt thereof;
(S)-4-[2-sec-butylamino-3,4-dioxo-cyclobut-1-enylamino)-methyl]-benzonitrile or a pharmaceutically acceptable salt thereof;
(R)-4-[(2-sec-butylamino-3,4-dioxo-cyclobut-1-enylamino)-methyl]-benzonitrile or a pharmaceutically acceptable salt thereof;
4-([3,4-dioxo-2-(2,2,3,3,3-pentafluoro-propylamino)-cyclobut-1-enylamino]-methyl}-benzonitrile or a pharmaceutically acceptable salt thereof;
N-[2-(4-cyano- benzylamino)-3,4-dioxo-cyclobut-1-enyl]-N-(1,2,2-trimethyl-propyl)-butyramide or a pharmaceutically acceptable salt thereof;
N-(4-cyano-benzyl)-N-[3,4-dioxo-2-(1,2,2-trimethyl-propylamino)-cyclobut-1-enyl]-but-2-enamide or a pharmaceutically acceptable salt thereof;
pentanoic acid (4-cyano-benzyl)-[3,4-dioxo-2-(1,2,2-trimethyl-propylamino)-cyclobut-1-enyl)-amide or a pharmaceutically acceptable salt thereof;
hexanoic acid (4-cyano-benzyl)-[3,4-dioxo-2-(1,2,2-trimethyl-propylamino)-cyclobut-1-enyl]-amide or a pharmaceutically acceptable salt thereof;
3-(2,4-dichlorobenzylamino)-4-(1,2,2-trimethylpropylamino)-cyclobut-3-ene-1,2-dione or a pharmaceutically acceptable salt thereof;
3-(2,4-dichloro-6-methyl-benzylamino)-4-(1,2,2-trimethyl-propylamino)-cyclobut-3-ene-1,2-dione or a pharmaceutically acceptable salt thereof;
3-(2,4-dichlorobenzylamino)-4-(1,1-dimethylpropylamino)-cyclobut-3-ene-1,2-dione or a pharmaceutically acceptable salt thereof;
N-(2,4-dichlorobenzyl)-N-[2-(1,1-dimethyl-propylamino)-3,4-dioxo-cyclobut-1-enyl)-acetamide or a pharmaceutically acceptable salt thereof;
N-(2,4-dichlorobenzyl)-N-[2-(1,1-dimethyl-propylamino)-3,4-dioxo-cyclobut-1-enyl]-butyramide or a pharmaceutically acceptable salt thereof;
3-(2,4-dichloro-6-methyl-benzylamino)-4-(1,1-dimethyl-propylamino)-cyclobut-3-ene-1,2-dione or a pharmaceutically acceptable salt thereof;
3-(t-butylamino)-4-(2,4-dichlorobenzylamino)-cyclobut-3-ene-1,2-dione or a pharmaceutically acceptable salt thereof;
3-tert-butylamino-4-(2,4-dichloro-6-methyl-benzylamino)-cyclobut-3-ene-1,2-dione or a pharmaceutically acceptable salt thereof;
3-(3,4-dichlorobenzylamino)-4-(1-ethyl-propylamino)-cyclobut-3-ene-1,2-dione or a pharmaceutically acceptable salt thereof;
3-(2,4-dichlorobenzylamino)-4-(1-ethyl-propylamino)-cyclobut-3-ene-1,2-dione or a pharmaceutically acceptable salt thereof;
3-(2,4-dichloro-6-methyl-benzylamino)-4-(2,2,3,3,3-pentafluoro-propylamino)-cyclobut-3-ene-1,2-dione or a pharmaceutically acceptable salt thereof;
N-(2,4-dichloro-6-methyl-benzyl)-N-(2-(1,1-dimethyl-propylamino)-3,4-dioxo-cyclobut-1-enyl]-butyramide or a pharmaceutically acceptable salt thereof;
N-(2,4-dichloro-6-methyl-benzyl)-N-[2-(1,1-dimethyl-propylamino)-3,4-dioxo-cyclobut-1-enyl]-acetamide or a pharmaceutically acceptable salt thereof;
N-(tert-butyl)-N-[2-(2,4-dichloro-6-methyl-benzylamino)-3,4-dioxo-cyclobut-1-enyl]-propionamide or a pharmaceutically acceptable salt thereof;
3-(2,4-dichloro-6-methyl-benzylamino)-4-(1,2-dimethyl-2-fluoro-propylamino)-cyclobut-3-ene-1,2-dione or a pharmaceutically acceptable salt thereof;
3-(2,4-dichlorobenzylamino)-4-(2-hydroxy-1,1-dimethylethylamino)-cyclobut-3-ene-1,2-dione or a pharmaceutically acceptable salt thereof;
(R)-3-(2,4-dichlorobenzylamino)-4-(1,2,2-trimethylpropylamino)-cyclobut-3-ene-1,2-dione or a pharmaceutically acceptable salt thereof;
3-tert-butylamino-4-(3,4-dichlorobenzylamino)-cyclobut-3-ene-1,2-dione or a pharmaceutically acceptable salt thereof;
3-(3,4-dichlorobenzylamino)-4-(1,1-dimethylpropylamino)-cyclobut-3-ene-1,2-dione or a pharmaceutically acceptable salt thereof;
(2,4-Dichloro-6-methyl-benzyl)-[2-(1,1-dimethyl-propylamino)-3,4-dioxo-cyclobut-1-enyl]-carbamic acid tert-butyl ester or a pharmaceutically acceptable salt thereof;
4-[(2-tert-butylamino-3,4-dioxo-cyclobut-1-enylamino)-methyl)-3-chloro-benzonitrile or a pharmaceutically acceptable salt thereof;
3-(2,6-dichloro-benzylamino)-4-(1,1-dimethyl-propylamino)-cyclobut-3-ene-1,2-dione or a pharmaceutically acceptable salt thereof;
3-chloro-4-{[3,4-dioxo-2-(1,2,2-trimethylpropylamino)-cyclobut-1-enylamino]-methyl}-benzonitrile or a pharmaceutically acceptable salt thereof;
3-{[2-(1,1-dimethyl-propylamino)-3,4-dioxocyclobut-1-enylamino]-methyl}-5-methyl-benzonitrile or a pharmaceutically acceptable salt thereof;
3-tert-butylamino-4-(2,4-dimethyl-benzylamino)-cyclobut-3-ene-1,2-dione or a pharmaceutically acceptable salt thereof;
4-[(2-tert-butylamino-3,4-dioxo-cyclobut-1-enylamino)-methyl]-3-ethyl-benzonitrile or a phamlaceutically acceptable salt thereof;
(R)-3-chloro-4-{[3,4-dioxo-2-(1,2,2-trimethyl-propylamino)-cyclobut-1-enylamino]-methyl}-benzonitrile or a pharmaceutically acceptable salt thereof;
3-chloro-4-{[2-(1,1-dimethyl-propylamino)-3,4-dioxo-cyclobut-1-enylamino]-methyl}-5-methyl-benzonitrile or a pharmaceutically acceptable salt thereof;
3-(4-bromo-2,6-dimethyl-benzylamino)-4-tert-butylamino-cyclobut-3-ene-1,2-dione or a pharmaceutically acceptable salt thereof;
3-tert-butylamino-4-(2-chloro-4,6-dimethyl-benzylamino)-cyclobut-3-ene-1,2-dione or a pharmaceutically acceptable salt thereof;
3-(2-chloro-4,6-dimethyl-benzylamino)-4-(1,1-dimethyl-propylamino)-cyclobut-3-ene-1,2-dione or a pharmaceutically acceptable salt thereof;
2-chloro-4-{[2-(1,1-dimethyl-propylamino]-3,4-dioxo-cyclobut-1-enylamino)-methyl}- benzonitrile or a pharmaceutically acceptable salt thereof;
3-(4-bromo-2-ethyl-benzylamino)-4-tert-butylamino-cyclobut-3-ene-1,2-dione or a pharmaceutically acceptable salt thereof;
4-[(1,1-dimethyl-propylamino-3,4-dioxo-cyclobut-1-enylamino)-methyl]-3-ethyl-benzonitrile or a pharmaceutically acceptable salt thereof;
3-(2,6-dimethyl-benzylamino)-4-(1,1-dimethyl-propylamino)-cyclobut-3-ene-1,2-dione or a pharmaceutically acceptable salt thereof;
3-chloro-4-{[2-(1,1-dimethyl-propylamino)-3,4-dioxo-cyclobut-1-enylamino]-methyl}-benzonitrile or a pharmaceutically acceptable salt thereof;
3-(2,4-dimethyl-benzylamino)-4-(1,1-dimethyl-propylamino)-cyclobut-3-ene-1,2-dione or a pharmaceutically acceptable salt thereof;
3-(1,1-dimethyl-propylamino)-4-(2,4,6-trimethyl-benzylami no)-cyclobut-3-ene-1,2-dione or a pharmaceutically acceptable salt thereof;
3-tert-butylamino-4-(2,5-dichloro-benzylamino)-cyclobut-3-ene-1,2-dione or a pharmaceutically acceptable salt thereof;
3-(2,5-dichloro-benzylamino)-4-(1,1-dimethyl-propylamino)-cyclobut-3-ene-1,2-dione or a pharmaceutically acceptable salt thereof;
3-(3-chloro-2-methyl-benzylamino)-4-(1,1-dimethyl-propylamino)-cyclobut-3-ene-1,2-dione or a pharmaceutically acceptable salt thereof;
3-(2,4-difluoro-benzylamino)-4-(1,1-dimethyl-propylamino)-cyclobut-3-ene-1,2-dione or a pharmaceutically acceptable salt thereof;
3-(2-chloro-5-fluoro-benzylamino)-4-(1,2,2-trimethyl-propylamino)-cyclobut-3-ene-1,2-dione or a pharmaceutically acceptable salt thereof;
3-(2,5-difluoro-benzylamino)-4-(1,2,2-trimethyl-propylamino)-cyclobut-3-ene-1,2-dione or a pharmaceutically acceptable salt thereof;
3-(3,5-difluoro-benzylamino)-4-(1,1-dimethyl-propylamino)-cyclobut-3-ene-1,2-dione or a pharmaceutically acceptable salt thereof;
4-[(2-tert-butylamino-3,4-dioxo-cyclobut-1-enylamino)-methyl]-3-chloro-benzonitrile or a pharmaceutically acceptable salt thereof;
3-(2,6-dichloro-benzylamino)-4-(1,1-dimethyl-propylamino)-cyclobut-3-ene-1,2-dione or a pharmaceutically acceptable salt thereof;
3-chloro-4-{[3,4-dioxo-2-(1,2,2-trimethylpropylamino)-cyclobut-1-enylamino]-methyl}-benzonitrile or a pharmaceutically acceptable salt thereof;
3-{[2-(1,1-dimethyl-propylamino)-3,4-dioxo-cyclobut-1-enylamino]-methyl}-5-methyl-benzonitrile or a pharmaceutically acceptable salt thereof;
3-tert-butylamino-4-(2,4-dimethyl-benzylamino)-cyclobut-3-ene-1,2-dione or a pharmaceutically acceptable salt thereof;
3-tert-butylamino-4-(2,4,6-trimethyl-benzylamino)-cyclobut-3-ene-1,2-dione or a pharmaceutically acceptable salt thereof;
3-tert-butylamino-4-(2,6-dichloro-benzylamino)-cyclobut-3-ene-1,2-dione or a pharmaceutically acceptable salt thereof;
3-{[3,4-dioxo-2-(1,2,2-trimethyl-propylamino)-cyclobut-1-enylamino]-methyl}-5-methyl-benzonitrile or a pharmaceutically acceptable salt thereof;
3-{[2-(1-ethyl-propylamino)-3,4-dioxo-cyclobut-1-enylamino]-methyl}-5-methyl-benzonitrile or a pharmaceutically acceptable salt thereof;
3-(3,4-dimethoxy-benzylamino)-4-(1-ethyl-propylamino)-cyclobut-3-ene-1,2-dione or a pharmaceutically acceptable salt thereof;
3-tert-butylamino-4-(2,4,6-trimethoxy-benzylamino)-cyclobut-3-ene-1,2-dione or a pharmaceutically acceptable salt thereof;
3-[(2-tert-butylamino-3,4-dioxo-cyclobut-1-enylamino)-methyl]-5-methyl-benzonitrile or a pharmaceutically acceptable salt thereof;
3-tert-butylamino-4-(2,4-dimethoxy-benzylamino)-cyclobut-3-ene- 1,2-dione or a pharmaceutically acceptable salt thereof;
3-tert-butylamino-4-(2,4-difluoro-benzylamino)-cyclobut-3-ene-1,2-dione or a pharmaceutically acceptable salt thereof;
3-tert-butylamino-4-(2,6-difluoro-benzylamino)-cyclobut-3-ene-1,2-dione or a pharmaceutically acceptable salt thereof;
3-tert-butylamino-4-(2,3-dimethoxy-benzylamino)-cyclobut-3-ene-1,2-dione or a pharmaceutically acceptable salt thereof;
3-tert-butylamino-4-(2,5-difluoro-benzylamino)-cyclobut-3-ene-1,2-dione or a pharmaceutically acceptable salt thereof;
3-tert-butylamino-4-(3,5-dimethoxy-benzylamino)-cyclobut-3-ene-1,2-dione or a pharmaceutically acceptable salt thereof;
3-tert-butylamino-4-(2,3-dimethyl-benzylamino)-cyclobut-3-ene-1,2-dione or a pharmaceutically acceptable salt thereof;
4-[(2-butylamino-3,4-dioxo-cyclobut-1-enylamino)-methyl]-3-ethyl-benzonitrile or a pharmaceutically acceptable salt thereof;
3-ethyl-4-[(2-isopropylamino-3,4-dioxo-cyclobut-1-enylamino)-methyl-benzonitrile or a pharmaceutically acceptable salt thereof;
3-ethyl-4-([(2-(1-ethyl-propylamino)-3,4-dioxo-cyclobut-1-enylamino]-methyl}-benzonitrile or a pharmaceutically acceptable salt thereof;
4-[(2-tert-butylamino-3,4-dioxo-cyclobut-1-enylamino)-methyl]-3-ethyl-benzonitrile or a pharmaceutically acceptable salt thereof;
4-{[3,4-dioxo-2-(1,2,2-trimethyl-propylamino)-cyclobut-1-enylamino]-methyl}-3-ethyl-benzonitrile or a pharmaceutically acceptable salt thereof;
3-(2-chloro-6-methyl-benzylamino)-4-(1,2,2-trimethyl-propylamino)-cyclobut-3-ene-1,2-dione or a pharmaceutically acceptable salt thereof;
(R)-3-chloro-4-{[3,4-dioxo-2-(1,2,2-trimethyl-propylamino)-cyclobut-1-enylamino]-methyl}-benzonitrile or a pharmaceutically acceptable salt thereof;
3-tert-butylamino-4-(2-chloro-6-methyl-benzylamino)-cyclobut-3-ene-1,2-dione or a pharmaceutically acceptable salt thereof;
3-(2-chloro-6-methyl-benzylamino)-4-(1,1-dimethyl-propylamino)-cyclobut-3-ene-1,2-dione or a pharmaceutically acceptable salt thereof;
3-chloro-4-{[2-(1,1-dimethyl-propylamino)-3,4-dioxo-cyclobut-1-enylamino]-methyl}-5-methyl-benzonitrile or a pharmaceutically acceptable salt thereof;
3-chloro-4-{[3,4-dioxo-2-(1,2,2-trimethyl-propylamino)-cyclobut-1-enylamino]-methyl }-5-methyl-benzonitrile or a pharmaceutically acceptable salt thereof;
3-(4-bromo-2,6-dimethyl-benzylamino)-4-(1,2,2-trimethyl-propylarnino)-cyclobut-3-ene-1,2-dione or a pharmaceutically acceptable salt thereof;
3-(4-bromo-2,6-dimethyl-benzylamino)-4-tert-butylamino-cyclobut-3-ene-1,2-dione or a pharmaceutically acceptable salt thereof;
3-(4-bromo-2,6-dimethyl-benzylamino)-4-(1,1-dimethyl-propylamino)-cyclobut-3-ene-1,2-dione or a pharmaceutically acceptable salt thereof;
3-(2-chloro-4,6-dimethyl-benzylamino)-4-(1,2,2-trimethyl-propylamino)-cyclobut-3-ene-1,2-dione or a pharmaceutically acceptable salt thereof;
3-tert-butylamino-4-(2-chloro-4,6-dimethyl-benzylamino)-cyclobut-3-ene-1,2-dione or a pharmaceutically acceptable salt thereof;
3-(2-chloro-4,6-dimethyl-benzylamino)-4-(1,1-dimethyl-propylamino)-cyclobut-3-ene-1,2-dione or a pharmaceutically acceptable salt thereof;
3-(2-chloro-4,6-dimethyl-benzylamino)-4-(2,2,3,33-pentafluoro-propylamino)-cyclobut-3-ene-1,2-dione or a pharmaceutically acceptable salt thereof;
2-chloro-4-{[3,4-dioxo-2-(1,2,2-trimethyl-propylamino)-cyclobut-1-enylamino]-methyl}-benzonitrile or a pharmaceutically acceptable salt thereof;
4-[(2-tert-butylamino-3,4-dioxo-cyclobut-1-enylamino)-methyl]-2-chloro-benzonitrile or a pharmaceutically acceptable salt thereof;
2-chloro-4-{[2-(1,1-dimethyl-propylamino)-3,4-dioxo-cyclobut-1-enylamino]-methyl}-benzonitrile or a pharmaceutically acceptable salt thereof;
4-{([3,4-dioxo-2-(1,2,2-trimethyl-propylamino)-cyclobut-1-enylamino]-methyl}-2-ethyl-benzonitrile or a pharmaceutically acceptable salt thereof;
4-[(2-tert-butylamino-3,4-dioxo-cyclobut-1-enylamino)-methyl]-2-ethyl-benzonitrile or a pharmaceutically acceptable salt thereof;
3-(4-bromo-2-ethyl-benzylamino)-4-(1,2,2-trimethyl-propylamino)-cyclobut-3-ene-1,2-dione or a pharmaceutically acceptable salt thereof;
3-(4-bromo-2-ethyl-benzylamino)-4-tert-butylamino-cyclobut-3-ene-1,2-dione or a pharmaceutically acceptable salt thereof;
3-(4-bromo-2-ethyl-benzylamino)-4-(1,1-dimethyl-propylamino)-cyclobut-3-ene-1,2-dione or a pharmaceutically acceptable salt thereof;
4-[(1,1-dimethyl-propylamino-3,4-dioxo-cyclobut-1-enylamino)-methyl]-3-ethyl-benzonitrile or a pharmaceutically acceptable salt thereof;
3-(2,6-dimethyl-benzylamino)-4-(1,2,2-trimethyl-propylamino)-cyclobut-3-ene-1,2-dione or a pharmaceutically acceptable salt thereof;
3-tert-butylamino-4-(2,6-dimethyl-benzylamino)-cyclobut-3-ene-1,2-dione or a pharmaceutically acceptable salt thereof;
3-(2,6-dimethyl-benzylamino)-4-(1,1-dimethyl-propylamino)-cyclobut-3-ene-1,2-dione or a pharmaceutically acceptable salt thereof;
3-(2,6-dimethyl-benzylamino)-4-(2,2,3,3,3-pentafluoro-propylamino)-cyclobut-3-ene-1,2-dione or a pharmaceutically acceptable salt thereof;
4-{(3,4-dioxo-2-(1,2,2-trimethyl-propylamino)-cyclobut-1-enylamino]-methyl}-3-methyl-benzonitrile or a pharmaceutically acceptable salt thereof;
4-{[3,4-dioxo-2-(1,2,2-trimethyl-propylamino)-cyclobut-1-enylamino]-methyl}-3-methoxy-benzonitrile or a pharmaceutically acceptable salt thereof;
3-(2-methoxy-6-methyl-benzylamino)-4-(1,2,2-trimethyl-propylamino)-cyclobut-3-ene-1,2-dione or a pharmaceutically acceptable salt thereof;
3-tert-butylamino-4-(2-ethyl-6-methyl-benzylamino)-cyclobut-3-ene-1,2-dione or a pharmaceutically acceptable salt thereof;
3-tert-butylamino-4-(4-fluoro-2-trifluoromethyl-benzylamino)-cyclobut-3-ene-1,2-dione or a pharmaceutically acceptable salt thereof;
3-tert-butylamino-4-(2-chloro-4-fluoro-benzylamino)-cyclobut-3-ene-1,2-dione or a pharmaceutically acceptable salt thereof;
3-(4-fluoro-2-trifluoromethyl-benzylamino)-4-(1,2,2-trimethyl-propylamino)-cyclobut-3-ene-1,2-dione or a pharmaceutically acceptable salt thereof;
3-(2,4-difluoro-benzylamino)-4-(1,1-dimethyl-propylamino)-cyclobut-3-ene-1,2-dione or a pharmaceutically acceptable salt thereof;
3-(2-chloro-4-fluoro-benzylamino)-4-(1,1-dimethyl-propylamino)-cyclobut-3-ene-1,2-dione or a pharmaceutically acceptable salt thereof;
3-tert-butylamino-4-(2-fluoro-5-trifluoromethyl-benzylamino)-cyclobut-3-ene-1,2-dione or a pharmaceutically acceptable salt thereof;
3-(2-chloro-5-fluoro-benzylamino)-4-(1,2,2-trimethyl-propylamino)-cyclobut-3-ene-1,2-dione or a pharmaceutically acceptable salt thereof;
3-(2,5-difluoro-benzylamino)-4-(1,2,2-trimethyl-propylamino)-cyclobut-3-ene-1,2-dione or a pharmaceutically acceptable salt thereof;
3-tert-butylamino-4-(3-chloro-4-fluoro-benzylamino)-cyclobut-3-ene-1,2-dione or a pharmaceutically acceptable salt thereof;
3-tert-butylamino-4-(3,4-difluoro-benzylamino)-cyclobut-3-ene-1,2-dione or a pharmaceutically acceptable salt thereof;
3-(3-chloro-4-fluoro-benzylarnino)-4-(1,2,2-trimethyl-propylamino)-cyclobut-3-ene-1,2-dione or a pharmaceutically acceptable salt thereof;
3-(3,4-difluoro-benzylamino)-4-(1,1-dimethyl-propylamino)-cyclobut-3-ene-1,2-dione or a pharmaceutically acceptable salt thereof;
3-tert-butylamino-4-(3,5-difluoro-benzylamino)-cyclobut-3-ene-1,2-dione or a pharmaceutically acceptable salt thereof;
3-tert-butylamino-4-(3-fluoro-5-trifluoromethyl-benzylamino)-cyclobut-3-ene-1,2-dione or a pharmaceutically acceptable salt thereof;
3-(3,5-bis-trifluoromethyl-benzylamino)-4-tert-butylamino-cyclobut-3-ene-1,2-dione or a pharmaceutically acceptable salt thereof;
3-(3,5-difluoro-benzylamino)-4-(1,1-dimethyl-propylamino)-cyclobut-3-ene-1,2-dione or a pharmaceutically acceptable salt thereof;
3-chloro-4-{[2-(2-fluoro-1,2-dimethyl-propylamino)-3,4-dioxo-cyclobut-1-enylamino]-methyl}-benzonitrile or a pharmaceutically acceptable salt thereof;
3-chloro-4-{[2-(1,1-dimethyl-propylamino)-3,4-dioxo-cyclobut-1-enylamino]-methyl}-benzonitrile or a pharmaceutically acceptable salt thereof;
N-(2-chloro-4-cyano-benzyl)-N-[2-(1,1-dimethyl-propylamino)-3,4-dioxo-cyclobut-1-enyl)-acetamide or a pharmaceutically acceptable salt thereof;
N-(2-chloro-4-cyano-benzyl)-N-[2-(1,1-dimethyl-propylamino)-3,4-dioxo-cyclobut-1-enyl]-butyramide or a pharmaceutically acceptable salt thereof;
3-(2,4-dimethyl-benzylamino)-4-(1,1-dimethyl-propylamino)-cyclobut-3-ene-1,2-dione or a pharmaceutically acceptable salt thereof;
3-(2,3-dimethyl-benzylamino)-4-(1,1-dimethyl-propylamino)-cyclobut-3-ene-1,2-dione or a pharmaceutically acceptable salt thereof;
3-(1,1-dimethyl-propylamino)-4-(2,4,6-trimethyl-benzylamino)-cyclobut-3-ene-1,2-dione or a pharmaceutically acceptable salt thereof;
3-tert-butylamino-4-(2,5-dichloro-benzylamino)-cyclobut-3-ene-1,2-dione or a pharmaceutically acceptable salt thereof;
3-(2,5-dichloro-benzylamino)-4-(1,1-dimethyl-propylamino)-cyclobut-3-ene-1,2-dione or a pharmaceutically acceptable salt thereof;
3-tert-butylamino-4-(3,4-dimethyl-benzylamino)-cyclobut-3-ene-1,2-dione or a pharmaceutically acceptable salt thereof;
3-(3,4-dimethyl-benzylamino)-4-(1,1-dimethyl-proylamino)-cyclobut-3-ene-1,2-dione or a pharmaceutically acceptable salt thereof;
3-tert-butylamino-4-(3,5-dimethyl-benzylamino)-cyclobut-3-ene-1,2-dione or a pharmaceutically acceptable salt thereof;
3-(3,5-dimethyl-benzylamino)-4-(1,1-dimethyl-propylamino)-cyclobut-3-ene-1,2-dione or a pharmaceutically acceptable salt thereof;
3-(3,5-dichloro-benzylamino)-4-(1,1-dimethyl-propylamino)-cyclobut-3-ene-1,2-dione or a pharmaceutically acceptable salt thereof;
3-tert-butylamino-4-(3,5-dichloro-benzylamino)-cyclobut-3-ene-1,2-dione or a pharmaceutically acceptable salt thereof;
3-tert-butylamino-4-(3-chloro-4-methyl-benzylamino)-cyclobut-3-ene-1,2-dione or a pharmaceutically acceptable salt thereof;
3-(3-chloro-4-methyl-benzylamino)-4-(1,1-dimethyl-propylamino)-cyclobut-3-ene-1,2-dione or a pharmaceutically acceptable salt thereof;
3-chloro-4-{[2-(2-hydroxy-1,1-dimethyl-ethylamino)-3,4-dioxo-cyclobut-1-enylamino]-methyl}-benzonitrile or a pharmaceutically acceptable salt thereof;
3-(2,5-dimethyl-benzylamino)-4-(1,1-dimethyl-propylamino)-cyclobut-3-ene-1,2-dione or a pharmaceutically acceptable salt thereof;
3-tert-butylamino-4-(2,5-dimethyl-benzylamino)-cyclobut-3-ene-1,2-dione or a pharmaceutically acceptable salt thereof;
3-tert-butylamino-4-(3-chloro-2-methyl-benzylamino)-cyclobut-3-ene-1,2-dione or a pharmaceutically acceptable salt thereof;
3-(3-chloro-2-methyl-benzylamino)-4-(1,1-dimethyl-propylamino-cyclobut-3-ene-1,2-dione or a pharmaceutically acceptable salt thereof;
N-(2-tert-butylamino-3,4-dioxo-cyclobut-1-enyl)-N-(2-chloro-4-cyano-benzyl)-acetamide or a pharmaceutically acceptable salt thereof;
3-(2,3-dichloro-benzylamino)-4-(1,1-dimethyl-propylamino)-cyclobut-3-ene-1,2-dione or a pharmaceutically acceptable salt thereof;
3-(t-butylamino)-4-(2,3-dichloro-benzylamino)-cyclobut-3-ene-1,2-dione or a pharmaceutically acceptable salt thereof;
N-(2-tert-butylamino-3,4-dioxo-cyclobut-1-enyl)-N-(2-chloro-4-cyano-benzyl)-butyramide or a pharmaceutically acceptable salt thereof;
3-(4-fluoro-benzylamino)-4-(1,2,2-trimethyl-propylamino)-cyclobut-3-ene-1,2-dione or a pharmaceutically acceptable salt thereof;
3-(4-trifluoromethyl-benzylamino)-4-(1,2,2-trimethyl-propylamino)-cyclobut-3-ene-1,2-dione or a pharmaceutically acceptable salt thereof;
3-tert-butylamino-4-(4-trifluoromethoxy-benzylamino)-cyclobut-3-ene-1,2-dione or a pharmaceutically acceptable salt thereof;
3-tert-butylamino-4-(4-trifluoromethyl-benzylamino)-cyclobut-3-ene-1,2-dione or a pharmaceutically acceptable salt thereof;
3-tert-butylamino-4-(4-fluoro-benzylamino)-cyclobut-3-ene-1,2-dione or a pharmaceutically acceptable salt thereof;
3-(4-trifluoromethoxy-benzylamino)-4-(1,2,2-trimethyl-propylamino)-cyclobut-3-ene-1,2-dione or a pharmaceutically acceptable salt thereof;
3-(1,1-dimethyl-propylamino)-4-(4-fluoro-benzylamino)-cyclobut-3-ene-1,2-dione or a pharmaceutically acceptable salt thereof;
3-tert-butylamino-4-(3-fluorobenzyl-amino)-cyclobut-3-ene-1,2-dione or a pharmaceutically acceptable salt thereof;
3-tert-butylamino-4-(2-fluoro-benzylamino)-cyclobut-3-ene-1,2-dione or a pharmaceutically acceptable salt thereof;
3-tert-butylamino-4-(3-trifluoromethyl-benzylamino)-cyclobut-3-ene-1,2-dione or a pharmaceutically acceptable salt thereof;
3-tert-butylamino-4-(2-trifluoromethyl-benzylamino)-cyclobut-3-ene-1,2-dione or a pharmaceutically acceptable salt thereof;
3-tert-butylamino-4-(3-trifluoromethoxy-benzylamino)-cyclobut-3-ene-1,2-dione or a pharmaceutically acceptable salt thereof;
3-(3-trifluoromethyl-benzylamino)-4-(1,2,2-trimethyl-propylamino) cyclobut-3-ene-1,2-dione or a pharmaceutically acceptable salt thereof;
3-(2-trifluoromethyl-benzylamino)-4-(1,2,2-trimethyl-propylamino) cyclobut-3-ene-1,2-dione or a pharmaceutically acceptable salt thereof;
3-(2-fluoro-benzylamino)-4-(1,2,2-trimethyl-propylamino) cyclobut-3-ene-1,2-dione or a pharmaceutically acceptable salt thereof;
3-(3-trifluoromethoxy-benzylamino)-4-(1,2,2-trimethyl-propylamino)-cyclobut-3-ene-1,2-dione or a pharmaceutically acceptable salt thereof.

It is understood that the definition of the compounds of formula (I), when R₁, R₂, R₃, R₄, or R₅ contain asymmetric carbons, encompass all possible stereoisomers and mixtures thereof which possess the activity discussed below. In particular, it encompasses racemic modifications and any optical isomers which possess the indicated activity. Optical isomers may be obtained in pure form by standard separation techniques. The pharmaceutically acceptable salts are those derived from such organic and inorganic acids as: lactic, citric, acetic, tartaric, succinic, maleic, malonic, hydrochloric, hydrobromic, phosphoric, nitric, sulfuric, methanesulfonic, and similarly known acceptable acids

The present invention also provides a process for the preparation of a compound of formula (I). More particularly, the compounds of formula (I) may be prepared by reacting a compound of formula (II): 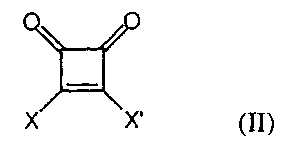 wherein X and X' is a suitably designed leaving group such as methoxy, ethoxy, butoxy, isopropoxy, halogeno, or a similar leaving group, with a compound of formula (III):

A₁-CH₂NH₂ (III)

wherein A₁ is A, as defined hereinbefore or a group of atoms convertible thereto, followed by treatment with a compound of formula (IV): 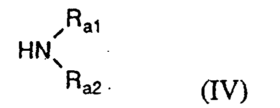 wherein Rₐ₁ and Rₐ₂ are R₁ and R₂, respectively, as defined hereinbefore or a group of atoms convertible thereto in a solvent such as ethanol, acetonitrile or the appropriate amine (IV) at elevated temperatures or room temperature. Dichloromethane can be used as a cosolvent. The order of addition of compound of formula (III) and compound of formula (IV) to compound of formula (II) may be reversed. Furthermore reaction of the sodium, potassium, or lithium salt of compound of formula (I) with the appropriate anhydride in tetrahydrofuran and/or N,N-dimethylformamide allows for the attachment of the acyl groups represented by R₃. Reaction of the sodium, potassium, or lithium salt of compound of formula (II), where X is a leaving group such as methoxy, ethoxy, butoxy, isopropoxy, or similar leaving group and X' is NHR₁, with the appropriate anhydride in dichloromethane, tetrahydrofuran and/or N,N-dimethylformamide or any other suitable solvent, followed by treatment with a compound of formula (III) as defined above in a solvent such as acetonitrile at room temperature allows for the attachment of the acyl groups represented by R₂.

Furthermore reaction of compound of formula (I) with the appropriate anhydride in pyridine with or without protection of the benzyl nitrogen allows for the attachment of R₂ and R₃.

As mentioned previously, the compounds of formula (I) have been found to relax smooth muscle. They are therefore useful in the treatment of disorders associated with smooth muscle contraction, disorders involving excessive smooth muscle contraction of the urinary tract (such as incontinence), or of the gastro-intestinal tract (such as irritable bowel syndrome), asthma and hair loss. Furthermore, the compounds of formula (I) are active as potassium channel activators which render them useful for treatment of peripheral vascular disease, hypertension, congestive heart failure, stroke, anxiety, cerebral anoxia and other neurodegenerative disorders. Thus, the present invention provides a method of treating smooth muscle disorders in mammals including man, which comprises administering to the afflicted mammal an effective amount of a compound or a pharmaceutical composition of the invention.

The present invention accordingly provides a pharmaceutical composition which comprises a compound of this invention in combination or association with a pharmaceutically acceptable carrier. In particular, the present invention provides a pharmaceutical composition which comprises an effective amount of a compound of this invention and a pharmaceutically acceptable carrier.

The compositions are preferably adapted for oral administration. However, they may be adapted for other modes of administration, for example, parenteral administration for patient suffering from heart failure.

In order to obtain consistency of administration, it is preferred that a composition of the invention is in the form of a unit dose. Suitable unit dose forms include tablets, capsules and powders in sachets or vials. Such unit dose forms may contain from 0.1 to 100 mg of a compound of the invention and preferably from 2 to 50 mg. Still further preferred unit dosage forms contain 5 to 25 mg of a compound of the present invention. The compounds of the present invention canbe administered orally at a dose range of about 0.01 to 100 mg/kg or preferably at a dose range of 0.1 to 10 mg/kg. Such compositions may be administered from 1 to 6 times a day, more usually from 1 to 4 times a day.

The compositions of the invention may be formulated with conventional excipients, such as a filler, a disintegrating agent, a binder, a lubricant, a flavoring agent and the like. They are formulated in conventional manner, for example, in a manner similar to that used for known antihypertensive agents, diuretics and β-blocking agents.

The following examples are presented to illustrate rather than limit the scope of the invention.

### EXAMPLE 1 4-{[3,4-Dioxo-2-(1,2,2-trimethyl-propylamino)-cyclobut-1-enylaminol-methyl}-benzonitrile

### Step 1) 3-Ethoxy-4-(4-cyano-benzylamino)-cyclobut-3-ene-1,2-dione

To 4-cyanobenzylamine (1.2 g, 9.1 mmol) in absolute ethanol (40 mL) was added all at once 3,4-diethoxy-3-cyclobutene-1,2-dione (1.6 g, 9.4 mmol). The reaction mixture was stirred at room temperature for 5 days. The suspension of white solid was filtered and dried (0.4 mm, 65°C). This gave 1.07 g (46%) of 3-ethoxy-4-(4-cyano-benzylamino)-cyclobut-3-ene-1,2-dione as a solid: ¹H NMR (DMSO-d₆) δ 9.29 and 9.08 (two br m, 1H, rotamers), 7.83 (d, 2H), 7.49 (d, 2H), 4.80-4.50 (m, 4H), 1.36 and 1.28 (two t, 3H, rotamers). MS (m/z) 257 ([M+H]⁺).

### Step 2) (R)-4-{[3,4-Dioxo-2-(1,2,2-trimethyl-propylamino)-cyclobut-1-enylamino]-methyl}-benzonitrile

The product from the preceding paragraph (0.4 g, 1.56 mmol) in absolute ethanol (15 mL) and (R)-2-amino-3,3-dimethylbutane (11.7 mL of a 0.2 M solution in absolute ethanol, 2.3 mmol) were heated at reflux for 17 hours. The reaction mixture was cooled to room temperature and the white suspension was filtered, rinsed with absolute ethanol (2 x 10 mL), and dried (0.4 mm, 85°C). This gave 0.45 g of the (R) isomer as a white solid: mp 288-291°C (dec); [α]²⁵_{D} = +28.2° (9.7 mg/mL, DMSO); ¹H NMR (DMSO-d₆) δ 7.85 (d, 2H), 7.70 (br m, 1H), 7.52 (d, 2H), 7.29 (br d, 1H), 4.84 (m, 2H), 3.91 (br m, 1H), 1.10 (d, 3H), 0.85 (s, 9H). IR (KBr): 3200, 2960, 2250, 1800, 1650 cm⁻¹; MS (m/z) 312 ([M+H]⁺).

| Elemental analysis for C₁₈H₂₁N₃O₃ | |
|---|---|
| Calc'd | C, 69.43; H, 6.80; N, 13.49 |
| Found | C, 69.48; H, 6.75; N, 13.58 |

### (S)-4-{[3,4-Dioxo-2-(1,2,2-trimethyl-propylamino)-cyclobut-1-enylamino]-methyl}-benzonitrile

Repeating the procedure of the preceding paragraph with (S)-2-amino-3,3-dimethylbutane provides the corresponding (S) isomer.

### EXAMPLE 2 4-{[2-(1,2-Dimethyl-propylamino)-3,4-dioxo-cyclobut-1-enylamino]-methyl}-benzonitrile

The product of Example 1, Step 1 (1.0 g, 3.9 mmol) and 1,2-dimethylpropylamine (1.6 mL, 13.9 mmol) in absolute ethanol (19.5 mL) was allowed to stand at room temperature for 3 days. The precipitate was filtered, rinsed with ethyl acetate, and dried. This gave 0.94 g (81%) of the title compound as a white solid: mp 257-260°C; ¹H NMR (DMSO-d₆) δ 7.84 (d, 2H), 7.70 (br m, 1H), 7.51 (d, 2H), 7.30 (br m, 1H), 4.80 (m, 2H), 3.88 (br m, 1H), 1.69 (br m, 1H), 1.11 (d, 3H), 0.84 (d, 6H). IR (KBr): 3190, 2980, 2220, 1800, 1660 cm⁻¹; MS (m/z) 297 (M⁺).

| Elemental analysis for C₁₇H₁₉N₃O₂ | |
|---|---|
| Calc'd | C, 68.67; H, 6.44; N, 14.13 |
| Found | C, 68.10; H, 6.33; N, 14.14 |

### EXAMPLE 3 4-[(2-Isopropylamino-3,4-dioxo-cyclobut-1-enylamino)-methyl]-benzonitrile

This compound was prepared according to the procedure described in Example 2. From the product of Example 1, Step 1 (1.0 g, 3.9 mmol) and isopropylamine (1.2 mL, 14 mmol) there was obtained 0.90 g (86%) of the title compound as a white solid: mp 276-278°C (softens 268°C); ¹H NMR (DMSO-d₆) δ 7.84 (d, 2H), 7.70 (br m, 1H), 7.51 (d, 2H), 7.40 (br m, 1H), 4.79 (d, 2H), 4.09 (br m, 1H), 1.18 (d, 6H), IR (KBr): 3150, 2980, 2250, 1800, 1660 cm⁻¹; MS (m/z) 270 ((M+H]⁺).

| Elemental analysis for C₁₅H₁₅N₃O₂ | |
|---|---|
| Calc'd | C, 66.90; H, 5.61; N, 15.60 |
| Found | C. 66.24; H, 5.45; N, 15.39 |

### EXAMPLE 4 4-{[2-(1-Ethyl-propylamino)-3,4-dioxo-cyclobut-1-enylamino]-methyl}-benzonitrile

This compound was prepared according to the procedure described in Example 2. From the product of Example 1, Step 1 (1.0 g, 3.9 mmol) and 1-ethylpropylamine (1.6 mL, 14 mmol) there was obtained 1.02 g (88%) of the title compound as a white solid: mp 265-268°C; ¹H NMR (DMSO-d₆) δ 7.84 (d, 2H), 7.70 (br m, 1H), 7.51 (d, 2H), 7.25 (br m, 1H), 4.80 (d, 2H), 3.78 (br m, 1H), 1.55 (m, 2H), 1.42 (m, 2H), 0.84 (t, 6H). IR (KBr): 3160, 2980, 2250, 1800, 1660 cm⁻¹; MS (m/z) 297 (M⁺).

| Elemental analysis for C₁₇H₁₉N₃O₂ | |
|---|---|
| Calc'd | C, 68.67; H, 6.44; N, 14.13 |
| Found | C, 68.34; H, 6.38; N, 14.14 |

### EXAMPLE 5 4-[(2-tert-Butylamino-3,4-dioxo-cyclobut-1-enylamino)-methyl]-benzonitrile

This compound was prepared according to the procedure described in Example 2. From the product of Example 1, Step 1 (1.5 g, 5.9 mmol) and t-butylamine (29 mL), which were refluxed for 6 hours, there was obtained 1.59 g (95%) of the title compound as a white solid: mp 283-287°C (dec); ¹H NMR (DMSO-d₆) δ 7.85 (d, 2H), 7.84 (br t, 1H), 7.56 (br s, 1H), 7.52 (d, 2H), 4.81 (d, 2H), 1.35 (s, 9H), IR (KBr): 3150, 2980, 2250, 1800, 1660 cm⁻¹; MS (m/z) 284 ([M+H]⁺).

| Elemental analysis for C₁₆H₁₇N₃O₂ | |
|---|---|
| Calc'd | C, 67.83; H, 6.05; N, 14.83 |
| Found | C, 67.49; H, 5.78; N, 14.66 |

### EXAMPLE 6 4-[(3,4-Dioxo-2-propylamino-cyclobut-1-enylamino)-methyl]-benzonitrile

This compound was prepared according to the procedure described in Example 2. From the product of Example 1, Step 1 (0.25 g, 0.98 mmol) and propylamine (0.08 mL, 0.98 mmol) there was obtained 0.18 g (68%) of the title compound as an off-white solid: mp 241-245°C; ¹H NMR (DMSO-d₆) δ 7.84 (d, 2H), 7.80 (br m, 1H), 7.50 (d, 2H), 7.45 (br m, 1H), 4.78 (d, 2H), 3.44 (m, 2H), 1.50 (m, 2H), 0.86 (t, 3H). IR (KBr): 3170, 2980, 2250, 1800, 1660 cm⁻¹; MS (m/z) 269 (M⁺).

| Elemental analysis for C₁₅H₁₅N₃O₂ | |
|---|---|
| Calc'd | C, 66.90; H, 5.62; N, 15.60 |
| Found | C, 66.72; H, 5.46; N, 15.46 |

### EXAMPLE 7 4-{[2-(2-Hydroxy-1,1-dimethyl-ethylamino)-3,4-dioxo-cyclobut-1-enylamino]-methyl}-benzonitrile

This compound was prepared according to the procedure described in Example 2. From the product of Example 1, Step 1 (0.25 g, 0.98 mmol) and 2-hydroxy-1,1-dimethylethylamine (0.093 mL, 0.98 mmol) there was obtained 0.13 g (44%) of the title compound as a white solid: mp 253-257°C; ¹H NMR (DMSO-d₆) δ 8.00 (br t, 1H), 7.85 (d, 2H), 7.52 (d, 2H), 7.49 (br·s, 1H), 5.04 (t, 1H), 4.81 (d, 2H), 3.40 (d, 2H), 1.28 (s, 6H). IR (KBr): 3240, 2980, 2250, 1800, 1660 cm⁻¹; MS (m/z) 299 (M⁺).

| Elemental analysis for C₁₆H₁₇N₃O₃ | |
|---|---|
| Calc'd | C, 64.20; H, 5.73; N, 14.04 |
| Found | C, 63.84; H, 5.56; N, 13.86 |

### EXAMPLE 8 4-[(2-Butylamino-3,4-dioxo-cyclobut-1-enylamino)-methyl]-benzonitrile

This compound was prepared according to the procedure described in Example 2. From, the product of Example 1, Step 1 (0.25 g, 0.98 mmol) and n-butylamine (0.096 mL, 0.98 mmol) there was obtained 0.22 g (79%) of the title compound as a white solid: mp 250-252°C; ¹H NMR (DMSO-d₆) δ 7.84 (d, 2H), 7.80 (br m, 1H), 7.50 (d, 2H), 7.40 (br m, 1H), 4.78 (d, 2H), 3.49 (br m, 2H), 1.47 (m, 2H), 1.29 (m, 2H), 0.87 (t, 3H). IR (KBr): 3160, 2950, 2250, 1810, 1640 cm⁻¹; MS (m/z) 283 (M⁺).

| Elemental analysis for C₁₆H₁₇N₃O₂ | |
|---|---|
| Calc'd | C. 67.83; H, 6.05; N, 14.83 |
| Found | C. 67.59; H, 5.91; N, 14.62 |

### EXAMPLE 9 4-[(2-Cyclopentylamino-3,4-dioxo-cyclobut-1-enylamino)-methyl]-benzonitrile

This compound was prepared according to the procedure described in Example 2. From the product of Example 1, Step 1 (0.25 g, 0.98 mmol) and cyclopentylamine (0.096 mL, 0.98 mmol) there was obtained 0.25 g (86%) of the title compound as a pale pink solid: mp 283-286°C (dec); IR (KBr): 3180, 2950, 2250, 1800, 1650 cm⁻¹; MS (m/z) 295 (M⁺).

| Elemental analysis for C₁₇H₁₇N₃O₂ | |
|---|---|
| Calc'd | C, 69.14; H, 5.80; N, 14.23 |
| Found | C, 68.61; H, 5.74; N, 14.13 |

### EXAMPLE 10 4-[(2-Isobutylamino-3,4-dioxo-cyclobut-1-enylamino)-methyl]-benzonitrile

This compound was prepared according to the procedure described in Example 2. From the product of Example 1, Step 1 (0.25 g, 0.98 mmol) and isobutylamine (0.097 mL, 0.98 mmol) there was obtained 0.23 g (83%) of the title compound as a pale orange solid: mp 255-257°C (softens 243°C); ¹H NMR (DMSO-d₆) δ 7.84 (d, 2H), 7.76 (br m, 1H), 7.50 (d, 2H), 7.45 (br m, 1H), 4.79 (d, 2H), 3.33 (br m, 2H), 1.73 (m, 1H), 0.85 (d, 6H). IR (KBr): 3160, 2950, 2250, 1800, 1640 cm⁻¹; MS (m/z) 283 (M⁺).

| Elemental analysis for C₁₆H₁₇N₃O₂ | |
|---|---|
| Calc'd | C, 67.83; H, 6.05; N, 14.83 |
| Found | C, 67.32; H, 6.00; N, 14.60 |

### EXAMPLE 11 4-[(2-Methylamino-3,4-dioxo-cyclobut-1-enylamino)-methyl]-benzonitrile

This compound was prepared according to the procedure described in Example 2. From the product of Example 1, Step 1 (0.25 g, 0.98 mmol) and methylamine (0.12 mL of an 8 M solution in ethanol, 0.98 mmol) there was obtained 0.21 g (89%) of the title compound as a white solid: mp 302-306°C (dec); ¹H NMR (DMSO-d₆) δ 7.90 (br m, 1H), 7.84 (d, 2H), 7.50 (d, 2H), 7.30 (br m, 1H), 4.77 (d, 2H), 3.11 (br s, 3H). IR (KBr): 3180, 2980, 2250, 1800, 1650 cm⁻¹; MS (m/z) 241 (M⁺).

| Elemental analysis for C₁₃H₁₁N₃O₂ | |
|---|---|
| Calc'd | C, 64.72; H, 4.60; N, 17.42 |
| Found | C, 64.19; H, 4.44; N, 17.10 |

### EXAMPLE 12 (S)-4-[2-sec-Butylamino-3,4-dioxo-cyclobut-1-enylamino)-methyl]-benzonitrile

This compound was prepared according to the procedure described in Example 2. From the product of Example 1, Step 1 (0.25 g, 0.98 mmol) and (S)-(+)-2-sec-butylamine (0.098 m, 0.98 mmol) there was obtained 0.18 g (64%) of the title compound as a white solid: mp 253-256°C; [α]²⁵_{D} = +3.82° (10 mg/mL, DMSO); IR (KBr): 3200, 2980, 2250, 1800, 1650 cm⁻¹; MS (m/z) 283 (M⁺).

| Elemental analysis for C₁₆H₁₇N₃O₂ | |
|---|---|
| Calc'd | C, 67.83; H, 6.05; N, 14.83 |
| Found | C, 67.85; H, 5.93; N, 14.65 |

### EXAMPLE 13 (R)-4-[(2-sec-Butylamino-3,4-dioxo-cyclobut-1-enylamino)-methyl]-benzonitrile

This compound was prepared according to the procedure described in Example 2. From, the product of Example 1, Step 1 (0.25 g, 0.98 mmol) and (R)-(-)-2-sec-butylamine (0.10 mL, 0.98 mmol) there was obtained 0.18 g (64%) of the title compound as a white solid: mp 254-257°C; [α]²⁵_{D} = -3.36° (10 mg/mL, DMSO); IR (KBr): 3180, 2970, 2250, 1800, 1650 cm⁻¹; MS (m/z) 283 (M⁺).

| Elemental analysis for C₁₆H₁₇N₃O₂ | |
|---|---|
| Calc'd | C, 67.83; H, 6.05; N, 14.83 |
| Found | C, 67.68; H, 6.02; N, 14.73 |

### EXAMPLE 14 4-{[3,4-Dioxo-2-(2,2,3,3,3-pentafluoro-propylamino)-cyclobut-1-enylamino]-methyl}-benzonitrile

This compound was prepared according to the procedure described in Example 2. From the product of Example 1, Step 1 (0.25 g, 0.98 mmol) and 2,2,3,3,3-pentafluoropropylamine (0.17 g, 0.98 mmol) there was obtained 0.11 g (31%) of the title compound as an off-white solid: mp 252-256°C (softens 246°C); ¹H NMR (DMSO-d₆) δ 7.92 (br m, 2H), 7.85 (d, 2H), 7.51 (d, 2H), 4.80 (d, 2H), 4.42 (m, 2H). IR (KBr): 3200, 2220, 1800, 1650 cm⁻¹; MS (m/z) 359 (M⁺).

| Elemental analysis for C₁₅H₁₀F₅N₃O₂ | |
|---|---|
| Calc'd | C, 50.15; H, 2.80; N, 11.70 |
| Found | C, 50.81; H, 2.69; N, 11.85 |

### EXAMPLE 15 N-[2-(4-Cyano-benzylamino)-3,4-dioxo-cyclobut-1-enyl]-N-(1,2,2-trimethyl-propyl)-butyramide

The (R) isomer from Example 1, Step 2 (0.5 g, 1.6 mmol) in tetrahydrofuran (20 mL) was placed under Argon at room temperature. Solid sodium hydride (77 mg of a 60% dispersion in mineral oil, 1.9 mmol) was added and the reaction mixture was stirred at room temperature for 20 minutes. Then butyric acid anhydride (0.79 mL, 4.8 mmol) was added. The solution was stirred at room temperature for 3 hours, then refluxed for 8 hours. The reaction mixture was loaded onto a plug of silica gel and eluted with hexane:ethyl acetate (1:1) to give a residue, which was triturated repeatedly with hexane until 0.28 g (45%) of the title compound as a pale yellow foam was obtained: [α]²⁵_{D} = -101.94° (10 mg/mL, DMSO); ¹H NMR (DMSO-d₆) δ 8.05 (br m, 1H), 7.83 (d, 2H), 7.45 (d, 2H), 5.22 (br m, 2H), 4.07 (m, 1H) 2.50-2.30 (m, 2H), 1.52 (m, 2H), 1.16 (d, 3H), 0.87 (s, 9H), 0.82 (t, 3H). IR (KBr): 3450, 3350, 2980, 2250, 1800, 1730 cm⁻¹; MS (m/z) 382 ([M+H]⁺).

| Elemental analysis for C₂₂H₂₇N₃O₃ | |
|---|---|
| Calc'd | C, 69.27; H, 7.13; N, 11.02 |
| Found | C, 69.22; H, 7.08; N, 11.29 |

### EXAMPLE 16 N-(4-Cyano-benzyl)-N-[3,4-dioxo-2-(1,2,2-trimethyl-propylamino)-cyclobut-1-enyl]-but-2-enamide

This compound was prepared according to the procedure described in Example 15. From the (R) isomer of Example 1, Step 2 (0.35 g, 1.12 mmol) and crotonic anhydride (0.50 mL, 3.4 mmol) there was obtained 0.12 g (28%) of the (R),(E) isomer of the title compound as a pale yellow solid: α]²⁵_{D} = -146.82° (10 mg/mL, DMSO); ¹H NMR (DMSO-d₆) δ 8.00 (br m, 1H), 7.82 (d, 2H), 7.47 (d, 2H), 6.99 (doublet of q, 1H), 6.33 (br d, 1H), 5.29 (m, 2H), 1.84 (dd, 3H), 1.15 (d, 3H), 0.88 (s, 9H). IR (KBr): 3450, 3350, 2980, 2250, 1800, 1730 cm⁻¹; MS (m/z) 379 (M⁺).

| Elemental analysis for C₂₂H₂₅N₃O₃ | |
|---|---|
| Calc'd | C, 69.64; H, 6.64; N, 11.07 |
| Found | C, 68.90; H, 6.52; N, 10.74 |

### EXAMPLE 17 Pentanoic acid (4-cyano-benzyl)-[3,4-dioxo-2-(1,2,2-trimethyl-propylamino)-cyclobut-1-enyl]-amide

This compound was prepared according to the procedure described in Example 15. From the (R) isomer of Example 1, Step 2 (0.35 g, 1.12 mmol) and valeric anhydride (0.68 mL, 3.4 mmol) there was obtained 0.36 g (81%) of the title compound as a yellow foam: [α]²⁵_{D} = -89.81° (10 mg/mL, DMSO); ¹H NMR (CDCl₃) δ 7.69 (d, 2H), 7.60 (br d, 1H), 7.33 (d, 2H), 5.46 (AB quartet, 2H), 4.27 (m, 1H), 2.35 (m, 2H), 1.62-1.50 (m, 2H), 1.30-1.20 (m with overlapping d at δ 1.24, 5H), 0.97 (s, 9H), 0.86 (t, 3H). IR (KBr): 3350, 2950, 2250, 1800, 1730 cm⁻¹; MS (m/z) 395 (M⁺).

| Elemental analysis for C₂₃H₂₉N₃O₃ | |
|---|---|
| Calc'd | C, 69.85; H, 7.39; N, 10.62. |
| Found | C, 69.51; H, 7.46; N, 10.41. |

### EXAMPLE 18 Hexanoic acid (4-cyano-benzyl)-[3,4-dioxo-2-(1,2,2-trimethyl-propylamino)-cyclobut-1-enyl]-amide

This compound was prepared according to the procedure described in Example 15. From the (R) isomer of Example 1, Step 2 (0.35 g, 1.12 mmol) and hexanoic anhydride (0.78 mL, 3.4 mmol) there was obtained 0.25 g (55%) of the title compound as a sticky yellow gum: ¹H NMR (CDCl₃) δ 7.68 (d, 2H), 7.60 (br d, 1H), 7.33 (d, 2H), 5.45 (AB quartet, 2H), 4.28 (m, 1H), 2.33 (m, 2H), 1.65-1.50 (m, 4H), 1.30-1.20 (m with overlapping d at δ 1.25, 5H), 0.98 (s, 9H), 0.86 (t, 3H). IR (KBr): 3300, 2970, 2250, 1800, 1730 cm⁻¹; MS (m/z) 410 ([M+H]⁺).

| Elemental analysis for C₂₄H₃₁N₃O₃ | |
|---|---|
| Calc'd | C, 70.39; H, 7.63; N, 10.26 |
| Found | C, 70.05; H, 7.84; N, 10.02 |

### EXAMPLE 19 3-Butoxy-4-(1,2,2-trimethyl-propylamino)-cyclobut-3-ene-1,2-dione

Tetrahydrofuran (15 mL), 3,4-dibutoxy-3-cyclobutene-1,2-dione (2.26 g, 10 mmol) and 2-amino-3,3-dimethylbutane (1.01 g, 10 mmol) were stirred together for approximately 65 hours at room temperature. The waxy solid remaining after removal of solvent was dissolved in approximately 15 mL chloroform and chromatographed (flash, ethyl acetate/hexane) on silica. The appropriate fractions were freed of solvent to yield 2.41 g (95%) of a cream-colored waxy solid: mp 90-9°C° (softens 85°C).

Two recrystallization of 1.1 g of this material from hexane provided 0.833 g of the title compound as a white solid: mp 90-93C° (softens 88C°); ¹H NMR: (DMSO-d₆): δ 8.73 and 8.50 (two br d, 1H, rotamers), 4.64 (m, 2H), 3.92 and 3.41 (two m, 1H, rotamers), 1.71 (m, 2H), 1.38 (m, 2H), 1.11 (m, 3H), 0.91 (t, 3H), 0.84 (m, 9H) ppm. IR (KBr): 3135, 1800, 1690 cm⁻¹; MS (m/z): 253 (M⁺).

| Elemental Analysis for C₁₄H₂₃NO₃ | |
|---|---|
| Calcd | C, 66.37; H, 9.15; N, 5.53. |
| Found | C, 66.47; H, 9.20; N, 5.50. |

### EXAMPLE 20 3-(2,4-Dichlorobenzylamino)-4-(1,2,2-trimethyl-propylamino)-cyclobut-3-ene-1,2-dione

Tetrahydrofuran (10 mL), 3-butoxy-4-(1,2,2-trimethyl-propylamino)-cyclobut-3-ene-1,2-dione (1.01 g, 3.99 mmol, Example 19) and 2,4-dichlorobenzylamine (0.70 g, 4.0 mmol) were stirred together at room temperature for 14 hours. Following removal of solvent, the residue was triturated with diethyl ether and dried. The off-white solid product was recrystallized twice from nitromethane to yield 0.408 g (29%) of the title compound as a white solid: mp 234-235°C; ¹H NMR: (DMSO-d₆): δ 7.67 (m, br, 1H), 7.65 (m, br, 1H), 7.48 (m, 2H), 7.32 (m, br, 1H), 4.80 (m, 2H), 3.90 (m, 1H), 1.10 (d, 3H), 0.86 (s, 9H) ppm. IR (KBr): 3140, 1790, 1640 cm⁻¹; MS (m/z): 354/356/358. HPLC indicates a major component (99%).

| Elemental Analysis for C₁₇H₂₀Cl₂N₂O₂ | |
|---|---|
| Calcd. | C, 57.47; H, 5.67; N, 7.88. |
| Found | C, 57.02; H, 5.44; N, 7.75. |
| | C, 57.69; H, 5.69; N, 7.79. |

### EXAMPLE 21 3-Ethoxy-4-(1,2,2-trimethyl-propylamino)-cyclobut-3-ene-1,2-dione

A solution of 3,4-diethoxy-3-cyclobutene-1,2-dione (10 g, 59 mmol) and (R)-2-amino-3,3-dimethylbutane (353 mL of a 0.2M solution in absolute ethanol, 71 mmol) was stirred at room temperature for 24 hours. Another portion of (R)-2-amino-3,3-dimethylbutane (150 mL of a 0.2 M solution in absolute ethanol, 30 mmol) was added and the resulting solution was stirred at room temperature for 24 hours. The slurry was filtered, and the filtrate concentrated under reduced pressure. The resulting solid was trituated with hexane:ethyl acetate (150:5 mL), then washed with hexane to give 9.78 g (74%) of (R)-3-ethoxy-4-(1,2,2-trimethyl-propylamino)-cyclobut-3-ene-1,2-dione as a white solid: ¹H NMR: (DMSO-d₆): δ 8.72 and 8.50 (two d, 1H, rotamers), 4.65 (m, 2H), 3.90 and 3.42 (two m, 1H, rotamers), 1.37 and 1.35 (two overlapping t, 3H, rotamers), 1.10 (two overlapping d, 3H, rotamers), 0.85 and 0.84 (two s, 9H, rotamers) ppm. IR (KBr): 3150, 2950, 1800, 1700 cm⁻¹; MS (m/z): 225 (M⁺).

| Elemental Analysis for C₁₂H₁₉NO₃ | |
|---|---|
| Calcd | C, 63.98; H, 8.50; N, 6.22. |
| Found | C, 64.33; H, 8.54; N, 6.52. |

(S)-3-Ethoxy-4-(1,2,2-trimethyl-propylamino)-cyclobut-3-ene-1,2-dione is produced by the same method by substituting (S)-2-amino-3,3-dimethylbutane for the (R)-2-amino-3,3-dimethylbutane employed in the preceding paragraph.

### EXAMPLE 22 3-(2,4-Dichloro-6-methyl-benzylamino)-4-(1,2,2-trimethyl-propylamino)-cyclobut-3-ene-1,2-dione

The product of Example 21 (0.2 g, 0.88 mmol) and 2,4-dichloro-6-methylbenzylamine (0.17 g, 0.89 mmol, containing approximately 5% of a compound which is regioisomeric with respect to the substitution on the aryl ring) were placed in absolute ethanol (4.4 mL) and dichloromethane (2 mL). The resulting clear solution was allowed to stand at room temperature for 4 days. The reaction mixture was diluted with acetonitrile (5 mL) and filtered, rinsed with acetonitrile, and dried to give 0.3 g of a solid. Trituation with 10% methanol in dichloromethane gave 0.21 g (63%) of (R)-3-(2,4-dichloro-6-methyl-benzylamino)-4-(1,2,2-trimethyl-propylamino)-cyclobut-3-ene-1,2-dione as a white solid, which contains approximately 5% of a compound which is regioisomeric with respect to substitution on the aryl ring: mp >300°C; [α]²⁵_{D} = +31.86° (7.7 mg/mL, DMSO); ¹H NMR (DMSO-d₆) d 7.54 (d, 1H), 7.39 (d, 1H), 7.31 (m, 1H), 7.17 (m, 1H), 4.89 (m, 2H), 4.70 (doublet of m, minor isomer), 3.89 (m, 1H), 2.41 (s, 3H), 2.31 (s, minor isomer), 1.09 (d, 3H), 0.85 (s, 9H) ppm. IR (KBr): 3150, 2950, 1800 cm⁻¹; MS (m/z) 368/370/372 (M⁺).

| Elemental analysis for C₁₈H₂₂Cl₂N₂O₂ | |
|---|---|
| Calc'd | C, 58.54; H, 6.01; N, 7.59. |
| Found | C, 58.48; H, 6.02; N, 7.45. |

(S)-3-(2,4-Dichloro-6-methyl-benzylamino)-4-(1,2,2-trimethyl-propylamino)-cyclobut-3-ene-1,2-dione is produced by the same method by substituting (S)-3-ethoxy-4-(1,2,2-trimethyl-propylamino)-cyclobut-3-ene-1,2-dione for the (R)-3-ethoxy-4-(1,2,2-trimethyl-propylamino)-cyclobut-3-ene-1,2-dione employed in the preceding paragraph.

### EXAMPLE 23 3-Butoxy-4-(1,1-dimethyl-propylamino)-cyclobut-3-ene-1,2-dione

A solution of 3,4-dibutoxy-3-cyclobutene-1,2-dione (4.53 g, 20 mmol) and 1,1-dimethylpropylamine (1.74 g, 20 mmol) in tetrahydrofuran (20 mL) was stirred at room temperature for approximately 19.5 hours. The solvent was removed and the residue was chromatographed (gravity, chloroform/hexane) on neutral, activity III silica (150 g). The white solid isolated from the appropriate eluates was recrystallized from hexane to give 4.105 g (86%) of a white product: mp 56.5-57.5°C (softens 55.5°C).

One gram of this material was recrystallized twice from hexane to provide 0.794 g of the title compound as a white solid: mp 56-57°C (softens 55°C); ¹H NMR (DMSO-d₆): δ 8.63 and 8.48 (two br s, 1H, rotamers), 4.67 (m, br, 2H), 1.67 (m, br, 4H), 1.39 (m, 2H), 1.26 (m, br, 6H), 0.91 (t, 3H), 0.78 (t, 3H) ppm. IR (KBr): 3170, 1790, 1700 cm⁻¹; MS (m/z): 239 (M⁺).

| Elemental Analysis for C₁₃H₂₁NO₃ | |
|---|---|
| Calcd | C, 65.24; H, 8.85; N, 5.85 |
| Found | C, 65.12; H, 8.90; N, 5.77 |

### EXAMPLE 24 3-(2,4-Dichlorobenzylamino)-4-(1,1-dimethylpropylamino)-cyclobut-3-ene-1,2-dione

A solution of 3-butoxy-4-(1,1-dimethyl-propylamino)-cyclobut-3-ene-1,2-dione (7.18 g, 30 mmol, Example 23) and 2,4-dichlorobenzylamine (5.28 g, 30 mmol) in tetrahydrofuran (40 mL) was stirred at room temperature for 16 hours. The solvent was removed and the residue was triturated thoroughly with diethyl ether and dried to give 8.94 g of a crude product. Successive recrystallizations of this material from acetonitrile (charcoal), acetonitrile (twice), acetone (charcoal) and acetone (twice) afforded 4.08 g (40%) of the title compound as a white, electrostatic solid: mp 196-197°C (softens 188°C); ¹H NMR (DMSO-d₆): δ 7.81 (m, 1H), 7.68 (m, 1H), 7.48 (m, 3H), 4.81 (d, 2H), 1.67 (m, 2H), 1.31 (s, 6H), 0.82 (t, 3H) ppm. IR (KBr): 3210, 1790, 1645 cm⁻¹; MS (m/z) 340/342/344 (M⁺). HPLC indicates a major component (99%). Differential scanning calorimetry studies indicate that this material is a mixture of crystal forms.

| Elemental Analysis for C₁₆H₁₈Cl₂N₂O₂ | |
|---|---|
| Calcd | C, 56.32; H, 5.32; N, 8.21. |
| Found | C, 55.93; H, 5.20; N, 8.18. |

### EXAMPLE 25 N-(2,4-Dichloro-benzyl)-N-[2-(1,1-dimethyl-propylamino)-3,4-dioxo-cyclobut-1-enyl]-acetamide

To the product of Example 24 (0.60g, 1.26 mmol) in a mixture of tetrahydrofuran (8 mL) and dimethylformamide (2 mL) at ambient temperature under a nitrogen atmosphere was added NaH (0.077g of a 60% dispersion in mineral oil, 1.94 mmol). After stirring for 15 minutes at ambient temperature, acetic anhydride (0.183 mL, 1.94 mmol) was added neat. The mixture was stirred for 2 hours and was then diluted with brine and extracted with ethyl acetate (3 x 50 mL). The organic phase was washed with 10% aqueous Na₂CO₃ and brine, dried (MgSO₄), decolorized (charcoal), and concentrated to afford a residue. Crystallization from diethyl ether afforded 0.36 g (53%) of N-(2,4-dichloro-benzyl)-N-[2-(1,1-dimethyl-propylamino)-3,4-dioxo-cyclobut-1-enyl]-acetamide as a white solid, mp: 114-116°C; 1H NMR δ (DMSO-d₆) 7.66 (d, 1H), 7.32 (dd, 1H), 7.29 (d, 1H), 5.20 (br s, 2H), 2.15 (s, 3H), 1.70 (q, 2H), 1.35 (s, 6H), 0.85 (t, 3H) ppm. IR (KBr): 3400, 3300, 2950, 1800, 1700, 1580 cm⁻¹. MS (m/z) 382/384/386 (M⁺).

| Elemental analysis for C₁₈H₂₀Cl₂N₂O₃ | |
|---|---|
| Calc'd | C, 56.41; H, 5.26; N, 7.31. |
| Found | C, 56.30; H, 5.27; N, 7.25. |

### EXAMPLE 26 N-(2,4-Dichloro-benzyl)-N-[2-(1,1-dimethyl-propylamino)-3,4-dioxo-cyclobut-1-enyl]-butyramide

To the product of Example 24 (0.60g, 1.26 mmol) in a mixture of tetrahydrqfuran (8 mL) and dimethylformamide (2 mL) at ambient temperature under a nitrogen atmosphere was added NaH (0.077g of a 60% dispersion in mineral oil, 1.94 mmol). After stirring for 15 minutes at ambient temperature, butyric anhydride (0.317 mL, 1.94 mmol) was added neat. The mixture was stirred for 2 hours and was then diluted with brine and extracted with ethyl acetate (3 x 50 mL). The organic phase was washed with 10% aqueous Na₂CO₃ and brine, dried (MgSO₄), decolorized (charcoal), and concentrated to afford a clear oil. Crystallization from diethyl ether afforded 0.52 g (72%) of N-(2,4-dichloro-benzyl)-N-[2-(1,1-dimethyl-propylamino)-3,4-dioxo-cyclobut-1-enyl]-butyramide as a white solid. mp: 112-117°C; ¹H NMR δ (DMSO-d₆) 7.65 (d, 1H), 7.43 (dd, 1H), 7.29 (d, 1H), 5.16 (br s, 2H), 2.42 (t, 2H), 1.72 (q, 2H), 1.54 (m, 2H), 1.35 (s, 6H), 0.83 (m, 6H) ppm. IR (KBr): 3400, 3300, 2950, 1800, 1725, 1580 cm⁻¹. MS (m/z) 410/412/414 (M⁺).

| Elemental analysis for C₂₀H₂₄Cl₂N₂O₃ | |
|---|---|
| Calc'd | C, 58.40; H, 5.88; N, 6.81. |
| Found | C, 58.40; H, 5.84; N, 6.86. |

### EXAMPLE 27 3-(2,4-Dichloro-6-methyl-benzylamino)-4-(1,1-dimethyl-propylamino)-cyclobut-3-ene-1,2-dione

This compound was prepared in a procedure similar to the one described in Example 22. From 3-ethoxy-4-(1,1-dimethyl-propylamino)-cyclobut-3-ene-1,2-dione (16.67 g, 79.0 mmol) and 2,4-dichloro-6-methylbenzylamine (15.02 g, 79.0 mmol) in absolute ethanol (395 mL) there was obtained after filtration a white solid, which was washed with diethyl ether/hexane and dried in vacuo. This yielded 25.7 g (92%) of the title compound as a white solid: mp 247.1-248.3°C; ¹H NMR (DMSO-d₆) δ 7.54 (d, 1H), 7.44 (br t, 1H), 7.39 (d, 1H), 7.31 (s, 1H), 4.90 (d, 2H), 2.40 (s, 3H), 1.66 (q, 2H), 1.28 (s, 6H), 0.80 (t, 3H) ppm. IR (KBr): 3200, 2980, 1800, 1650 cm⁻¹; MS (m/z) 354/356/358 (M⁺). Analytical HPLC indicates a major component (99.9%).

| Elemental analysis for C₁₇H₂₀Cl₂N₂O₂ | |
|---|---|
| Calc'd | C, 57.47; H, 5.67; N, 7.89. |
| Found | C, 57.31; H, 5.50; N, 7.80. |

### EXAMPLE 28 3-Butoxy-4-tert-butylamino-cyclobut-3-ene-1,2-dione

A solution of 3,4-dibutoxy-3-cyclobutene-1,2-dione (11.31 g, 50 mmol) and *tert*-butylamine (3.66 g, 50 mmol) in tetrahydrofuran (80 mL) was stirred at room temperature for 71 hours. The solvent was removed and a solution of the residue in chloroform was washed with water and dried (anhydrous Na₂SO₄). Removal of the solvent and chromatographic (gravity, chloroform/hexane) purification of the amber liquid residue on a column of neutral, activity III silica (350 g ) provided 9.83 g (87%) of a white solid product, mp 67.0-68.5°C. Two recrystallizations of an aliquot (800 mg) afforded *551* mg of the title compound as a white solid: mp 68-69°C (softens 67°C); ¹H NMR (DMSO-d₆): δ 8.75 and 8.59 (two br s, 1H, rotamers). 4.66 (m, br, 2H), 1.72 (m, 2H), 1.40 (m, 2H), 1.31 (m, 9H), 0.91 (t, 3H) ppm. IR (KBr): 3140, 1780, 1700 cm⁻¹; MS (m/z) 225 (M⁺).

| Elemental Analysis for C₁₂H₁₉NO₃ | |
|---|---|
| Calcd | C, 63.98; H, 8.50; N, 6.22. |
| Found | C, 64.13; H, 8.60; N, 6.24. |

### EXAMPLE 29 3-(t-Butylamino)-4-(2.4-dichlorobenzyl-amino)-cyclobut-3-ene-1,2-dione

A solution of 3-butoxy-4-tert-butylamino-cyclobut-3-ene-1,2-dione (1.13 g, 5.0 mmol, Example 28) and 2,4-dichlorobenzyl amine (0.884 g, 5.0 mmol) in tetrahydrofuran (15 mL) was stirred at room temperature for 16.5 hours. Removal of solvent, thorough trituration of the residue with diethyl ether and drying gave 1.50 g of a solid. Two recrystallizations of the crude product from acetonitrile afforded 1.22 g (74%) of the title compound as a white solid: mp 229-230°C (dec.); ¹H NMR (DMSO-d₆): δ 7.77 (m, 1H), 7.68 (m, 1H), 7.61 (s, br, 1H), 7.48 (m, 2H), 4.79 (d, 2H), 1.36 (s, 9H) ppm. IR (KBr): 3300, 3220, 1780, 1660 cm⁻¹; MS (m/z): 326/328/330 (M⁺). HPLC indicates a major component (99.6%).

| Elemental Analysis for C₁₅H₁₆Cl₂N₂O₂ | |
|---|---|
| Calcd | C, 55.06; H, 4.93; N, 8.56 |
| Found | C, 54.86; H, 4,89; N, 8.48 |

### EXAMPLE 30 3-tert-Butylamino-4-(2,4-dichloro-6-methyl-benzylamino)-cyclobut-3-ene-1,2-dione

This compound was prepared in a procedure similar to the one described in Example 22. From 3-ethoxy-4-tert-butylamino-cyclobut-3-ene-1,2-dione (0.22 g, 1.1 mmol) and 2,4-dichloro-6-methylbenzylamine (0.22 g, 1.2 mmol, containing approximately 5% of a compound which is regioisomeric with respect to the substitution on the aryl ring) in absolute ethanol (5.5 mL) there was obtained 0.34 g (89%) of 3-*tert*-butylamino-4-(2,4-dichloro-6-methyl-benzylamino)- cyclobut-3-ene-1,2-dione as a white solid, which contains approximately 5% of a compound which is regioisomeric with respect to substitution on the aryl ring: mp 264-268°C; ¹H NMR (DMSO-d₆) δ 7.54 (d, 1H), 7.46 (s, 1H), 7.43 (br t, 1H), 7.39 (d, 1H), 4.89 (d, 2H), 4.72 (d, minor isomer), 2.40 (s, 3H), 2.31 (s, minor isomer), 1.34 (s, 9H) ppm. IR (KBr): 3200, 2950, 1800, 1650 cm ⁻¹; MS (m/z) 340/342/344 (M⁺).

| Elemental analysis for C₁₆H₁₈Cl₂N₂O₂ | |
|---|---|
| Calc'd | C, 56.32; H, 5.32; N, 8.21. |
| Found | C, 56.09; H, 5.28; N, 8.16. |

### EXAMPLE 31 3-Butoxy-4-(1-ethyl-propylamino)-cyclobut-3-ene-1,2-dione

A solution of 3,4-dibutoxy-3-cyclobut-3-ene-1,2-dione (2.26 g, 10 mmol) and 1-ethylpropylamine (0.872 g, 10 mmol) in tetrahydrofuran (8 mL) was stirred at room temperature for 2.5 hours. The residue remaining after removal of solvent was dissolved in chloroform and the solution was washed with water and dried (anhydrous Na₂SO₄). Removal of solvent gave a waxy- solid that was chromatographed (flash, chloroform/hexane) on silica. The solid isolated from the appropriate fractions was recrystallized twice from hexane to yield 0.896 g (37%) of the title compound: mp 65-66°C; ¹H NMR (DMSO-d₆): δ 8.63 and 8.40 (two d, 1H, rotamers), 4.64 (m, 2H), 3.74 and 3.30 (two m, 1H, rotamers), 1.71 (m, 2H), 1.54 (m, 2H), 1.39 (m, 4H), 0.90 (m, 3H), 0.82 (m, 6H) ppm. IR (KBr): 3140, 1790, 1720 cm⁻¹; MS (m/z) 239 (M⁺).

| Elemental Analysis for C₁₃H₂₁NO₃ | |
|---|---|
| Calcd | C, 65.25; H, 8.85; N, 5.85. |
| Found | C, 65.37; H, 9.07; N, 5.87. |

### EXAMPLE 32 3-(3,4-Dichloro-benzylamino)-4-(1-ethyl-propylamino)-cyclobut-3-ene-1,2-dione

This compound was prepared in a manner similar to Example 24 using the appropriate starting materials, to afford 3-(3,4-dichloro-benzylamino)-4-(1-ethyl-propylamino)-cyclobut-3-ene-1,2-dione as a white solid: mp 268-269°C.

### EXAMPLE 33 3-(2,4-Dichloro-benzylamino)-4-(1-ethyl-propylamino)-cyclobut-3-ene-1,2-dione

This compound was prepared in a manner similar to Example 24 using the appropriate starting materials to afford 3-(2,4-dichloro-benzylamino)-4-(1-ethyl-propylamino)-cyclobut-3-ene-1,2-dione as very pale yellow solid: mp 210-211°C.

### EXAMPLE 34 3-(2,4-Dichloro-6-methyl-benzylamino)-4-(2,2,3,3,3-pentafluoro-propylamino)-cyclobut-3-ene-1,2-dione

A solution of 3,4-diethoxy-3-cyclobutene-1,2-dione (4.2 mL, 28.4 mmol) and 2,2,3,3,3-pentafluoropropylamine (4.24 g, 28.4 mmol) in absolute ethanol (142 mL) was stirred at room temperature for 24 hours. The solution was concentrated under reduced pressure and triturated with 10% ethyl acetate in hexane to yield 1.14 g (14.6%) of a white solid,: mp 95-100°C; ¹H NMR (DMSO-d₆) δ 9.40 and 9.20 (two br m, 1H, rotamers), 4.67 (q, 2H), 4.33 and 4.11 (two br t, 2H, rotamers), 1.36 (br m, 3H); MS (m/z) 274 ([M+H]⁺). Following the procedure described in Example 22, from a portion of this solid, 3-ethoxy-4-(2,2,3,3,3-pentafluoro-propylamino)-cyclobut-3-ene-1,2-dione (0.72 g, 2.6 mmol) and 2,4-dichloro-6-methylbenzylamine (0.5 g, 2.6 mmol) there was obtained 1.03 g (94%) of the title compound as a white solid: mp 287-292°C; ¹H NMR (DMSO-d₆) δ 7.62 (br m, 2H), 7.53 (s, 1H), 7.38 (d, 1H), 4.89 (d, 2H), 4.43 (doublet of t, 2H), 2.40 (s, 3H) ppm.

| Elemental analysis for C₁₅H₁₁Cl₂ F₅N₂O₂ | |
|---|---|
| Calc'd | C, 43.19; H, 2.66; N, 6.72. |
| Found | C, 43.13; H, 2.61; N, 6.74. |

### EXAMPLE 35 N-(2,4-Dichloro-6-methyl-benzyl)-N-[2-(1,1-dimethyl-propylamino)-3,4-dioxo-cyclobut-1-enyl]-butyramide

To 3-(2,4-dichloro-6-methyl-benzylamino)-4-(1,1-dimethyl-propylamino)-cyclobut-3-ene-1,2-dione (0.50 g, 1.41 mmol) in N,N-dimethylformamide (2 mL) and tetrahydrofuran (8 mL) was added sodium hydride (0.062 g of a 60% dispersion in mineral oil, 1.54 mmol) at 0°C. The frothy suspension was stirred for 1 hour as the mixture was warmed to 25°C. Butyric anhydride (0.24 g, 1.54 mmol) was added and the reaction mixture was stirred at 0°C for 15 minutes and then allowed to warm to room temperature. After stirring overnight, the reaction mixture was poured into brine (50 mL) and extracted with ethyl acetate (3 x 50 mL). The organic layer was dried over magnesium sulfate and decolorized (charcoal). The solvent was removed *in vacuo* and the remaining oil was triturated with diethyl ether/petroleum ether to yield 0.31 g (53%) of a white solid: mp 117.2-118.4°C; ¹H NMR (DMSO-d₆) δ 8.80 (br s, 1H), 7.39 (d, 1H), 7.28 (d, 1H), 5.06 (s, 2H), 2.34 (s, 3H), 2.29 (t, 2H), 1.67 (q, 2H), 1.51 (q, 2H), 1.30 (s, 6H), 0.82 (q, 6H) ppm. IR (KBr): 3230, 2950, 1800, 1744, 1700, 1570 cm⁻¹; MS (m/z) 424 (M⁺).

| Elemental analysis for C₂₁H₂₆Cl₂N₂O₃ | |
|---|---|
| Calc'd | C, 59.30; H, 6.16; N, 6.59. |
| Found | C, 59.34; H, 6.09; N, 6.52. |

### EXAMPLE 36 N-(2,4-Dichloro-6-methyl-benzyl)-N-[2-(1,1-dimethyl-propylamino)-3,4-dioxo-cyclobut-1-enyl]-acetamide

This compound was prepared according to the procedure described in Example 35. From 3-(2,4-dichloro-6-methyl-benzylamino)-4-(1,1-dimethyl-propylamino)-cyclobut-3-ene-1,2-dione (0.50 g, 1.41 mmol) and acetic anhydride (0.16 g, 1.54 mmol) there was obtained 0.36 g (64%) of the title compound as a white solid: mp 112.2-113.9°C; ¹H NMR (DMSO-d₆) δ 8.71 (br s, 1H), 7.40 (d, 1H), 7.29 (d, 1H), 5.06 (s, 2H), 2.35 (s, 2H), 2.05 (s, 3H), 1.67 (q, 2H), 1.30 (s, 6H), 0.81 (t, 3H) ppm. IR (KBr): 3230, 2950, 1800, 1755, 1590 cm⁻¹; MS (m/z) 396 (M⁺).

| Elemental analysis for C₁₉H₂₂Cl₂ N₂O₃ | |
|---|---|
| Calc'd | C, 57.44; H, 5.58; N, 7.05. |
| Found | C, 57.16; H, 5.52; N, 6.94. |

### EXAMPLE 37 N-(tert-butyl)-N-[2-(2,4-Dichloro-6-methyl-benzylamino)-3,4-dioxo-cyclobut-1-enyl]-propionamide

To 3-butoxy-4-tert-butylamino-cyclobut-3-ene-1,2-dione (0.50 g, 2.5 mmol) in tetrahydrofuran (12.6 mL) was added sodium hydride (0.091 g of a 80% dispersion in mineral oil, 3.0 mmol). The suspension was stirred for 20 minutes at room temperature. The slightly cloudy yellow solution was concentrated under reduced pressure, and the resulting white solid was suspended in propionic anhydride (2 mL) and dichloromethane (3 mL). The reaction mixture was stirred at room temperature for 18 hours, then heated at 115°C for 24 hours. After standing at room temperature for 5 days the reaction mixture was diluted with dichloromethane, filtered, and concentrated under reduced pressure. Purification by column chromatography (silica gel, hexane/ethyl acetate) gave 0.09 g of material that was placed in acetonitrile (1.8 mL). 2,4-Dichloro-6-methylbenzylamine (67 mg, 0.35 mmol) and acetonitrile (2 mL) was added. After 3 days at room temperature, the reaction mixture was concentrated under reduced pressure, and the resulting solid was recrystallized from hexane and ethyl acetate to give 74 mg (53%) of a white solid: mp 177-180°C; ¹H NMR (DMSO-d₆) δ 9.31 (t, 1H), 7.54 (d, 1H), 7.40 (d, 1H), 4.94 (d, 2H), 2.40 (s, 3H), 2.09 (q, 2H), 1.37 (s, 9H), 0.87 (t, 3H) ppm.

| Elemental analysis for C₁₉H₂₂Cl₂N₂O₃ | |
|---|---|
| Calc'd | C, 57.44; H, 5.58; N, 7.05. |
| Found | C, 56.41; H, 5.19; N, 6.98. |

### EXAMPLE 38 3-(2,4-dichloro-6-methyl-benzylamino)-4-(1,2-dimethyl-2-fluoro-propylamino)-cyclobut-3-ene-1,2-dione

### Step 1) 3-Fluorovalinol

To a solution of lithium borohydride (1.61 g, 74 mmol) in THF(40 mL) under a nitrogen atmosphere was added trimethylsilyl chloride(18.8 mL, 148 mmol) via pipet. A precipitate quickly formed. After 3 minutes, 3-fluorovaline (5 g, 37 mmol) was added in three portions. This mixture was stirred for 24 hours. The reaction was quenched by the dropwise addition of methanol. The methanol and THF were removed on a rotary evaporator (30 degree water bath) and water (25 mL) was added. The aqueous mixture was made basic with 2.5N aq. NaOH and was then extracted with dichloromethane (4 x 50 mL). The combined organics were dried(Na₂SO₄), filtered and evaporated to give 3.83 g of 3-fluorovalinol: ¹H NMR (CDCl₃) δ 3.71 (dd, 1H), 3.36 (m, 1H), 2.90 (m,1H), 2.10 (br, 2H), 1.38 (d, 3H), 1.33 (d, 3H) ppm.

### Step 2) N-Butoxycarbonyl-3-fluorovalinol

To a solution of 3-fluorovalinol (3.79 g, 31.4 mmol) in chloroform (35 mL) under a nitrogen atmosphere was added a solution of di-t-butyl dicarbonate (6.84 g, 31.4 mmol) in chloroform (15 mL). The mixture was stirred at room temperature for four hours, then the solvent was removed on a rotary evaporator. The residue was dissolved in diethyl ether (100 mL), washed with 20% phosphoric acid (1 x50 mL), brine (1 x 50 mL), saturated aqueous sodium bicarbonate (1 x 50 mL), brine (1 x 50 mL), and then dried(MgSO₄). Filteration and concentration under reduced pressure gave 6.34 g of N-butoxycarbonyl-3-fluorovalinol as a white solid: ¹H NMR (CDCl₃) δ 5.08 (br, 1H), 3.82 (m, 2H), 3.68 (m, 1H), 1.46 (s, 9H), 1.46 (d, 3H) and 1.39 (d, 3H) ppm.

### Step 3) N-Butoxycarbonyl-1-iodo-2-amino-3-fluoro-3-methyl-n-butane

To a well-stirred mixture of polystyryl supported triphenyl phosphine (29.3 mmol) in dry dichloromethane (40 mL) under a nitrogen atmosphere was added iodine (7.44 g, 29.3 mmol). After ten minutes, imidazole (2.0 g, 29.3 mmol) was added followed in ten minutes by a solution of N-butoxycarbonyl-3-fluorovalinol (13.3 mmol) in dichloromethane (200 mL). The mixture was heated to reflux for two hours. The cooled mixture was filtered through Celite® and the filtrate was evaporated. The residue was dissolved in diethyl ether (150 mL) and this solution was washed with dilute aqueous sodium thiosulfate (1 x 75 mL) and water (2 x 75 mL). The organic layer was dried (Na₂SO₄), filtered through a pad of silica gel and evaporated to afford 3.46 grams of N-butoxycarbonyl-1-iodo-2-amino-3-fluoro-3-methyl-n-butane: ¹H NMR (CDCl₃) δ 4.72 (br d, 1H), 3.86 (br m, 1H), 3.56 (dd, 1H), 1.47 (s, 9H), 1.43 (m, 6H) ppm.

### Step 4) N-Butoxycarbonyl-2-amino-3-fluoro-3-methyl-n-butane

A Parr bottle was charged with palladium (II) hydroxide (800 mg), a solution of N-butoxycarbonyl-1-iodo-2-amino-3-fluorro-3-methyl-n-butane (3.26 g, 9.8 mmol) in ethanol (80 mL) and triethylamine (0.99 g, 9.8 mmol). The reaction mixture was placed under hydrogen gas (50 psig) and shaken for 20 hours. The mixture was filtered through celite and evaporated. The residue was dissolved in diethyl ether (100 mL) and washed with 1N aq. HCl (2 x 50 mL), water (2 x 50 mL), and then dried (MgSO₄). Filtration and evaporation gave a residue that was chromatographed (silica gel, diethyl ether/hexane (3/1)) to afford 1.80 g of N-butoxycarbonyl-2-amino-3-fluoro-3-methyl-n-butane: ¹H NMR (CDCl₃) δ 4.65 (br, 1H), 3.70 (br m, 1H), 1.45 (s, 9H), 1.39 (d, 3H), 1.32 (d, 3H) and 1.18 (d, 3H) ppm.

### Step 5) 3-Ethoxy-4-(3-fluoro-3-methyl-n-butyl-2-amino)-3-cyclobutene-1,2-dione

A mixture of N-butoxycarbonyl-2-amino-3-fluoro-3-methyl-n-butane (1.75 g, 8.5 mmol), dichloromethane (5 mL), trifluoroacetic acid (4 mL), and methanol (0.75 mL) was warmed to 45°C for five hours. The volatile components were removed on a rotary evaporator and the syrupy residue was used without further purification. To a solution of 3-fluoro-3-methyl-n-butyl-2-amine trifluoroacetate salt (8.5 mmol) in ethanol (42.5 mL) was added 3,4-diethoxy-3-cyclobutene-1,2-dione (1.44 g, 8.5 mmol) followed by triethylamine (2.58 g, 25.5 mmol). The reaction mixture was stirred under a nitrogen atmosphere at room temperature for two hours then the temperature was raised to 50°C for five hours. The mixture was cooled and the solvents removed on a rotary evaporator. The residue was dissolved in diethyl ether (90 mL) and washed with water (1 x 60 mL), 1 N aq. HCl (1 x 60 mL), water 1 x 60 mL). The organic layer was dried (MgSO₄), filtered, and evaporated. The residue was chromatographed (silica gel, diethyl ether) to afford 1.65 g of 3-ethoxy-4-(3-fluoro-3-methyl-n-butyl-2-amino)-3-cyclobutene-1,2-dione as a white solid: ¹H NMR (CDCl₃) δ 6.21 (br, 1H), 4.77 (br m, 2H), 3.80 (br, 1H), 1.47 (t, 3H), 1.43 (d, 3H), 1.36 (d, 3H) and 1.32 (d, 3H) ppm.

### Step 6) 3-(2,4-dichloro-6-methyl-benzylamino)-4-(1,2-dimethyl-2-fluoro-propylamino)-cyclobut-3-ene-1,2-dione

To a solution of 3-ethoxy-4-(3-fluoro-3-methyl-n-butyl-2-amino)-3-cyclobutene-1,2-dione (0.573 g, 2.5 mmol) in dry THF (8 mL) was added 2,4-dichloro-6-methylbenzylamine (0.523 g, 2.75 mmol). The mixture was heated to 70°C under a nitrogen atmosphere for 18 hours. The mixture was cooled to room temperature with stirring and vacuum filtered through a fritted glass filter. The solid was washed well with several portions of an ethanol/diethyl ether (1/1) solvent mixture. The solid was air dried then heated to 77°C under high vacuum for 16 hours. This afforded 0.48 g of the title compound as a white solid: ¹H NMR (DMSO-d₆) δ 7.54 (s, 1H), 7.40 (br, 1H), 7.38 (s, 2H), 4.90 (m, 2H), 4.17 (br, 1H), 2.41 (s, 3H), 1.32 (d, 3H), 1.27 (d, 3H) ,1.18 (d, 3H) ppm. IR (KBr): 1850 cm⁻¹; MS (m/z) 373 ([M+H]⁺).

### EXAMPLE 39 3-Butoxy-4-(2,4-dichlorobenzylamino)-cyclobut-3-ene-1,2-dione

A solution of 3,4-dibutoxy-3-cyclobutene-1,2-dione (3.39 g, 15 mmol) and 2,4-dichlorobenzylamine (2.64 g, 15 mmol) in tetrahydrofuran (20 mL) was stirred at room temperature for 5.5 hours. After removal of solvent, the residue was dissolved in chloroform (approximately 30 mL) and chromatographed (flash, ethyl acetate/hexane) on silica. The appropriate fractions were freed of solvent to give 4.31 g (88%) of a white product: mp 140-142°C (softens 137°C). Three recrystallizations of 1.1 g of this material from methyl *t*-butyl ether provided 0.566 g of the title compound as a white solid: mp 139.5-140.0°C (softens 137.5°C; ¹H NMR (DMSO-d₆) δ 9.25 and 9.00 (two m, 1H, rotamers), 7.64 (d, 1H), 7.51-7.34 (m, 2H), 4.69 (m, 2H), 4.56 (s, 2H), 1.72 (m, 1H), 1.58 (m, 1H), 1.38 (m, 1H), 1.19 (m, 1H), 0.91 and 0.82 (two t, 3H, rotamers) ppm. IR (KBr): 3160, 1760, 1700 cm⁻¹. MS (m/z) 327/329/331 (M⁺). HPLC indicates a major component (>99%).

| Elemental analysis for C₁₅H₁₅Cl₂NO₃ | |
|---|---|
| Calc'd | C, 54.90; H, 4.61; N, 4.27. |
| Found | C, 54.98; H, 4.51; N, 4.11. |

### EXAMPLE 40 3-(2,4-Dichlorobenzylamino)-4-(2-hydroxy-1,1-dimethylethylamino)-cyclobut-3-ene-1,2-dione

Tetrahydrofuran (10 mL), 3-butoxy-4-(2,4-dichlorobenzylamino)-cyclobut-3-ene-1,2-dione (1.31 g, 3.94 mmol, Example 39), and 2-amino-2-methyl-1-propanol (0.36 g, 4.0 mmol) were stirred together for 43.5 hours at room temperature. Following removal of solvent, the residue was trituated with diethyl ether and dried to yield 1.13 g of a yellow solid. Three recrystallizations of the crude product from methanol gave 0.503 g of the title compound as cream-colored solid: mp 237-238°C (softens 234°C); ¹H NMR(DMSO-d₆) δ 7.94 (t, 1H), 7.68 (m, 1H), 7.54 (m, 1H), 7.48 (m, 2H), 5.04 (t, 1H), 4.79 (d, 2H), 3.39 (d, 2H), 1.15 (s, 6H) ppm. IR (KBr): 3380, 3250, 1780, 1650 cm⁻¹. MS (m/z) 343 ([M+H]⁺). HPLC indicates a major component (99.9%).

| Elemental analysis for C₁₅H₁₆Cl₂N₂O₃ | |
|---|---|
| Calc'd | C, 52.49; H, 4.70; N, 8.16. |
| Found | C, 52.57; H, 4.59; N, 8.12. |

### EXAMPLE 41 3-(2,4-Dichlorobenzylamino)-4-(1,2,2-trimethylpropylamino)-cyclobut-3-ene-1,2-dione

The product of Example 21 (0.901 g, 4.0 mmol) and 2,4-dichlorobenzylamine (0.704 g, 4.0 mmol) in tetrahydrofuran (20 mL) were stirred at room temperature for approximately 16 hours and then were refluxed for approximately 24 hours. After removal of solvent, the residue was recrystallized from methanol (charcoal) and again recrystallized from methanol to afford 0.25 g (18%) of (R)-3-(2,4-dichlorobenzylamino)-4-(1,2,2-trimethylpropylamino)-cyclobut-3-ene-1,2-dione as a white solid: mp 235-239°C (softens 233°C); [α]²⁵_{D} = +26.28° (9.58 mg/mL, DMSO); ¹H NMR (DMSO-d₆) δ 7.67 (s, 1H), 7.63 (br s, 1H), 7.48 (m, 2H), 7.30 and 7.15 (two br d, 1H, rotamers), 4.80 (m, 2H), 3.90 (br s, 1H), 1.10 (m, 3H), 0.86 (m, 9H) ppm. IR (KBr): 3180, 1800, 1650 cm⁻¹. MS (m/z) 354/356/358 (M⁺). Analytical HPLC indicates chemical purity (96%) and optical purity (100%).

| Elemental analysis for C₁₇H₂₀Cl₂N₂O₃ | |
|---|---|
| Calc'd | C, 57.48; H, 5.68; N, 7.89. |
| Found | C, 57.96; H, 5.86; N, 7.77. |
| | C, 58.11; H, 5.76; N, 8.05. |

(S)-3-(2,4-Dichlorobenzylamino)-4-(1,2,2-trimethylpropylamino)-cyclobut-3-ene-1,2-dione is produced by the same method by substituting (S)-3-ethoxy-4-(1,2,2-trimethyl-propylamino)-cyclobut-3-ene-1,2-dione for the (R)-3-ethoxy-4-(1,2,2-trimethyl-propylamino)-cyclobut-3-ene-1,2-dione employed in the preceding paragraph.

### EXAMPLE 42 3-tert-Butylamino-4-(3,4-dichlorobenzylamino)-cyclobut-3-ene-1,2-dione

A solution of 3-butoxy-4-tert-butylamino-cyclobut-3-ene-1,2-dione (1.13 g, 5 mmol, Example 28) and 3,4-dichlorobenzylamine (0.880 g, 5.0 mmol) in tetrahydrofuran (15 mL) was stirred at room temperature for approximately 96 hours. The residue isolated after removal of solvent was recrystallized from N,N-dimethylformamide (twice) and from 2-methoxyethanol to provide 0.687 g (42%) of the title compound as a white solid: mp 302-303°C; ¹H NMR (DMSO-d₆) δ 7.77 (m, 1H), 7.65 (d, 1H), 7.62 (d, 1H), 7.55 (s, 1H), 7.33 (m, 1H), 4.71 (d, 2H), 1.35 (s, 9H) ppm. IR (KBr): 3450, 3230, 1800, 1650 cm⁻¹. MS (m/z) 326/328/330 (M⁺). HPLC indicates a major component (>99%).

| Elemental analysis for C₁₅H₁₆Cl₂N₂O₂ | |
|---|---|
| Calc'd | C, 55.06; H, 4.93; N, 8.56. |
| Found | C, 54.81; H, 4.56; N, 8.44. |

### EXAMPLE 43 3-(3,4-Dichlorobenzylamino)-4-(1,1-dimethylpropylamino)-cyclobut-3-ene-1,2-dione

Tetrahydrofuran (15 mL), 3-butoxy-4-(1,1-dimethylpropylamino)-cyclobut-3-ene-1,2-dione (1.20 g, 5.0 mmol) and 3,4-dichlorobenzylamine (0.88 g, 5.0 mmol) were stirred together at room temperature for approximately 16 hours. Removal of solvent gave 1.67 g of a white solid which was recrystallized from methanol (twice) to yield 1.06 g (62%) of the title compound as a white solid: mp 277-279°C; ¹H NMR (DMSO-d₆) δ 7.80 (m, 1H), 7.65 (d, 1H), 7.62 (d, 1H), 7.42 (s, 1H), 7.34 (m, 1H), 4.73 (d, 2H), 1.66 (m, 2H), 1.30 (s, 6H), 0.81 (t, 3H) ppm. IR (KBr): 3300, 3240, 1790, 1650 cm⁻¹. MS (m/z) 340/342/344 (M⁺). HPLC indicates a major component (98.9%).

| Elemental analysis for C₁₆H₁₈Cl₂N₂O₂ | |
|---|---|
| Calc'd | C, 56.32; H, 5.32; N, 8.21. |
| Found | C, 56.34; H, 5.03; N, 8.03. |

### EXAMPLE 44 3-Butoxy-4-tert-butylamino-cyclobut-3-ene-1,2-dione

A solution of 3,4-dibutoxy-3-cyclobutene-1,2-dione (11.31 g, 50 mmol) and tert-butylamine (3.66 g, 50 mmol) in tetrahydrofuran (80 mL) was stirred at room temperature for 71 hours. The solvent was removed and a solution of the residue in chloroform was washed with water and dried (anhydrous Na₂SO₄). Removal of the solvent and chromatographic (gravity, chloroform/hexane) purification of the amber liquid residue on a column of neutral, activity III silica (350 g ) provided 9.83 g (87%) of a white solid product, mp 67.0-68.5°C. Two recrystallizations of an aliquot (800 mg) afforded 551 mg of the title compound as a white solid: mp 68-69°C (softens 67°C); ¹H NMR (DMSO-d₆) δ 8.75 and 8.59 (two br s, 1H, rotamers), 4.66 (br m, 2H), 1.72 (m, 2H), 1.40 (m, 2H), 1.31 (m, 9H), 0.91 (t, 3H) ppm. IR (KBr): 3140, 1780, 1700 cm⁻¹; MS (m/z) 225 (M⁺).

| Elemental analysis for C₁₂H₁₉NO₃ | |
|---|---|
| Calc'd | C, 63.98; H, 8.50; N, 6.22. |
| Found | C, 64.13; H, 8.60; N, 6.24. |

### EXAMPLE 45 4-[(2-tert-Butylamino-3,4-dioxo-cyclobut-1-enylamino)-methyl]-3-chloro-benzonitrile

### Step 1) Preparation of 2-Chloro-4-cyanobenzyl bromide

A mixture of 3-chloro-4-methylbenzonitrile (22.74 g, 150 mmol), N-bromosuccinimide (32.04 g, 180 mmol) and 2,2'-azobis-2-methylpropionitrile (2.46 g, 15 mmol) in carbon tetrachloride (120 mL) was carefully warmed to reflux temperature whereupon a moderate exotherm occurred and refluxing proceeded for approximately 10 minutes without external heating. Heating was then resumed and refluxing continued for 26 hours. The hot reaction mixture was suction filtered and the insolubles were rinsed with carbon tetrachloride (3 x 25 mL). The combined carbon tetrachloride fractions were washed with water and dried (anhydrous Na₂SO₄). Removal of solvent gave a yellow mush which was crystallized from hexane (charcoal). The product again was recrystallized from hexane to yield 20.44 g (59%) of the white bromide: mp 80.5 - 83.5°C (softens 71.5°C) (lit. mp 85-85.5°C (B. Gogolimska, Acta Pol. Pharm., 25 (4), 391 (1968) [C.A., 70, 87493e (1969)].)); ¹H NMR (DMSO-d₆) δ 8.10 (d, 1H), 7.82 (m, 2H), 4.69 (s, 2H) ppm. IR (KBr): 2220 cm⁻¹.

### Step 2) Preparation of N-(2-chloro-4-cyanobenzyl)phthalimide

A mixture of product from Example 45, Step 1 (20.29 g, 88.0 mmol) and potassium phthalimide (17.92 g, 96.8 mmol) in N,N-dimethylformamide (200 mL) was stirred as the reaction temperature rose to approximately 36°C during approximately 5 minutes with formation of a tan suspension. The temperature then receded and stirring was continued for 2 hours. After removal of solvent, the residue was triturated thoroughly with water and dried.

The buff solid product was treated with approximately 500 mL boiling ethyl acetate, gravity filtered to remove a small amount of white insoluble material, heated to boiling, treated with charcoal and filtered. Concentration and cooling of the filtrate afforded (after drying) 20.26 g (78%) of the title compound phthalimide as a white solid: mp 172.5 - 173.0°C (softens 170.5°C); ¹H NMR (DMSO-d₆) δ 8.10 (d, 1H), 7.90 (m, 4H), 7.75 (dd, 1H), 7.52 (d, 1H), 4.88 (s, 2H) ppm. IR (KBr): 2220, 1770, 1715 cm⁻¹.

### Step 3) Preparation of 2-chloro-4-cyanobenzylamine

A mechanically stirred suspension of product from Example 45, Step 2 (18.99 g, 64 mmol) in absolute ethanol (150 mL) was treated with hydrazine hydrate (6.41 g, 128 mmol) and the mixture was stirred and refluxed for 1 hour and then was allowed to stand at room temperature for approximately 16.5 hours. With stirring 2N HCl (90 mL) was added slowly, and after 10 minutes of further stirring the mixture was filtered. The insolubles were triturated thoroughly with ethanol and then with water. The combined filtrate and triturates were freed of solvent and the residue in approximately 250 mL ice-H₂O was basified with 2.5 N NaOH (90 mL). The mass was extracted thoroughly with chloroform and the extracts were washed with water, with brine and dried (anhydrous Na₂SO₄). Removal of solvent gave a cream-colored solid which was recrystallized from hexane to provide 6.85 g (64%) of a white amine: mp 85.0 - 87.0°C (soften 82.5°C); ¹H NMR(DMSO-d₆) δ 7.96 (d, 1H), 7.82 (dd, 1H), 7.77 (m, 1H), 3.82 (s, 2H), 2.12 (br m, 2H) ppm. IR (KBr): 3380, 3320, 2230 cm⁻¹.

### Step 4) 4-[(2-tert-Butylamino-3,4-dioxo-cyclobut-1-enylamino)-methyl]-3-chloro-benzonitrile

Tetrahydrofuran (50 mL), 3-butoxy-4-tert-butylamino-cyclobut-3-ene-1,2-dione (6.76 g, 30 mmol, Example 44) and the product of Example 45, Step 3 (5.00 g, 30 mmol) were refluxed for 6 hours and allowed to stand at room temperature for 16 hours. Following removal of solvent from the reaction mixture, the residue was triturated thoroughly with diethyl ether and dried to give a buff solid. This material in approximately 1.4 L of boiling acetone, was filtered to remove a small amount of white solid. The hot filtrate was treated with charcoal, filtered, concentrated and cooled to afford 6.521 g of a cream-colored solid. Two additional recrystallizations of this material from acetone gave 4.779 g (50%) of the title compound as a white solid: mp 243.5-245.°C (softens 241.0°C); ¹H NMR (DMSO-d₆) δ 8.10 (d, 1H), 7.88 (dd, 1H), 7.82 (m, 1H), 7.66 (s, br, 1H), 7.61 (d, 1H), 4.88 (d, 2H), 1.34 (s, 9H) ppm. IR (KBr): 3320, 3230, 2240, 1780, 1665 cm⁻¹; MS (m/z) 317/319 (M⁺). HPLC indicates a major component (99.6%).

| Elemental analysis for C₁₆H₁₆ClN₃O₂ | |
|---|---|
| Calc'd | C, 60.48; N, 5.08; N, 13.22; Cl, 11.16. |
| Found | C, 60.08; H, 4.97; N, 13.06; Cl, 10.82, 10.71. |

The following is another method for the preparation of 4-[(2-tert-butylamino-3,4-dioxo-cyclobut-1-enylamino)-methyl]-3-chloro-benzonitrile. To a solution of 4-[(2-butoxy-3,4-dioxo-cyclobut-1-enylamino)-methyl]-3-chlorobenzonitrile (0.638 g, 2 mmol, Example 135) in tetrahydrofuran (8 mL) was added t-butylamine (0.146 g, 2 mmol). After stirring overnight at room temperature, the mixture was refluxed for 6.5 hours. Additional t-butylamine (0.146g, 2 mmol) was added and the mixture was stirred overnight at room temperature. Removal of solvent gave a yellow solid that was recrystallized from acetone to afford 0.217 g of the title compound: mp 244-246°C (dec.). MS (m/z) 317/319 (M⁺). Based on NMR (DMSO-d₆) spectral comparison, this product is the same as that described in Example 45, Step 4.

### EXAMPLE 46 3-Butoxy-4-(1,1-dimethyl-propylamino)-cyclobut-3-ene-1,2-dione

A solution of 3,4-dibutoxy-3-cyclobutene-1,2-dione (4.53 g, 20 mmol) and 1,1-dimethylpropylamine (1.74 g, 20 mmol) in tetrahydrofuran (20 mL) was stirred at room temperature for approximately 19.5 hours. The solvent was removed and the residue was chromatographed (gravity, chloroform/hexane) on neutral, activity III silica (150 g). The white solid isolated from the appropriate eluates was recrystallized from hexane to give 4.105 g (86%) of a white product: mp 56.5-57.5°C (softens 55.5°C).

One gram of this material was recrystallized twice from hexane to provide 0.794 g of the title compound as a white solid: mp 56-57°C (softens 55°C); ¹H NMR (DMSO-d₆) δ 8.63 and 8.48 (two br s, 1H, rotamers), 4.67 (br m, 2H), 1.67 (br m, 4H), 1.39 (m, 2H), 1.26 (br m, 6H), 0.91 (t, 3H), 0.78 (t, 3H) ppm. IR (KBr): 3170, 1790, 1700 cm⁻¹; MS (m/z) 239 (M⁺).

| Elemental analysis for C₁₃H₂₁NO₃ | |
|---|---|
| Calc'd | C, 65.24; H, 8.85; N, 5.85 |
| Found | C, 65.12; H, 8.90; N, 5.77 |

### EXAMPLE 47 3-(2,6-Dichloro-benzylamino)-4-(1,1-dimethyl-propylamino)-cyclobut-3-ene-1,2-dione

### Step 1) Preparation of N-(2,6-dichlorobenzyl)phthalimide

To α,2,6-trichlorotoluene (19.55 g, 100 mmol) in N,N-dimethylformamide (130 mL) was added with stirring potassium phthalimide (18.54 g, 100 mmol) and stirring was continued for 22 hours. Solvent was removed and the residue was dissolved in chloroform (400 mL)/water (350 mL). The chloroform extract was washed with water, with brine and dried (anhydrous Na₂SO₄). Removal of solvent and drying gave 28.98 g of a white solid. Recrystallization of the crude product from ethyl acetate provided 24.63 g (81%) of white phthalimide: mp 147-150°C (The preparation of this compound is given in U.S. Pat. 4,146,647 (March 27, 1979, to Laboratoire L. Lafon) but no physical constants are reported); ¹H NMR (DMSO-d₆): δ 7.83 (s, 4H), 7.47 (m, 2H), 7.35 (m, 1H), 5.00 (s, 2H) ppm. IR (KBr): 1770, 1715 cm⁻¹; MS (m/z) 306/308/310 ([M+H]⁺).

### Step 2) Preparation of 2,6-dichlorobenzylamine hydrochloride

The phthalimide from Example 47, Step 1 (6.13 g, 20 mmol), ethanol (90 mL) and hydrazine monohydrate (3.00 g, 60 mmol) were refluxed for 1.25 hours. After cooling to room temperature the mixture was filtered and the insolubles were rinsed with ethanol (3 x 50 mL). The combined filtrate and rinsings were freed of solvent and the residue was shaken with ethyl acetate and 10% aqueous sodium carbonate solution. The ethyl acetate fraction was washed successively with 10% w/v aqueous sodium carbonate solution, brine and dried (anhydrous Na₂SO₄). Removal of solvent and drying of the residue gave 3.17 g of a pale yellow oil.

Dissolution of 3.15 g of the preceding oil in diethyl ether and addition of isopropanolic hydrogen chloride gave a white solid that was collected, rinsed with diethyl ether and dried. The crude hydrochloride was recrystallized from isopropanol in the presence of several drops of isopropanolic hydrogen chloride, rinsed with diethyl ether and dried to afford 2.70 g (64% overall) of white hydrochloride: mp 260-261°C (dec.) (lit. mp 237-238°C (S. Angyal et al., J. Chem. Soc., 1949, 2704; The preparation of this compound is given in U.S. Pat. 4,146,647 (March 27, 1979, to Laboratoire L. Lafon) but no physical constants are reported)); ¹H NMR (DMSO-d₆): δ 8.54 (s, br, 3H), 7.58 (m, 2H), 7.48 (m, 1H), 4.23 (s, 2H) ppm. IR (KBr): 2860 cm⁻¹; MS (m/z) 174/176/178 (M⁺).

### Step 3) 3-(2,6-Dichloro-benzylamino)-4-(1,1-dimethyl-propylamino)-cyclobut-3-ene-1,2-dione

To 2,6-dichlorobenzylamine hydrochloride of Example 47, Step 2 (1.06 g 5.0 mmol) in tetrahydrofuran (10 mL) was added methyl amine (0.505 g, 5.0 mmol) followed by 3-butoxy-4-(1,1-dimethyl-propylamino)-cyclobut-3-ene-1,2-dione (1.20 g, 5.0 mmol, Example 46) and stirring was continued at room temperature for approximately 16 hours. The reaction mixture was freed of solvent and the residue was triturated successively with water and with diethyl ether, and then dried. The resulting light yellow solid was recrystallized from acetonitrile three times to provide 0.755 g (44%) of the title compound as a white solid: mp 239-241°C (dec); ¹H NMR (DMSO-d₆): δ 7.57 (m, 1H), 7.55 (m, 1H), 7.52 (br m, 1H),m 7.43 (m, 1H), 7.30 (s, br, 1H), 5.07 (d, 2H), 1.65 (q, 2H), 1.28 (s, 6H), 0.80 (t, 3H) ppm. IR (KBr): 3250, 1785, 1660 cm⁻¹; MS (m/z) 340/342/344 (M⁺). HPLC indicates a major component (99.7%).

| Elemental analysis for C₁₆H₁₈Cl₂N₂O₂ | |
|---|---|
| Calc'd | C, 56.32; H, 5.32; N, 8.21. |
| Found | C, 56.17; H, 5.27; N, 8.17. |

### EXAMPLE 48 3-Butoxy-4-(1,2,2-trimethyl-propylamino)-cyclobut-3-ene-1,2-dione

Tetrahydrofuran (15 mL), 3,4-dibutoxy-3-cyclobutene-1,2-dione (2.26 g, 10 mmol) and 2-amino-3,3-dimethylbutane (1.01 g, 10 mmol) were stirred together for approximately 65 hours at room temperature. The waxy solid remaining after removal of solvent was dissolved in chloroform (15 mL) and chromatographed (flash, ethyl acetate/hexane) on silica. The appropriate fractions were freed of solvent to yield 2.41 g (95%) of cream-colored waxy solid: mp 90-9°C° (softens 85°C).

Two recrystallization of 1.1 g of this material from hexane provided 0.833 g of the title compound as a white solid: mp 90-93C° (softens 88C°); ¹H NMR: (DMSO-d₆): δ 8.73 and 8.50 (two br d, 1H, rotamers), 4.64 (m, 2H), 3.92 and 3.41 (two m, 1H, rotamers), 1.71 (m, 2H), 1.38 (m, 2H), 1.11 (m, 3H), 0.91 (t, 3H), 0.84 (m, 9H). IR(KBr): 3135, 1800, 1690 cm⁻¹; MS (m/z) 253 (M⁺).

| Elemental analysis for C₁₄H₂₃NO₃ | |
|---|---|
| Calc'd | C, 66.37; H, 9.15; N, 5.53. |
| Found | C, 66.47; H, 9.20; N, 5.50. |

### EXAMPLE 49 3-Chloro-4-{[3,4-dioxo-2-(1,2,2-trimethyl-propylamino)-cyclobut-1-enylamino]-methyl]}-benzonitrile

A solution of 3-butoxy-4-(1,2,2-trimethyl-propylamino)cyclobut-3-ene-1,2-dione (1.27 g, 5.0 mmol, Example 48), 2-chloro-4-cyanobenzylamine (0.833 g, 5.0 mmol, Example 45, Step 3) and tetrahydrofuran (8 mL) was stirred at room temperature for 23 hours, refluxed for 4 hours and allowed to stand at room temperature for approximately 62 hours. The mixture was freed of solvent and the residue was triturated with diethyl ether and dried. The resulting white solid (1.096 g) was recrystallized three times from methanol to yield 771 mg (45%) of the title compound as a faintly pink solid: mp 250.0-251.5°C (softens 248.0°C); ¹H NMR (DMSO-d₆) δ 8.10 (d, 1H), 7.88 (m, 1H), 7.70 (br m, 1H), 7.61 (d, 1H), 7.37 (br m, 1H), 4.88 (m, 1H), 3.91 (br m, 1H), 1.11 (d, 3H), 0.86 (s, 9H) ppm. IR (KBr): 3200, 2230, 1790, 1635 cm⁻¹; MS (m/z) 345/347 (M⁺). HPLC indicates a major component (98.7%).

| Elemental analysis for C₁₈H₁₀ClN₃O₂ | |
|---|---|
| Calc'd | C, 62.52; H, 5.83; N, 12.15. |
| Found | C, 62.57; H, 5.74; N, 12.00. |

### EXAMPLE 50 3-{[2-(1,1-Dimethyl-propylamino)-3,4-dioxo-cyclobut-1-enylamino]-methyl}-5-methyl-benzonitrile

### Step 1) Preparation of 3-cyano-5-methylbenzyl bromide

N-Bromosuccinimide (3.56 g, 20 mmol), 3,5-dimethylbenzonitrile (2.62 g, 20 mmol) and 2,2'-azobis-2-methylpropionitrile (0.328 g, 2.0 mmol) in carbon tetrachloride (25 mL) were refluxed for 23 hours. The hot mixture was filtered (twice) and the filtrate was freed of solvent to give a yellow oil that was chromatographed (flash, silica, ethyl acetate/hexane). From the appropriate eluates 2.587 g of a white solid were isolated. Recrystallization of this material from methanol provided 1.51 g (36%) of bromide: mp 82-85°C (softens 75°C) (lit. mp 92-93°C (T.H. Fisher et al, J. Org. Chem., 55, 1040 (1990)); 87-88°C (E. Gryszkiewicz-Trochimowski et al, Bull. Soc. Chem. Fr., 1948, 593 [C.A., 42, 7240h (1948)])); ¹H NMR (DMSO-d₆) δ 7.72 (s, 1H), 7.61 (s, 1H), 4.68 (s, 2H), 2.33 (s, 3H) ppm. IR (KBr): 2230 cm⁻¹; MS (m/z) 209/211 (M⁺).

### Step 2) Preparation of N-(3-cyano-5-methyl)phthalimide

A mixture of 3-cyano-5-methylbenzyl bromide from Example 50, Step 1 (15.79 g, 75.16 mmol) and potassium phthalimide (15.31 g, 82.68 mmol) in N,N-dimethylformamide (150 mL) was vigorously stirred for 4 hours. The reaction mixture was freed of solvent and the residue was dissolved in chloroform (200 mL)/water (400 mL). The chloroform fraction was separated and the aqueous phase was extracted with chloroform (2 x 75 mL). The combined chloroform fractions were washed with water, with brine and dried (anhydrous Na₂SO₄). Removal of solvent gave 20.2 g of crude product which was recrystallized from acetonitrile to yield 13.12 g (63%) of white phthalimide: mp 180-184°C; ¹H NMR (DMSO-d₆) δ 7.87 (br m, 4H), 7.60 (s, br, 1H), 7.56 (s, br, 1H), 7.47 (s, br, 1H) 4.78 (s, 2H), 2.32 (s, 3H) ppm. IR (KBr): 2240, 1770, 1720 cm⁻¹; MS (m/z) 276 (M⁺).

### Step 3) Preparation of 3-cyano-5-methylbenzylamine

A suspension of N-(3-cyano-5-methyl)phthalimide from Example 50, Step 2 (1.11 g, 4.0 mmol), hydrazine monohydrate (0.24 g, 4.8 mmol) and ethanol (20 mL) was refluxed for 3 hours, diluted with water (60 mL) and the mixture was acidified with conc. HCl (0.5 mL). After brief stirring, the mixture was filtered and the filtrate was basified with conc. sodium hydroxide and extracted with ethyl acetate (3 x 25 mL). The combined extracts were washed with water, with brine and dried (anhydrous Na₂SO₄). Removal of solvent gave 0.386 g (66%) of amine as a clear oil: ¹H NMR (DMSO-d₆) δ 7.57 (m, 1H), 7.47 (m, 2H), 3.70 (s, 2H), 2.33 (s, 3H) ppm. IR (KBr): 3380, 3300, 2220 cm⁻¹; MS (m/z) 146 (M⁺).

### Step 4) 3-{[2-(1,1-Dimethyl-propylamino)-3,4-dioxo-cyclobut-1-enylamino]-methyl}-5-methyl-benzonitrile

A solution of 3-butoxy-4-(1,1-dimethyl-propylamino)cyclobut-3-ene-1,2-dione (1.44 g, 6.0 mmol, Example 46) and 3-cyano-5-methylbenzylamine from Example 50, Step 3 (6.0 mmol) and tetrahydrofuran (15 mL) were stirred at room temperature for approximately 20 hours. An additional 7 mL of tetrahydrofuran were added and stirring continued for an additional 48 hours. The solvent was removed and the residue was recrystallized twice from methanol to provide 1.251 g (67%) of the title compound as a white solid: mp 231-133°C (softens 229°C); ¹H NMR (DMSO-d₆) δ 7.78 (m, 1H), 7.62 (m, 2H), 7.51 (s, 1H), 7.41 (s, br, 1H), 4.74 (d, 2H), 2.35 (s, 3H), 1.67 (m, 2H), 1.30 (s, 6H), 0.82 (m, 3H) ppm. IR (KBr): 3290, 2240, 1780, 1670 cm⁻¹; MS (m/z) 311 (M⁺). HPLC indicates a major component (99.7%).

| Elemental analysis for C₁₈H₂₁N₃O₂ | |
|---|---|
| Calc'd | C, 69.43; H, 6.80; N, 13.49. |
| Found | C, 69.25; H, 6.72; N, 13.60. |

### EXAMPLE 51 3-tert-Butylamino-4-(2,4-dimethyl-benzyl-amino)-cyclobut-3-ene-1,2-dione

A solution of 3-butoxy-4-tert-butylamino-cyclobut-3-ene-1,2-dione (1.13 g, 5,0 mmol, Example 44) and 2,4-dimethylbenzylamine (0.68 g, 5.0 mmol, a mixture of 2,4-and 2,6-dimethylbenzylamine isomers) in tetrahydrofuran (10 mL) was stirred at room temperature for approximately 16 hours. Removal of solvent, thorough trituration of the residue with diethyl ether and drying provided 1.07 g of crude product. Three recrystallizations of this material from acetonitrile gave 0.67 g (47%) of the title compound as a white solid: mp 228-229°C; ¹H NMR (DMSO-d₆) δ 7.57 (m, 1H), 7.48 (s, br, 1H), 7.17 (d, 1H), 7.03 (s, br, 1H), 7.00 (d, br, 1H), 4.69 (d, 2H), 2.27 (s, 3H), 2.24 (s, 3H), 1.35 (s, 9H) ppm. IR (KBr): 3310, 1785, 1670 cm⁻¹; MS (m/z) 286 (M⁺). HPLC indicates a major component (98.7%).

| Elemental analysis for C₁₇H₂₂N₂O₂. | |
|---|---|
| Calc'd | C, 71.30; H, 7.74; N, 9.78. |
| Found | C, 71.29; H, 7.79; N, 9.82. |

### EXAMPLE 52 3-tert-Butylamino-4-(2,4,6-trimethyl-benzylamino)-cyclobut-3-ene-1,2-dione

### Step 1) Preparation of N-(2,4,6-trimethylbenzyl)phthalimide

N,N-Dimethylformamide (50 mL), 2,4,6-trimethylbenzyl chloride (5.06 g, 30 mmol) and potassium phthalimide (6.11 g, 33 mmol) were combined with stirring. After an initial reaction temperature rise from approximately 25°C to 34°C during 15 minutes, the temperature receded to room temperature and stirring was continued for approximately 17 hours. Solvent then was removed and the residue was thoroughly triturated with water and dried. The 7.62 g crude product was recrystallized from ethyl acetate and dried to yield 5.62 g of white phthalimide: mp 204.5-206.5°C (softens 196.5°C) (lit. mp 209.5-210°C (R.T.Fuson and J.J. Denton, J. Am. Chem. Soc., 63, 654 (1941))); ¹H NMR (DMSO-d₆) δ 7.82 (s, 4H), 6.78 (s, 2H), 4.72 (s, 2H), 2.28 (s, 6H), 2.17 (s, 3H). IR(KBr): 1765, 1710 cm⁻¹; MS (m/z) 279 (M⁺).

### Step 2) Preparation of 2,4,6-trimethylbenzylamine hydrochloride

The phthalimide from Example 52, Step 1 (5.52 g, 19.8 mmol), ethanol (90 mL) and hydrazine monohydrate (2.97 g, 59.4 mmol) were refluxed for 1.3 hours. After cooling to room temperature the mixture was filtered and the insolubles were rinsed with ethanol (3 x 50 mL). The combined filtrate and rinsings were freed of solvent and the residue was dissolved in ethyl acetate. The solution was washed with 10% w/v aq. Na₂CO₃ solution, with water and dried. Removal of solvent gave 2.68 g of yellow oil.

Dissolution of the preceding oil in diethyl ether, filtration and addition of isopropanolic hydrogen chloride afforded a solid that was collected, rinsed with diethyl ether and dried. The crude salt was recrystallized from absolute ethanol in the presence of several drops of isopropanolic hydrogen chloride, rinsed with diethyl ether and dried to provide 1.25 g (37%) of off-white hydrochloride: mp > 320°C (dec) (lit. mp 315°C (dec) (R.C. Fuson and J.J. Denton, J. Am. Chem. Soc., 63, 654 (1941)); ¹H NMR (DMSO-d₆) δ 8.05 (s, br, 3H), 6.90 (s, 2H), 2.34 (s, 6H), 2.21 (s, 3H) ppm. IR (KBr): 2900 br, 1880 br cm⁻¹; MS (m/z) 149 (M⁺).

### Step 3) 3-tert-Butylamino-4-(2,4,6-trimethyl-benzylamino)-cyclobut-3-ene-1,2-dione

Tetrahydrofuran (10 mL), 2.4.6-trimethylbenzylamine hydrochloride from Example 52, Step 2 (0.610 g, 3.29 mmol), triethylamine (0.334 g, 3.3 mmol) and 3-butoxy-4-tert-butylamino-cyclobut-3-ene-1,2-dione (0.741 g, 3.29 mmol, Example 44) were stirred together at room temperature for approximately 65 hours. After removal of solvent, the residue was triturated with water, with diethyl ether and dried to provide 0.633 g white solid. Three recrystallizations of this material from acetonitrile gave 0.256 g (26%) of title compound as a cream-colored solid: mp 309-310°C dec; ¹H NMR (DMSO-d₆) δ 7.37 (s, 1H), 7.24 (m, 1H), 6.89 (s, 2H), 4.74 (d, 2H), 2.30 (s, 6H), 2.21 (s, 3H), 134 (s, 9H) ppm. IR (KBr): 3280, 1780, 1660 cm⁻¹; MS (m/z) 300 (M⁺). HPLC indicates a major component (99%).

| Elemental analysis for C₁₈H₂₄N₂O₂ | |
|---|---|
| Calc'd | C, 71.97; H, 8.05; N, 9.33. |
| Found | C, 72.05; H, 8.02; N, 9.32. |

### EXAMPLE 53 3-tert-Butylamino-4-(2,6-dichloro-benzylamino)-cyclobut-3-ene-1,2-dione

To 2, 6-dichlorobenzylamine hydrochloride from Example 48, Step 2 (1.06 g, 5.0 mmol) in tetrahydrofuran (10 mL) was added triethylamine (0.505 g, 5.0 mmol) followed by 3-butoxy-4-tert-butylamino-cyclobut-3-ene-1,2-dione (1.13 g, 5.0 mmol, Example 44) and stirring was continued for approximately 16 hours. The reaction mixture was freed of solvent and the residue was triturated with water, with diethyl ether and dried. The white solid thus isolated was crystallized twice from methanol to give 0.623 g (38%) of the title compound as a white solid: mp 265-6°C dec (softens 263°C); ¹H NMR (DMSO-d₆) δ 7.55 (d, 2H), 7.49 (br m, 1H), 7.42 (m, 2H), 5.05 (d, 2H), 1.34 (s, 9H) ppm. IR (KBr): 3185, 1785, 1660 cm⁻¹; MS (m/z) 326/328/330 (M⁺). HPLC indicates a major component (99.9%).

| Elemental analysis for C₁₅H₁₆N₂O₂ | |
|---|---|
| Calc'd | C, 55.06; H, 4.93; N, 8.56. |
| Found | C, 54.70; H, 4.84; N, 8.45. |

### EXAMPLE 54 3-Butoxy-4-(1-ethyl-propylamino)-cyclobut-3-ene-1,2-dione

A solution of 3,4-dibutoxy-3-cyclobut-3-ene-1,2-dione (2.26 g, 10 mmol) and 1-ethylpropylamine (0.872 g, 10 mmol) in tetrahydrofuran (8 mL) was stirred at room temperature for 2.5 hours. The residue remaining after removal of solvent was dissolved in chloroform and the solution was washed with water and dried (anhydrous Na₂SO₄). Removal of solvent gave a waxy solid that was chromatographed (flash, chloroform/hexane) on silica. The solid isolated from the appropriate fractions was recrystallized twice from hexane to yield 0.896 g (37%) of the title compound: mp 65-66°C; ¹H NMR (DMSO-d₆) δ 8.63 and 8.40 (two d, 1H, rotamers), 4.64 (m, 2H), 3.74 and 3.30 (two m, 1H, rotamers), 1.71 (m, 2H), 1.54 (m, 2H), 1.39 (m, 4H), 0.90 (m, 3H), 0.82 (m, 6H) ppm. IR (KBr): 3140, 1790, 1720 cm⁻¹; MS (m/z) 239 (M⁺).

| Elemental analysis for C₁₃H₂₁NO₃ | |
|---|---|
| Calc'd | C, 65.25; H, 8.85; N, 5.85. |
| Found | C, 65.37; H, 9.07; N, 5.87. |

### EXAMPLE 55 3-{[3,4-Dioxo-2-(1,2,2-trimethyl-propylamino)-cyclobut-1-enylamino]-methyl}-5-methyl-benzonitrile

This compound was prepared in a manner similar to Example 50, Step 4 using appropriate starting materials to afford 3-{[3,4-dioxo-2-(1,2,2-trimethyl-propylamino)-cyclobut-1-enylamino]-methyl}-5-methyl-benzonitrile 0.03 ethanolate as a white solid: mp 244-247°C.

### EXAMPLE 56 3-{[2-(1-Ethyl-propylamino)-3,4-dioxo-cyclobut-1-enylamino]-methyl}-5-methyl-benzonitrile

This compound was prepared in a manner similar to Example 50, Step 4 using appropriate starting materials to afford 3-{[2-(1-ethyl-propylamino)-3,4-dioxo-cyclobut-1-enylaminol-methyl}-5-methyl-benzonitrile as a white solid: mp 248-250°C.

### EXAMPLE 57 2-(3,4-Dimethoxy-benzylamino)-4-(1-ethyl-propylamino)-cyclobut-3-ene-1,2-dione

This compound was prepared in a manner similar to Example 51 using appropriate starting materials to afford 3-(3,4-dimethoxy-benzylamino)-4-(1-ethyl-propylamino)-cyclobut-3-ene-1,2-dione as a white solid: mp 223-224°C.

### EXAMPLE 58 3-tert-Butylamino-4-(2,4,6-trimethoxy-benzylamino)-cyclobut-3-ene-1,2-dione

This compound was prepared in a manner similar to Example 47, Step 3 using appropriate starting materials and pyridine as the solvent to afford 3-tert-butylamino-4-(2,4,6-trimethoxy-benzylamino)-cyclobut-3-ene-1,2-dione as a white solid: mp partially melts and resolidifies at 225.5-227.0°C, then decomposes 294-306°C.

### EXAMPLE 59 3-[(2-tert-Butylamino-3,4-dioxo-cyclobut-1-enylamino)-methyl]-5-methyl-benzonitrile

This compound was prepared in a manner similar to Example 45 using appropriate starting materials to afford 3-[(2-tert-butylamino-3,4-dioxo-cyclobut-1-enylamino)-methyl]-5-methyl-benzonitrile as a white solid: mp 253-255°C.

### EXAMPLE 60 3-tert-Butylamino-4-(2,4-dimethoxy-benzylamino)-cyclobut-3-ene-1,2-dione

This compound was prepared in a manner similar to Example 47, Step 3 using appropriate starting materials to afford 3-tert-butylamino-4-(2,4-dimethoxy-benzyl-amino)-cyclobut-3-ene-1,2-dione as a white solid: mp 225-226°C.

### EXAMPLE 61 3-tert-Butylamino-4-(2,4-difluoro-benzylamino)-cyclobut-3-ene-1,2-dione

This compound was prepared in a manner similar to Example 57 using appropriate starting materials to afford 3-tert-butylamino-4-(2,4-difluoro-benzylamino)-cyclobut-3-ene-1,2-dione as a white solid: mp 227-228°C (dec).

### EXAMPLE 62 3-tert-Butylamino-4-(2,6-difluoro-benzylamino)-cyclobut-3-ene-1,2-dione

This compound was prepared in a manner similar to Example 51 using appropriate starting materials to afford 3-tert-butylamino-4-(2,6-difluoro-benzylamino)-cyclobut-3-ene-1,2-dione as an off-white solid: mp 269°C (dec).

### EXAMPLE 63 3-tert-Butylamino-4-(2,3-dimethoxy-benzylamino)-cyclobut-3-ene-1,2-dione

This compound was prepared in a manner similar to Example 51 using appropriate starting materials to afford 3-tert-butylamino-4-(2,3-dimethoxy-benzylamino)-cyclobut-3-ene-1,2-dione as a solid: mp 215-216°C (dec).

### EXAMPLE 64 3-tert-Butylamino-4-(2,5-difluoro-benzylamino)-cyclobut-3-ene-1,2-dione

This compound was prepared in a manner similar to Example 51 using appropriate starting materials to afford 3-tert-butylamino-4-(2,5-difluoro-benzylamino)-cyclobut-3-ene-1,2-dione as an off-white solid: mp 273-274°C (dec).

### EXAMPLE 65 3-tert-Butylamino-4-(3,5-dimethoxy-benzylamino)-cyclobut-3-ene-1,2-dione

This compound was prepared in a manner similar to Example 51 using appropriate starting materials to afford 3-tert-butylamino-4-(3,5-dimethoxy-benzylamino)-cyclobut-3-ene-1,2-dione as a white solid: mp 222-223°C.

### EXAMPLE 66 3-tert-Butylamino-4-(2,3-dimethyl-benzylamino)-cyclobut-3-ene-1,2-dione

This compound was prepared in a manner similar to Example 51 using appropriate starting materials to afford 3-tert-butylamino-4-(2,3-dimethyl-benzylamino)-cyclobut-3-ene-1,2-dione as a white solid: mp 237-239°C.

### EXAMPLE 67 4-[(2-Butylamino-3,4-dioxo-cyclobut-1-enylamino)-methyl]-3-ethyl-benzonitrile

### Step 1) Preparation of 3-ethoxy-4-(2-ethyl-4-cyano-benzylamino)-cyclobut-3-ene-1,2-dione

4-Cyano-2-ethylbenzaldehyde oxime (0.17 g, 0.98 mmol, synthesized by the procedure of Jolad et al., Org. Syntheses, Coll. Vol. V, 139 (1973), Example 72, Step 1) was dissolved in glacial acetic acid. Zinc powder (0.13 g, 2.0 mmol) was added. The slurry was heated at 50°C for 30 minutes, then a second portion of zinc powder (0.13 g, 2.0 mmol) was added. The slurry was heated at 60°C for 30 minutes. The reaction mixture was cooled, diluted with ethanol (30 mL), and filtered through Celite® . The filtrate was treated with 3,4-diethoxy-3-cyclobutene-1,2-dione (0.14 mL, 0.95 mmol), and the solution was left under vacuum (100 mm) overnight. The resulting reddish residue was combined with the crude product from another similar reaction in which 4-cyano-2-ethylbenzaldehyde oxime (0.17 g, 0.98 mmol) was used. The combined residues were triturated six times with 5% ethyl acetate in hexane to give 0.47 g (90%) of a pale pink solid: ¹H NMR (DMSO-d₆) δ 9.28 and 9.05 (two br m, 1H, rotamers), 7.68 (m, 2H); 7.43 (m, 1H), 4.90-4.50 (m, 4H), 2.67 (m, 2H), 1.37 and 1.25 (two t, 3H. rotamers), 1.16 (t, 3H) ppm. MS (m/z) 284 (M⁺).

### Step 2) 4-[(2-Butylamino-3,4-dioxo-cyclobut-1-enylamino)-methyl]-3-ethyl-benzonitrile

The product of Example 67, Step 1 (0.18 g, 0.63 mmol) and n-butylamine (0.15 mL, 1.55 mmol) was placed in absolute ethanol (3.5 mL) and dichloromethane (2 mL). The clear solution was allowed to stand at room temperature for 24 hours. The resulting solid slurry was filtered, rinsed with hexane:ethyl acetate (1:1), and dried to give 0.10 g (50%) of the title compound as a white solid: ¹H NMR (DMSO-d₆) δ 7.70 (overlapping m and br m, 3H), 7.45 (d, 1H), 7.38 (br m, 1H), 4.83 (d, 2H), 3.90 (q, 2H), 2.69 (q, 2H), 1.49 (quintet, 2H), 1.29 (sextet, 2H), 1.17 (t, 3H), 0.88 (t, 3H) ppm. IR (KBr): 3280, 2950, 2200, 1800, 1650 cm⁻¹; MS (m/z) 311 (M⁺).

| Elemental analysis for C₁₈H₂₁N₃O₂ | |
|---|---|
| Calc'd | C, 69.43; H, 6.80; N, 13.49. |
| Found | C, 68.67; H, 6.72; N, 13.33. |

### EXAMPLE 68 3-Ethyl-4-[(2-isopropylamino-3,4-dioxo-cyclobut-1-enylamino)-methyl-benzonitrile

This compound was prepared according to the procedure described in Example 67, Step 2. From the product of Example 67, Step 1 (0.18 g, 0.63 mmol) and isopropylamine (0.13 mL, 1.55 mmol) in absolute ethanol (3.5 mL) and dichloromethane (2 mL) there was obtained 0.17 g (89%) of the title compound as a pale orange solid: ¹H NMR (DMSO-d₆) δ 7.70 (m, 2H), 7.65 (br m, 1H), 7.46 (d, 1H), 7.41 (br m, 1H), 4.83 (d, 2H), 3.30 (m, 1H), 2.69 (q, 2H), 1.19 (overlapping d and t, 9H) ppm. IR (KBr): 3280, 2960, 2200, 1800, 1650 cm⁻¹; MS (m/z) 297 (M⁺).

| Elemental analysis for C₁₇H₁₉N₃O₂ | |
|---|---|
| Calc'd | C, 68.67; H, 6.44; N, 14.13. |
| Found | C, 68.47; H, 6.43; N, 14.21. |

### EXAMPLE 69 3-Ethyl-4-{[(2-(1-ethyl-propylamino)-3,4-dioxo-cyclobut-1-enylamino]-methyl}-benzonitrile

This compound was prepared according to the procedure described in Example 67, Step 2. From the product of Example 67, Step 1 (0.18 g, 0.63 mmol) and 1-ethylpropylamine (0.18 mL, 1.55 mmol) in absolute ethanol (3.5 mL) and dichloromethane (2 mL) there was obtained 0.16 g (76%) of the title compound as an off-white solid: ¹H NMR (DMSO-d₆) δ 7.70 (m, 2H), 7.59 (br m, 1H), 7.46 (d, 1H), 7.23 (br d, 1H), 4.85 (d, 2H), 3.30 (m, 1H), 2.69 (q, 2H), 1.56 (m, 2H), 1.43 (m, 2H), 1.17 (t, 3H), 0.85 (t, 6H) ppm. IR (KBr): 3180, 2970, 2210, 1795, 1650 cm⁻¹; MS (m/z) 325 (M⁺).

| Elemental analysis for C₁₉H₂₃N₃O₂ | |
|---|---|
| Calc'd | C, 70.13; H, 7.12; N, 12.91. |
| Found | C, 69.66; H, 7.06; N, 12.89. |

### EXAMPLE 70 4-[(2-tert-Butylamino-3,4-dioxo-cyclobut-1-enylamino)-methyl]-3-ethyl-benzonitrile

The product of Example 67, Step 1 (0.45 g, 1.58 mmol) in t-butylamine (8 mL) was heated at 80°C under argon for 2 hours. The reaction mixture was cooled to room temperature and concentrated under reduced pressure. The residue was triturated with ethyl acetate to give 0.25 g of a solid, which was further purified by chromatography (silica gel, 3% methanol in dichloromethane. The resulting solid was suspended in 10% ethyl acetate in hexane, filtered, and rinsed with 10% ethyl acetate in hexane to yield 0.18 g (37%) of a pale yellow solid: mp 224-228°C (softens 229°C); ¹H NMR (DMSO-d₆) δ 7.76-7.69 (m, 3H), 7.57 (br s, 1H), 7.47 (br d, 1H), 4.85 (d, 2H), 2.69 (q, 2H), 1.36 (s, 9H), 1.18 (t, 3H) ppm. IR (KBr): 3210, 2980, 2210, 1790, 1650 cm⁻¹; MS (m/z) 311 (M⁺).

| Elemental analysis for C₁₈H₂₁N₃O₂ | |
|---|---|
| Calc'd | C, 69.43; H, 6.80; N, 13.49. |
| Found | C, 69.12; H, 6.79; N, 13.69. |

### EXAMPLE 71 4-{[3,4-Dioxo-2-(1,2,2-trimethyl-propylamino)-cyclobut-1-enylamino]-methyl}-3-ethyl-benzonitrile

### Step 1) Preparation of 4-Cyano-2-ethylbenzaldehyde oxime

4-Cyano-2-ethylbenzaldehyde oxime was prepared by the procedure of Jolad et al., *Org. Syntheses,* Coll. Vol. V, 139 (1973). A mixture of paraformaldehyde (2.8 g, 93 mmol) and hydroxylamine hydrochloride (6 g, 86 mmol) in water (39 mL) was heated in a 500 mL 3-necked flask. When a clear, colorless solution was obtained, sodium acetate trihydrate (11.8 g, 87 mmol) was added. The solution was heated for 15 minutes, then cooled in an ice-salt bath to approximately 5-10°C. To this solution was added copper sulfate (0.97 g, 6.08 mmol), sodium sulfite (0.23 g, 1.82 mmol) and sodium acetate trihydrate (37 g, 272 mmol) in water (42 mL). The resulting green solution was cooled to an internal temperature of approximately 5-10°C. A diazonium salt solution of 4-amino-3-ethylbenzonitrile was previously prepared as follows: A mixture of 4-amino-3-ethylbenzonitrile (8.32 g, 57 mmol), concentrated hydrochloric acid (13.2 mL), ice (23 g), and water (11.6 mL) was cooled in an ice-salt bath. While maintaining the internal temperature below 10°C, a solution of sodium nitrite (4.2 g, 61 mmol) in water (5.8 mL) was added dropwise. The resulting slurry was stirred at 5-10°C for an additional 15 minutes. Then a solution of sodium acetate trihydrate (5.1 g, 37 mmol) in water (8.1 mL) was added. This solution of the diazonium salt was siphoned under the surface of the 10% formaldoxime solution. The reaction mixture was stirred vigorously and allowed to slowly warm to room temperature as the ice-salt bath melted. After 2 hours the reaction mixture was diluted with brine (100 mL), extracted with dichloromethane (2 x 400 mL), and dried (Na₂SO₄). Purification by chromatography (silica gel, hexane:ethyl acetate (7:1)) gave 2.97 g (30%) of a solid: ¹H NMR (DMSO-d₆) δ 11.80 (s, 1H), 8.45 (s, 1H), 8.00-7.60 (m, 3H), 2.90 (m, 2H), 1.20 (m, 3H).

### Step 2) Preparation of 4-Cyano-2-ethylbenzyl alcohol

The product from Example 71, Step 1 (2.9 g, 17 mmol) and 2N hydrochloric acid (142 mL) was stirred at room temperature. Acetone (100 mL) was added to produce a homogeneous solution. After 5 days the reaction mixture was diluted with ethyl acetate (500 mL) and solid sodium chloride was added until the aqueous layer was saturated. After stirring at room temperature for 1 hour, the ethyl acetate layer was separated and dried (Na₂SO₄). Concentration under reduced pressure yielded 7 g of a solid which was chromatographed (silica gel) with hexane, then hexane:ethyl acetate (8:1). This gave 2.0 g (75%) of 4-cyano-2-ethylbenzaldehyde, which was used without further purification. 4-Cyano-2-ethylbenzaldehyde (2.0 g, 13 mmol) in methanol (63 mL) was cooled to 0°C. Solid sodium borohydride (0.48 g, 13 mmol) was added. The reaction mixture was stirred at 0°C for 1 hour. A second portion of sodium borohydride (0.48 g, 13 mmol) was added, and the reaction mixture was stirred at 0°C for 40 minutes. A third portion of sodium borohydride (0.48 g, 13 mmol) was added and the reaction mixture was stirred at 0°C for 20 minutes. The ice bath was then removed and the reaction mixture was stirred at room temperature for 1 hour. Water (28 mL) was then added and the reaction mixture was stirred at room temperature for 24 hours. The solution was then diluted with ethyl acetate (100 mL) and divided into two portions, which were each extracted with ethyl acetate (300 mL) and brine (40 mL). The combined ethyl acetate layers were concentrated under reduced pressure and the resulting residue was chromatographed (silica gel) with hexane:ethyl acetate (7:1), then hexane:ethyl acetate (3:1) to yield 1.53 g (75%) of a solid: ¹H NMR (DMSO-d₆) δ 7.70-7.50 (m, 3H), 5.39 (br m, 1H), 4.59 (s, 2H), 2.60 (q, 2H), 1.18 (t, 3H).

### Step 3) Preparation of N-(4-Cyano-2-ethylbenzyl)phthalimide

The product of Example 71, Step 2 (0.95 g, 5.9 mmol), phthalimide (1.05 g, 7.1 mmol), triphenylphosphine (1.85 g, 7.1 mmol), and tetrahydrofuran (39 mL) were mixed and cooled to 0°C in an ice bath. Diethylazodicarboxylate (1.09 mL, 6.9 mmol) was added dropwise. The reaction mixture was allowed to warm to room temperature as the ice bath melted. After 24 hours the reaction mixture was concentrated under reduced pressure and the resulting residue was chromatographed (silica gel) with hexane:ethyl acetate (5:1), hexane:ethyl actate (3:1), then hexane:ethyl acetate (2:1) to yield 1.85 g (108%) of a solid: ¹H NMR (DMSO-d₆) δ 8.00-7.80 (m, 4H), 7.70 (s, 1H), 7.59 (d, 1H), 7.30 (d, 1H), 4.86 (s, 2H), 2.81 (q, 2H), 1.22 (t, 3H).

### Step 4) Preparation of 4-Cyano-2-ethylbenzylamine

The product of Example 71, Step 3 (1.73 g, 6.0 mmol), 35% hydrazine (1.07 mL, 12 mmol), and absolute ethanol (105 mL) were mixed and heated at 65°C under argon for 3 hours. Then the reaction was heated at 85°C for 5 hours. The reaction mixture was concentrated under reduced pressure, resuspended in absolute ethanol (35 mL), filtered and rinsed with absolute ethanol (2 x 30 mL). Concentration under reduced pressure yielded a solid, which was suspended in ethyl acetate (100 mL) and filtered. The filtrate was concentrated under reduced pressure to give 0.77 g (81%) of a wet solid: ¹H NMR (CDCl₃) δ 7.50 (m, 3H), 3.94 (s, 2H), 2.70 (m, 2H), 1.43 (br m, 2H), 1.21 (t, 3H).

### Step 5) 4-{[3,4-Dioxo-2-(1,2,2-trimethyl-propylamino)-cyclobut-1-enylamino]-methyl}-3-ethyl-benzonitrile

The product of Example 71, Step 4 (0.21 g, 1.3 mmol) was placed in absolute ethanol (5.5 mL). (R)-3-Ethoxy-4-(1,2,2-trimethyl-propylamino)-cyclobut-3-ene-1,2-dione (0.3 g, 1.3 mmol, prepared in a manner similar to Example 48 from 3,4-diethoxy-3-cyclobutene-1,2-dione and (R)-2-amino-3,3-dimethylbutane in ethanol) was added, followed by dichloromethane (5 mL). The clear solution was stirred at room temperature for 5 days. The reaction mixture was concentrated under reduced pressure and the resulting solid was chromatographed (silica gel) with 1% methanol in dichloromethane, then 3% methanol in dichloromethane. This gave 0.19 g (43%) of the (R) isomer of the title compound as a light tan solid: mp 208-212°C; [α]²⁵_{D} = +13.44° (8.6 mg/mL, DMSO) ¹H NMR (DMSO-d₆) δ 7.71 (m, 2H), 7.60 (br m, 1H), 7.48 (br d, 1H), 7.27 (br d, 1H), 4,85 (m, 2H), 3.90 (m, 1H), 2.69 (q, 2H), 1.17 (t, 3H), 1.10 (d, 3H), 0.86 (s, 9H) ppm. IR (KBr): 3200, 2970, 2230, 1800, 1650 cm⁻¹; MS (m/z) 339 (M⁺).

| Elemental analysis for C₂₀H₂₅N₃O₂ | |
|---|---|
| Calc'd | C, 70.77; H, 7.42; N, 12.38. |
| Found | C, 70.05; H, 7.29; N, 12.13. |

### EXAMPLE 72 3-(2-Chloro-6-methyl-benzylamino)-4-(1,2,2-trimethyl-propylamino)-cyclobut-3-ene-1,2-dione

This compound was prepared according to the procedure described in Example 71, Step 5. From (R)-3-ethoxy-4-(1,2,2-trimethyl-propylamino)-cyclobut-3-ene-1,2-dione (0.3 g, 1.3 mmol) and 2-chloro-6-methylbenzylamine (0.2 g, 1.3 mmol) in absolute ethanol (2.7 mL) there was obtained a solid, which was diluted with acetonitrile (4 mL), filtered, rinsed with acetonitrile (2 x 2 mL), and dried. This gave 0.42 g (96%) of the (R) isomer of the title compound as a white solid: mp 288-292°C (dec); [α]²⁵_{D} = +24.92° (10.0 mg/mL, DMSO); ¹H NMR (DMSO-d₆) δ 7.40-7.20 (m, 4H), 7.15 (br d, 1H), 4.93 (m, 2H), 3.90 (m, 1H), 2.40 (s, 3H), 1.09 (d, 3H), 0.85 (s, 9H) ppm. IR (KBr): 3150, 2980, 2230, 1800, 1650 cm⁻¹; MS (m/z) 334/336 (M⁺).

| Elemental analysis for C₁₈H₂₃ClN₂O₂ | |
|---|---|
| Calc'd | C, 64.57; H, 6.92; N, 8.37. |
| Found | C, 64.28; H, 6.76; N, 8.16. |

### EXAMPLE 73 (R)-3-Chloro-4-{[3,4-dioxo-2-(1,2,2-trimethyl-propylamino)-cyclobut-1-enylamino]-methy}-benzonitrile

2-Chloro-4-cyano-benzylamine (0.30 g, 1.80 mmol, Example 45, Step 3) and (R)-3-ethoxy-4-(1,2,2-trimethyl-propylamino)-cyclobut-3-ene-1,2-dione (0.406 g, 1.80 mmol) were stirred in ethanol (10 mL) at 70°C for 18 hours. The reaction was cooled and diluted with diethyl ether. Filtration afforded 0.49 g (79%) of (R)-3-chloro-4-{[3,4-dioxo-2-(1,2,2-trimethyl-propylamino)-cyclobut-1-enylamino]-methyl}-benzonitrile which was collected as a white solid: mp 237-241°C; [α]²⁵_{D} = +27.30° (10.99 mg/mL, DMSO); ¹H NMR (DMSO-d₆) δ 8.10 (d, 1H), 7.89 (dd, 1H), 7.68 (m, 1H), 7.69 (d, 1H), 7.36 (br d, 1H), 4.87 (m, 2H), 3.90 (m, 1H), 1.12 (d, 3H), 0.86 (s, 9H), ppm. IR (KBr): 3100, 2950, 2250, 1800 cm⁻¹. MS (m/z) 345/347 (M⁺).

| Elemental analysis for C₁₈H₂₀ClN₃O₂ | |
|---|---|
| Calc'd | C, 62.52; H, 5.83; N, 12.15. |
| Found | C, 62.78; H, 6.17; N, 11.90. |

### EXAMPLE 74 3-tert-Butylamino-4-(2-chloro-6-methyl-benzylamino)-cyclobut-3-ene-1,2-dione

A mixture of 3,4-diethoxy-3-cyclobutene-1,2-dione (5 g, 29 mmol) and 2-chloro-6-methylbenzylamine (4.57 g, 29 mmol) in absolute ethanol (147 mL) was allowed to stand at room temperature for 4 days. The reaction mixture was concentrated under reduced pressure and suspended in dichloromethane (200 mL). The slurry was stirred at room temperature for 18 hours, filtered, rinsed with dichloromethane, and concentrated under reduced pressure to give 6.42 g (78%) of a solid. A portion of this solid, 3-(2-chloro-6-methyl-benzylamino)-4-ethoxy-cyclobut-3-ene-1,2-dione, (0.4 g, 1.43 mmol) was placed in t-butylamine (4 mL) and dichloromethane (4 mL) and allowed to stand at room temperature for 6 days. The solid was filtered, rinsed several times with ethyl acetate, and dried. This gave 0.31 g (71%) of the title compound as a white solid: mp 254-257°C; ¹H NMR (DMSO-d₆) δ 7.46 (br s, 1H), 7.42 (br t, 1H), 7.38-7.22 (m, 3H), 4.93 (d, 2H), 2.40 (s, 3H), 1.34 (s, 9H) ppm. IR (KBr): 3200, 2960, 1790, 1650 cm⁻¹; MS (m/z) 306/308 (M⁺).

| Elemental analysis for C₁₆H₁₉ClN₂O₂ | |
|---|---|
| Calc'd | C, 62.64; H, 6.24; N, 9.13. |
| Found | C, 62.40; H, 6.29; N, 9.08. |

### EXAMPLE 75 3-(2-chloro-6-methyl-benzylamino)-4-(1,1-dimethyl-propylamino)-cyclobut-3-ene-1,2-dione

3-(2-Chloro-6-methyl-tenzylamino)-4-ethoxy-cyclobut-3-ene-1,2-dione (0.4 g, 1.43 mmol) was placed in 1,1-dimethylpropylamine (5 mL) and dichloromethane (2 mL) and refluxed for 14 hours. The solvent was removed under reduced pressure to give 0.37 g of a solid, which was placed in isopropanol, filtered and concentrated under reduced pressure. This was repeated with ethyl acetate. The residue was placed in 5% ethanol in water, filtered, rinsed with water, then ethyl acetate, and dried in vacuo (0.4 mm, 70°C) to yield 0.27 g (59%) of the title compound as a solid: mp 224-226°C (softens 192°C); ¹H NMR (DMSO-d₆) δ 7.44 (br t, 1H), 7.39-7.22 (m, 4H), 4.93 (d, 2H), 2.40 (s, 3H), 1.65 (q, 2H), 1.28 (s, 6H), 0.80 (t, 3H) ppm. IR (KBr): 3200, 2970, 1800, 1650 cm⁻¹; MS (m/z) 320/322 (M⁺).

| Elemental analysis for C₁₇H₂₁ClN₂O₂ | |
|---|---|
| Calc'd | C, 63.65; H, 6.60; N, 8.73. |
| Found | C, 63.37; H, 6.56; N, 8.58. |

### EXAMPLE 76 3-Chloro-4-{[2-(1,1-dimethyl-propylamino)-3,4-dioxo-cyclobut-1-enylamino]-methyl}-5-methyl-benzonitrile

### Step 1) Preparation of 3-(2-chloro-4-cyano-6-methyl-benzylamino)-4-ethoxy-cyclobut-3-ene-1,2-dione

To 2-chloro-4-cyano-6-methylbenzaldehyde oxime (1.2 g, 6.2 mmol, prepared according to the procedure of Example 71, Step 1) in glacial acetic acid (12.3 mL) was added zinc powder (1.6 g, 24 mmol). The slurry was heated to boiling. When the bubbling subsided, a second portion of zinc powder (1.6 g, 24 mmol) was added and the slurry was heated to boiling. When the reaction had cooled to room temperature, it was diluted with absolute ethanol, filtered through Celite®, rinsed with absolute ethanol, and concentrated under reduced pressure. The resulting residue was mixed with 3,4-diethoxy-3-cyclobutene-1,2-dione (0.91 mL, 6.2 mmol) and absolute ethanol, then allowed to stand at room temperature for 18 hours. The solid precipitate was filtered and rinsed with ethyl acetate to give 0.51 g of a solid. This solid was dissolved in dichloromethane, filtered, rinsed with dichloromethane, and the filtrate concentrated under reduced pressure to give 0.19 g of a solid: ¹H NMR (DMSO-d₆) δ 8.97 and 8.74(br m, 1H, rotamers), 7.92 (s, 1H), 7.74 (s, 1H), 4.93 (br m, 1H), 4.78-4.60 (br m, 3H), 2.43 (s, 3H), 1.36 (br m, 3H) ppm. MS (m/z) 304/306 (M⁺).

### Step 2) 3-Chloro-4-{[2-(1,1-dimethyl-propylamino)-3,4-dioxo-cyclobut-1-enylamino]-methyl}-5-methyl-benzonitrile

The product from Example 76, Step 1 (0.24 g, 0.79 mmol) was placed in 1,1-dimethylpropylamine (5 mL) and dichloromethane (3 mL) and refluxed for 8 hours. The solvent was removed under reduced pressure to give a solid, which was placed in ethyl acetate, filtered, rinsed with ethyl acetate, and dried in vacuo (0.4 mm, 70°C). This gave 0.17 g (62%) of the title compound as a solid: mp 258-262°C (dec); ¹H NMR (DMSO-d₆) δ 7.99 (s, 1H), 7.78 (s, 1H), 7.51 (br t, 1H), 7.35 (s, 1H), 4.98 (d, 2H), 2,46 (s, 3H), 1.66 (q, 2H), 1.29 (s, 6H), 0.80 (t, 3H) ppm. IR (KBr): 3200, 2980, 2200, 1800, 1650 cm⁻¹; MS (m/z) 345/347 (M⁺).

| Elemental analysis for C₁₈H₂₀ClN₃O₂ | |
|---|---|
| Calc'd | C, 62.52; H, 5.83; N, 12.15. |
| Found | C, 62.74; H, 5.87; N, 12.15. |

### EXAMPLE 77 3-Chloro-4-{[3,4-dioxo-2-(1,2,2-trimethyl-propylamino)-cyclobut-1-enylamino]-methyl}-5-methyl-benzonitrile

The product from Example 76, Step 1 (0.19 g, 0.62 mmol) and (R)-2-amino-3,3-dimethylbutane (4.7 mL of a 0.2 M solution in absolute ethanol, 0.94 mmol) was stirred at room temperature for 2 days. The slurry was filtered, rinsed with acetonitrile (3 x 3 mL), and dried. This gave 0.15 g (67%) of the (R) isomer of the title compound as a white solid: mp >300°C; ¹H NMR (DMSO-d₆) δ 7.98 (s, 1H), 7.77 (s, 1H), 7.38 (br m, 1H), 7.18 (br d, 1H), 4.97 (m, 2H), 3.90 (m, 1H), 2.46 (s, 3H), 1.08 (d, 3H), 0.84 (s, 9H) ppm. IR (KBr): 3180, 2980, 2250, 1800, 1640 cm⁻¹; MS (m/z) 359/361 (M⁺).

| Elemental analysis for C₁₉H₂₂ClN₃O₂ ^{.}0.04 CH₂Cl₂ | |
|---|---|
| Calc'd | C, 62.95; H, 6.13; N, 11.57. |
| Found | C, 62.18; H, 6.09; N, 11.28. |

### EXAMPLE 78 3-(4-Bromo-2,6-dimethyl-benzylamino)-4-(1,2,2-trimethyl-propylamino)-cyclobut-3-ene-1,2-dione

This compound was prepared according to the procedure described in Example 76. From 4-bromo-2,6-dimethylbenzaldehyde oxime (1.13 g, 4.95 mmol), two portions of zinc powder (1.3 g, 20 mmol), glacial acetic acid (19.8 mL), and subsequently 3,4-diethoxy-3-cyclobutene-1,2-dione (0.73 mL, 4.94 mmol) in absolute ethanol (25 mL) there was obtained after chromatography on silica gel (hexane:ethyl acetate (1:0.3), 3% methanol in dichloromethane) 1.11 g (66%) of a solid. From a portion of this solid, 3-(4-bromo-2,6-dimethyl-benzylamino)-4-ethoxy-cyclobut-3-ene-1,2-dione, (0.4 g, 1.18 mmol) and (R)-2-amino-3,3-dimethylbutane (20 mL of a 0.2 M solution in absolute ethanol, 4.0 mmol) and dichloromethane (15 mL) there was obtained after 4 days at room temperature a solid, which was filtered, rinsed with absolute ethanol, and dried (0.4 mm, 40°C). This gave 0.23 g (50%) of the (R) isomer of the title compound as a white solid: mp >300°C; ¹H NMR (DMSO-d₆) δ 7.32 (s, 2H), 7.18 (br m, 1H), 7.08 (br d, 1H), 4.76 (d, 2H), 3.89 (m, 1H), 2.35 (s, 6H), 1.09 (d, 3H), 0.85 (s, 9H) ppm. IR (KBr): 3160, 2950, 1800, 1650 cm⁻¹; MS (m/z) 392/394 (M⁺).

| Elemental analysis for C₁₉H₂₅BrN₂O₂ | |
|---|---|
| Calc'd | C, 58.02; H, 6.41; N, 7.12. |
| Found | C. 57.91; H. 6.25; N, 7.00. |

### EXAMPLE 79 3-(4-Bromo-2,6-dimethyl-benzylamino)-4-tert-butylamino-cyclobut-3-ene-1,2-dione

A solution of 3-(4-bromo-2,6-dimethyl-benzylamino)-4-ethoxy-cyclobut-3-ene-1,2-dione (0.49 g, 1.45 mmol), t-butylamine (26 mL) and dichloromethane (8 mL) was allowed to stand at room temperature for 18 hours, then refluxed for 8 hours. The resulting solid was filtered, rinsed with acetonitrile, and dried (0.4 mm, 40°C). This gave 0.33 g (62%) of the title compound as an off-white solid: mp 267-271°C (dec); ¹H NMR (DMSO-d₆) δ 7.40 (br s, 1H), 7.32 (s, 2H), 7.30 (br t, 1H), 4.76 (d, 2H), 2.34 (s, 6H), 1.34 (s, 9H) ppm. IR (KBr): 3200, 2980, 1790, 1650 cm⁻¹; MS (m/z) 364/366 (M⁺).

| Elemental analysis for C₁₇H₂₁BrN₂O₂ | |
|---|---|
| Calc'd | C, 55.90; H, 5.80; N, 7.67. |
| Found | C, 55.65; H, 5.63; N, 7.63. |

### EXAMPLE 80 3-(4-Bromo-2,6-dimethyl-benzylamino)-4-(1,1-dimethyl-propylamino)-cyclobut-3-ene-1,2-dione

This compound was prepared according to the procedure described in Example 75. From 3-(4-bromo-2,6-dimethyl-benzylamino)-4-ethoxy-cyclobut-3-ene-1,2-dione (0.2 g, 0.59 mmol), 1,1-dimethylpropylamine (5 mL), acetonitrile (10 mL) and dichloromethane (10 mL) there was obtained after 7 days at room temperature, followed by refluxing for 8 hours and removal of solvent under reduced pressure 0.31 g of a solid. This was placed in 5% ethanol in water, filtered, rinsed with water, then ethyl acetate and dried (0.4 mm, 70°C) to yield 0.16 g (71%) of the title compound as a solid: mp 246-250°C (dec) (softens 240°C); ¹H NMR (DMSO-d₆) δ 7.38-7.30 (m with overlapping s at δ 7.32, 3H), 7.26 (br s, 1H), 4.76 (d, 2H), 2.34 (s, 6H), 1.65 (q, 2H), 1.28 (s, 6H), 0.80 (t, 3H) ppm. IR (KBr): 3200, 2980, 1800, 1650 cm⁻¹; MS (m/z) 378/380 (M⁺).

| Elemental analysis for C₁₈H₂₃BrN₂O₂ | |
|---|---|
| Calc'd | C, 57.00; H, 6.11; N, 7.39. |
| Found | C. 56.55; H, 6.08; N, 7.34. |

### EXAMPLE 81 3-(2-Chloro-4,6-dimethyl-benzylamino)-4-(1,2,2-trimethyl-propylamino)-cyclobut-3-ene-1,2-dione

This compound was prepared according to the procedure described in Example 76. From 2-chloro-4,6-dimethylbenzaldehyde oxime (6.63 g, 36 mmol), zinc powder (9.4 g, 144 mmol), glacial acetic acid (72 mL), and 3,4-diethoxy-3-cyclobutene-1,2-dione (5.3 mL, 36 mmol) in absolute ethanol (180 mL) there was obtained after chromatography on silica gel (hexane:ethyl acetate, then 5% methanol in dichloromethane) followed by trituation with 10% ethyl acetate in hexane 5.32 g (50 *%)* of a solid. From a portion of this solid, 3-(2-chloro-4,6-dimethyl-benzylamino)-4-ethoxy-cyclobut-3-ene-1,2-dione, (0.4 g, 1.36 mmol) and (R)-2-amino-3,3-dimethylbutane (8.2 mL of a 0.2 M solution in absolute ethanol, 1.64 mmol) and dichloromethane (3 mL) there was obtained after 4 days at room temperature a solid, which was filtered, rinsed with ethyl acetate, and dried. This gave 0.36 g (76%) of the (R) isomer of the title compound as a white solid: mp >300°C; ¹H NMR (DMSO-d₆) δ 7.26 (br s, 1H), 7.19 (s, 1H), 7.14 (br d, 1H), 7.05 (s, 1H), 4.88 (d, 2H), 3.90 (m, 1H), 2.35 (s, 3H), 2.26 (s, 3H), 1.08 (d, 3H), 0.84 (s, 9H) ppm. IR (KBr): 3170, 2970, 1800, 1650 cm⁻¹; MS (m/z) 348/350 (M⁺).

| Elemental analysis for C₁₉H₂₅ClN₂O₂ | |
|---|---|
| Calc'd | C, 65.41; H, 7.22; N, 8.03. |
| Found | C, 64.55; H, 7.15; N, 7.86. |

### EXAMPLE 82 3-tert-Butylamino-4-(2-chloro-4,6-dimethyl-benzylamino)-cyclobut-3-ene-1,2-dione

This compound was prepared according to the procedure described in Example 79. From, 3-(2-chloro-4,6-dimethyl-benzylamino)-4-ethoxy-cyclobut-3-ene-1,2-dione (0.40 g, 1.36 mmol), t-butylamine (2.7 mL) and dichloromethane (1 mL) there was obtained after 4 days at room temperature a solid which was filtered, rinsed with ethyl acetate and dried. This gave 0.28 g (64%) of the title compound as a white solid: mp 292-294°C; ¹H NMR (DMSO-d₆) δ 7.46 (br m, 1H), 7.38 (br t, 1H), 7.19 (s, 1H), 7.06 (s, 1H), 4.87 (d, 2H), 2.35 (s, 3H), 2.26 (s, 3H), 1.34 (s, 9H) ppm. IR (KBr): 3200, 2980, 1790, 1650 cm⁻¹; MS (m/z) 320/322 (M⁺).

| Elemental analysis for C₁₇H₂₁ClN₂O₂ | |
|---|---|
| Calc'd | C, 63.65; H, 6.60; N, 8.73. |
| Found | C, 63.55; H, 6.61; N, 8.93. |

### EXAMPLE 83 3-(2-Chloro-4,6-dimethyl-benzylamino)-4-(1,1-dimethyl-propylamino)-cyclobut-3-ene-1,2-dione

This compound was prepared according to the procedure described in Example 75. From 3-(2-chloro-4,6-dimethyl-benzylamino)-4-ethoxy-cyclobut-3-ene-1,2-dione (0.40 g, 1.36 mmol), 1,1-dimethylpropylamine (2.7 mL) and dichloromethane (1 mL) there was obtained after 4 days at room temperature a solution which was concentrated under reduced pressure to yield 0.34 g of a solid. This solid was washed with isopropanol, then hexane, and dried (0.4 mm, 70°C) to give 0.12 g (26%) of the title compound as a white solid: mp 265-271°C (dec); ¹H NMR (DMSO-d₆) δ 7.41 (t, 1H), 7.32 (s, 1H), 7.19 (s, 1H), 7.06 (s, 1H), 4.89 (d, 2H), 2.35 (s, 3H), 2.26 (s, 3H), 1.65 (q 2H), 1.28 (s, 6H), 0.80 (t, 3H) ppm. IR (KBr): 3190, 2980, 1800, 1650 cm⁻¹; MS (m/z) 334/336 (M⁺).

| Elemental analysis for C₁₈H₂₃ClN₂O₂ | |
|---|---|
| Calc'd | C, 64.57; H, 6.92; N, 8.37. |
| Found | C, 63.81; H, 7.26; N, 8.48. |

### EXAMPLE 84 3-(2-Chloro-4,6-dimethyl-benzylamino)-4-(2,2,3,3,3-pentafluoro-propylamino)-cyclobut-3-ene-1,2-dione

From a solution of 3-(2-chloro-4,6-dimethyl-benzylamino)-4-ethoxy-cyclobut-3-ene-1,2-dione (0.30 g, 1.02 mmol), 2,2,3,3,3-pentafluoropropylamine (2.4 mL), absolute ethanol (2 mL), and dichloromethane (2 mL) there was obtained after 6 days at room temperature a solution which was concentrated under reduced pressure and triturated with dichloromethane to give 0.10 g (25%) of the title compound as a white solid: mp 273-275°C; ¹H NMR (DMSO-d₆) δ 7.64 (br m, 1H), 7.55 (br m, 1H), 7.19 (s, 1H), 7.05 (s, 1H). 4.87 (d, 2H), 4.43 (dt, 2H), 2.35 (s, 3H), 2.25 (s, 3H) ppm. IR (KBr): 3200, 2980, 1810, 1670 cm⁻¹; MS (m/z) 396/398 (M⁺).

| Elemental analysis for C₁₆H₁₄ClF₅N₂O₂ | |
|---|---|
| Calc'd | C, 64.57; H, 6.92; N, 8.37. |
| Found | C, 63.81; H, 7.26; N, 8.48. |

### EXAMPLE 85 2-Chloro-4-{[3,4-dioxo-2-(1,2,2-trimethyl-propylamino)-cyclobut-1-enylamino]-methyl}-benzonitrile

This compound was prepared according to the procedure described in Example 76, Step 1. From 3-chloro-4-cyanobenzaldehyde oxime (1.26 g, 6.98 mmol), two portions of zinc powder (1.83 g, 28 mmol), glacial acetic acid (13.9 mL), and 3,4-diethoxy-3-cyclobutene-1,2-dione (1.0 mL, 6.76 mmol) in absolute ethanol there was obtained after chromatography on silica gel (hexane:ethyl acetate (1:0.3), 3% methanol in dichloromethane) 1.64 g (81%) of a solid. Following the procedure described in Example 77, a portion of this solid, 3-(3-chloro-4-cyano-benzylamino)-4-ethoxy-cyclobut-3-ene-1,2-dione (0.4 g, 1.38 mmol), (R)-2-amino-3,3-dimethylbutane (20 mL of a 0.2 M solution in absolute ethanol, 4.0 mmol), and dichloromethane (15 mL) was allowed to stand for 4 days at room temperature. The resulting solid was filtered, rinsed with absolute ethanol, and dried (0.4 mm, 40°C). This gave 0.25 g (52%) of the (R) isomer of the title compound as a white solid: mp 298-300°C (dec); [α]²⁵_{D} = +27.91° (10.0 mg/mL, DMSO); ¹H NMR (DMSO-d₆) δ 7.99 (d, 1H), 7.71 (overlapping s and br m, 2H), 7.49 (d, 1H), 7.33 (br d, 1H), 4.81 (m, 2H), 3.93 (m, 1H), 1.11 (d, 3H), 0.86 (s, 9H) ppm. IR (KBr): 3200, 2960, 2200, 1800, 1650 cm⁻¹; MS (m/z) 345/347 (M⁺).

| Elemental analysis for C₁₈H₂₀ClN₃O₂ | |
|---|---|
| Calc'd | C, 62.52; H, 5.83; N, 12.15. |
| Found | C, 62.38; H, 5.64; N, 12.00. |

### EXAMPLE 86 4-[(2-tert-Butylamino-3,4-dioxo-cyclobut-1-enylamino)-methyl]-2-chloro-benzonitrile

This compound was prepared according to the procedure described in Example 79. From 3-(3-chloro-4-cyano-benzylamino)-4-ethoxy-cyclobut-3-ene-1,2-dione (0.68 g, 2.34 mmol), t-butylamine (6 mL) and dichloromethane (6 mL) there was obtained after 4 days at room temperature a solid which was filtered, rinsed with acetonitrile and dried (0.4 mm, 40°C). This gave 0.22 g (30%) of the title compound as a yellow solid: mp 276-278°C (dec); ¹H NMR (DMSO-d₆) δ 7.99 (d, 1H), 7.84 (br t, 1H), 7.72 (d, 1H), 7.61 (br s, 1H), 7.49 (dd, 1H), 4.81 (d, 2H), 1.36 (s, 9H) ppm. IR (KBr): 3220, 2950, 2200, 1795, 1650 cm⁻¹; MS (m/z) 317/319 (M⁺).

| Elemental analysis for C₁₆H₁₆ClN₃O₂ | |
|---|---|
| Calc'd | C, 60.48; H, 5.08; N, 13.22. |
| Found | C, 60.66; H, 5.02; N, 13.11. |

### EXAMPLE 87 2-Chloro-4-{[2-(1,1-dimethyl-propylamino]-3,4-dioxo-cyclobut-1-enylamino)-methyl}-benzonitrile

From a solution of 3-(1,1-dimethypropylamino)-4-ethoxy-cyclobut-3-ene-1,2-dione (0.51 g, 2.41 mmol) and 3-chloro-4-cyanobenzylamine (3.65 mmol) in absolute ethanol there was obtained after 24 hours at room temperature and followed by refluxing for 8 hours a solid, which was filtered, rinsed with ethyl acetate, then hexane, and dried. This gave 0.30 g of a solid which was suspended in 5% ethanol in water, filtered, and rinsed successively with 5% ethanol in water, ethyl acetate, then hexane, and dried (0.4 mm, room temperature) to give 0.26 g (32%) of the title compound as a pale yellow solid: mp 267-271°C; ¹H NMR (DMSO-d₆) δ 7.99 (d, 1H), 7.85 (br t, 1H), 7.72 (d, 1H), 7.49 (dd, 1H), 7.47 (br s, 1H), 4.81 (d, 2H), 1.67 (q, 2H), 1.30 (s, 6H), 0.82 (t, 3H) ppm. IR (KBr): 3220, 2980, 2250, 1800, 1660 cm⁻¹; MS (m/z) 332/334 ([M+H]⁺).

| Elemental analysis for C₁₇H₁₈ClN₃O₂ | |
|---|---|
| Calc'd | C, 61.54; H, 5.47; N, 12.66. |
| Found | C, 60.91; H, 5.21; N, 12.54. |

### EXAMPLE 88 4-{[3,4-Dioxo-2-(1,2,2-trimethyl-propylamino)-cyclobut-1-enylamino]-methyl}-2-ethyl-benzonitrile

This compound was prepared according to the procedure described in Example 67, Step 1. From 3-ethyl-4-cyanobenzaldehyde oxime (0.88 g, 5.05 mmol), zinc powder (1.32 g, 20 mmol), glacial acetic acid (10 mL), and 3,4-diethyoxy-3-cyclobutene-1,2-dione (0.75 mL, 5.07 mmol) in absolute ethanol (100 mL) there was obtained after chromatography on silica gel (hexane:ethyl acetate (1:1), 5% methanol in dichloromethane) 0.64 g (45%) of a solid. Following the procedure described in Example 77, a portion of this solid, 3-ethoxy-4-(3-ethyl-4-cyano-benzylamino)-cyclobut-3-ene-1,2-dione (0.38 g, 1.34 mmol), (R)-2-amino-3,3-dimethylbutane (10 mL of a 0.2 M solution in absolute ethanol, 2.0 mmol), and dichloromethane was allowed to stand for 24 hours at room temperature. The resulting solid was filtered, rinsed with acetonitrile (5 x 1 mL) and dried. This gave 0.22 g (48%) of the (R) isomer of the title compound as a white solid: mp 251-253°C; [α]²⁵_{D} = +28.48° (4.2 mg/mL, DMSO); ¹H NMR (DMSO-d₆) δ 7.79 (d, 1H), 7.68 (br m, 1H), 7.44 (s, 1H), 7.34 (d, 1H), 7.28 (br d, 1H), 4.78 (m, 2H), 3.91 (br m, 1H), 2.79 (q, 2H), 1.21 (t, 3H), 1.10 (d, 3H), 0.85 (s, 9H) ppm. IR (KBr): 3210, 2980, 2210, 1790, 1650 cm⁻¹; MS (m/z) 339 (M⁺).

| Elemental analysis for C₂₀H₂₅N₃O₂ | |
|---|---|
| Calc'd | C, 70.77; H, 7.42; N, 12.38. |
| Found | C, 70.60; H, 7.37; N, 12.40. |

### EXAMPLE 89 4-[(2-tert-Butylamino-3,4-dioxo-cyclobut-1-enylamino)-methyl]-2-ethyl-benzonitrile

This compound was prepared according to the procedure described in Example 79. From 3-ethoxy-4-(3-ethyl-4-cyano-benzylamino)-cyclobut-3-ene-1,2-dione (0.28 g, 0.98 mmol), t-butylamine (2 mL), and dichloromethane there was obtained after 24 hours at room temperature a solid which was filtered, rinsed with acetonitrile (5 x 1 mL) and dried to give 0.16 g (52%) of the title compound as a pale yellow solid: mp 239-242°C; ¹H NMR (DMSO-d₆) δ 7.83 (br m, 1H), 7.79 (d, 1H), 7.58 (br s, 1H), 7.45 (s, 1H), 7.34 (dd, 1H), 4.78 (d, 2H), 2.79 (q, 2H), 1.35 (s, 9H), 1.22 (t, 3H) ppm. IR (KBr): 3300, 3240, 2950, 2200, 1780, 1650 cm⁻¹; MS (m/z) 311 (M⁺).

| Elemental analysis for C₁₈H₂₁N₃O₂ | |
|---|---|
| Calc'd | C, 69.43; H, 6.80; N, 13.49. |
| Found | C, 68.64; H, 6.89; N, 13.51. |

### EXAMPLE 90 3-(4-Bromo-2-ethyl-benzylamino)-4-(1,2,2-trimethy]-propylamino)-cyclobut-3-ene-1,2-dione

This compound was prepared according to the procedure described in Example 76. From 4-bromo-2-ethylbenzaldehyde oxime (1.67 g, 7.32 mmol), two portions of zinc powder (1.9 g, 29 mmol), glacial acetic acid (14.6 mL), and 3,4-diethoxy-3-cyclobutene-1,2-dione (1.08 mL, 7.31 mmol) in absolute ethanol (37 mL) there was obtained after chromatography on silica gel (hexane:ethyl acetate (1:0.3), 5% methanol in dichloromethane) and trituation with 10% ethyl acetate in hexane 1.08 g (44%) of a solid. From a portion of this solid, 3-(4-bromo-2-ethyl-benzylamino)-4-ethoxy-cyclobut-3-ene-1,2-dione, (0.28 g, 0.83 mmol) and (R)-2-amino-3,3-dimethylbutane (20 mL of a 0.2 M solution in absolute ethanol, 4.0 mmol) and dichloromethane (2 mL) there was obtained after 4 days at room temperature a solid, which was filtered, rinsed with ethyl acetate, and dried (0.4 mm, 40°C). This gave 0.24 g (73%) of the (R) isomer of the title compound as a white solid: mp 217-224°C; [α]²⁵_{D} = +10.52° (9.51 mg/mL, DMSO); ¹H NMR (DMSO-d₆) δ 7.50 (br s, 1H), 7.46-7.40 (m with overlapping s at δ 7.41, 2H), 7.26 (d, 1H), 7.19 (br d, 1H), 4.74 (d, 2H), 3.90 (m, 1H), 2.64 (q, 2H), 1.15 (t, 3H), 1.09 (d, 3H), 0.85 (s, 9H) ppm. IR (KBr): 3170, 2980, 1800, 1650 cm⁻¹; MS (m/z) 392/394 (M⁺).

| Elemental analysis for C₁₉H₂₅BrN₂O₂ | |
|---|---|
| Calc'd | C, 58.02; H, 6.41; N, 7.12. |
| Found | C, 57.58; H, 6.35; N, 7.10. |

### EXAMPLE 91 3-(4-Bromo-2-ethyl-benzylamino)-4-tert-butylamino-cyclobut-3-ene-1,2-dione

This compound was prepared according to the procedure described in Example 79. From 3-(4-bromo-2-ethyl-benzylamino)-4-ethoxy-cyclobut-3-ene-1,2-dione (0.40 g, 1.18 mmol), t-butylamine (5 mL) and dichloromethane (3 mL) there was obtained after 4 days at room temperature a solid which was filtered, rinsed with ethyl acetate, and dried (0.4 mm, 70°C). This gave 0.16 g (37%) of the title compound as a pale yellow solid: mp 228-232°C; ¹H NMR (DMSO-d₆) δ 7.64 (br t, 1H), 7.51 (br s, 1H), 7.45-7.40 (m with overlapping s at δ 7.43, 2H), 7.26 (d, 1H), 4.74 (d, 2H), 2.64 (q, 2H), 1.35 (s, 9H), 1.15 (t, 3H) ppm. IR (KBr): 3210, 2990, 1800, 1660 cm⁻¹; MS (m/z) 364/366 (M⁺).

| Elemental analysis for C₁₇H₂₁BrN₂O₂ | |
|---|---|
| Calc'd | C, 55.90; H, 5.80; N, 7.67. |
| Found | C, 55.99; H, 5.67; N, 7.64. |

### EXAMPLE 92 3-(4-Bromo-2-ethyl-benzylamino)-4-(1,1-dimethyl-propylamino)-cyclobut-3-ene-1,2-dione

This compound was prepared according to the procedure described in Example 75. From 3-(4-bromo-2-ethyl-benzylamino)-4-ethoxy-cyclobut-3-ene-1,2-dione (0.4 g, 1.18 mmol), 1,1-dimethylpropylamine (5 mL), and dichloromethane (2 mL) there was obtained after 4 days at room temperature and followed by removal of solvent under reduced pressure a solid. This was placed in acetonitrile, filtered, rinsed with acetonitrile, and dried (0.4 mm, 70°C) to yield 0.30 g (67%) of the title compound as a white solid: mp 182-186°C; ¹H NMR (DMSO-d₆) δ 7.68 (br t, 1H), 7.45-7.40 (m with overlapping s at δ 7.43, 2H), 7.38 (br s, 1H), 7.25 (d, 1H), 4.75 (d, 2H), 2.64 (q, 2H), 1.66 (q 2H), 1.29 (s, 6H), 1.15 (t, 3H), 0.80 (t, 3H) ppm. IR (KBr): 3210, 2980, 1800, 1650 cm⁻¹; MS (m/z) 378/380 (M⁺).

| Elemental analysis for C₁₈H₂₃BrN₂O₂ | |
|---|---|
| Calc'd | C, 57.00; H, 6.11; N, 7.38. |
| Found | C, 56.23; H, 6.14; N, 7.35. |

### EXAMPLE 93 4-[(1,1-Dimethyl-propylamino-3,4-dioxo-cyclobut-1-enylamino)-methyl]-3-ethyl-benzonitrile

From a solution of 3-(1,1-dimethypropylamino)-4-ethoxy-cyclobut-3-ene-1,2-dione (0.34 g, 1.61 mmol) and 4-cyano-2-ethylbenzylamine (7.7 mmol) in absolute ethanol there was obtained 9 days at room temperature and followed by removal of solvent under reduced pressure a solid. This solid was chromatographed on silica gel (5% methanol in dichloromethane) to give a residue, which was trituated with acetonitrile, filtered, rinsed with acetonitrile, and dried to yield 0.31 g (12%) of the title compound as an off-white solid: mp 183-186°C; ¹H NMR (DMSO-d₆) δ 7.80-7.70 (m with overlapping br t at δ 7.75, 3H), 7.47 (d, 1H), 7.43 (br s, 1H), 4.85 (d, 2H), 2.69 (q, 2H), 1.66 (q, 2H), 1.30 (s, 6H), 1.17 (t, 3H), 0.81 (t, 3H) ppm. IR (KBr): 3210, 2980, 2240, 1800, 1650 cm⁻¹; MS (m/z) 326 ([M+H]⁺).

| Elemental analysis for C₁₉H₂₃N₃O₂ | |
|---|---|
| Calc'd | C, 70.13; H, 7.13; N, 12.91. |
| Found | C, 70.18; H, 6.99; N, 12.82. |

### EXAMPLE 94 3-(2,6-Dimethyl-benzylamino)-4-(1,2,2-trimethyl-propylamino)-cyclobut-3-ene-1,2-dione

### Step 1) Preparation of 3-(2,6-dimethyl-benzylamino)-4-ethoxy-cyclobut-3-ene-1,2-dione

This compound was prepared according to the procedure described in Example 67, Step 1. From 2,6-dimethylbenzaldehyde oxime (2.67 g, 18 mmol), zinc powder (4.68 g, 72 mmol), glacial acetic acid (35.7 mL), and 3,4-diethoxy-3-cyclobutene-1,2-dione (2.65 mL, 18 mmol) in absolute ethanol there was obtained after chromatography on silica gel (hexane:ethyl acetate (3:1), 5% methanol in dichloromethane) 2.0 g (43%) of a solid: ¹H NMR (DMSO-d₆) δ 8.90 and 8.70 (br m, 1H, rotamers), 7.20-7.00 (m, 3H), 4.90-4.50 (m, 4H), 2.30 (s, 6H), 1.38 (m, 3H).

### Step 2) 3-(2,6-Dimethyl-benzylamino)-4-(1,2,2-trimethyl-propylamino)-cyclobut-3-ene-1,2-dione

This compound was prepared according to the procedure described in Example 77. From the product of Example 94, Step 1 (0.3 g, 1.16 mmol) and (R)-2-amino-3,3-dimethylbutane (26 mL of a 0.2 M solution in absolute ethanol, 5.2 mmol), there was obtained after 24 hours at room temperature, followed by refluxing for 4-5 hours, a solid, which was filtered, rinsed with acetonitrile (3 x 3 mL) and dried to give 0.33 g (90%) of the (R) isomer of the title compound as a white solid: mp 298-301°C (dec); [α]²⁵_{D} = +15.38° (9.9 mg/mL, DMSO); ¹H NMR (DMSO-d₆) δ 7.17 (br m, 1H), 7.16-7.05 (m, 4H), 4.80 (d, 2H), 3.90 (m, 1H), 2.35 (s, 6H), 1.08 (d, 3H), 0.84 (s, 9H) ppm. IR (KBr): 3150, 2980, 1800, 1650 cm⁻¹; MS (m/z) 314 (M⁺).

| Elemental analysis for C₁₉H₂₆N₂O₂ | |
|---|---|
| Calc'd | C, 72.58; H, 8.34; N, 8.91. |
| Found | C, 72.44; H, 8.41; N, 8.91. |

### EXAMPLE 95 3-tert-Butylamino-4-(2,6-dimethyl-benzylamino)-cyclobut-3-ene-1,2-dione

This compound was prepared according to the procedure described in Example 79. From the product of Example 94, Step 1 (0.32 g, 1.23 mmol), t-butylamine (2 mL), and dichloromethane there was obtained after 48 hours at room temperature a solid which was diluted with acetonitrile (4 mL), filtered, rinsed with acetonitrile (3 x 2 mL) and dried to give 0.17 g (48%) of the title compound as a white solid: mp 265-267°C; ¹H NMR (DMSO-d₆) δ 7.39 (br s, 1H), 7.29 (br t, 1H), 7.16-7.05 (m, 3H), 4.79 (d, 2H), 2.34 (s, 6H), 1.34 (s, 9H) ppm. IR (KBr): 3200, 2950, 1795, 1650 cm⁻¹; MS (m/z) 287 ([M+H]⁺).

| Elemental analysis for C₁₇H₂₂N₂O₂ | |
|---|---|
| Calc'd | C, 71.30; H, 7.74; N, 9.78. |
| Found | C, 70.58; H, 7.75; N, 9.69. |

### EXAMPLE 96 3-(2,6-Dimethyl-benzylamino)-4-(1,1-dimethyl-propylamino)-cyclobut-3-ene-1,2-dione

This compound was prepared according to the procedure described in Example 75. From the product of Example 94, Step1 (0.4 g, 1.54 mmol), 1,1-dimethylpropylamine (5 mL), and dichloromethane (2 mL) there was obtained after refluxing for 8 hours and removal of solvent under reduced pressure 0.44 g of a solid, which was placed in isopropanol, filtered, and the filtrate was concentrated under reduced pressure. The resulting solid was placed in ethyl acetate, filtered, rinsed with ethyl acetate, and dried (0.4 mm, 70°C). This gave 0.21 g (45%) of the title compound as a solid: mp 227-231°C (softens 199°C); ¹H NMR (DMSO-d₆) δ 7.32 (br t, 1H), 7.26 (br s, 1H), 7.17-7.05 (m, 3H), 4.80 (d, 2H), 2.34 (s, 6H), 1.65 (q 2H), 1.28 (s, 6H), 0.79 (t, 3H) ppm. IR (KBr): 3200, 2980, 1800, 1650 cm⁻¹; MS (m/z) 300 (M⁺).

| Elemental analysis for C₁₈H₂₄N₂O₂ | |
|---|---|
| Calc'd | C, 71.97; H, 8.05; N, 9.33. |
| Found | C, 71.65; H, 8.27; N, 9.30. |

### EXAMPLE 97 3-(2,6-Dimethyl-benzylamino)-4-(2,2,3,3,3-pentafluoro-propylamino)-cyclobut-3-ene-1,2-dione

From a solution of 3-(2,6-dimethyl-benzylamino)-4-ethoxy-cyclobut-3-ene-1,2-dione (0.30 g, 1.16 mmol), 2,2,3,3,3-pentafluoropropylamine (2.4 mL), absolute ethanol (2 mL), and dichloromethane (2 mL) there was obtained after 6 days at room temperature a solution which was concentrated under reduced pressure and triturated with dichioromethane to give 0.05 g (12%) of the title compound as a white solid: mp 281-283°C; ¹H NMR (DMSO-d₆) δ 7.56 (br m, 1H), 7.46 (br m, 1H), 7.18-7.04 (m, 3H), 4.80 (d, 2H), 4.42 (dt, 2H), 2.34 (s, 6H) ppm. MS (m/z) 363 ([M+H]⁺).

| Elemental analysis for C₁₆H₁₅F₅N₂O₂ | |
|---|---|
| Calc'd | C, 53.04; H, 4.17; N, 7.73. |
| Found | C, 52.08; H, 4.06; N, 7.54. |

### EXAMPLE 98 4-{[3,4-Dioxo-2-(1,2,2-trimethyl-propylamino)-cyclobut-1-enylamino]-methyl}-3-methyl-benzonitrile

This compound was prepared in a manner similar to Example 94 using appropriate starting materials to afford the (R) isomer of the title compound as a pale pink solid: mp 250-258°C (softens 242°C); ¹H NMR (DMSO-d₆) δ 7.70 (m, 2H), 7.60 (br m, 1H), 7.45 (d, 1H), 7.27 (br d, 1H), 4.80 (m, 2H), 3.91 (m, 1H), 2.35 (s, 3H), 1.11 (d 3H), 0.86 (s, 9H) ppm. IR (KBr): 3180, 2950, 2200, 1790, 1650 cm⁻¹; MS (m/z) 325 (M⁺).

| Elemental analysis for C₁₉H₂₃N₃O₂ | |
|---|---|
| Calc'd | C, 70.13; H, 7.12; N, 12.91. |
| Found | C, 69.81; H, 7.14; N, 12.96. |

### EXAMPLE 99 4-{[3,4-Dioxo-2-(1,2,2-trimethyl-propylamino)-cyclobut-1-enylamino]-methyl}-3-methoxy-benzonitrile

This compound was prepared in a manner similar to Example 94 using appropriate starting materials to afford the (R) isomer of the tide compound as a white solid: mp 172-178°C; ¹H NMR (DMSO-d₆) δ 7.61 (br m, 1H), 7.51 (s, 1H), 7.49-7.38 (m, 2H), 7.32 (br d, 1H), 4.73 (m, 2H), 3.89 (overlapping s and m, 4H), 1.10 (d, 3H), 0.86 (s, 9H) ppm. MS (m/z) 341 (M⁺).

| Elemental analysis for C₁₉H₂₃N₃O₃ | |
|---|---|
| Calc'd | C, 66.84; H, 6.79; N, 12.31. |
| Found | C, 66.38; H, 6.78; N, 12.16. |

### EXAMPLE 100 3-(2-Methoxy-6-methyl-benzylamino)-4-(1,2,2-trimethyl-propylamino)-cyclobut-3-ene-1,2-dione

This compound was prepared in a manner similar to Example 94 using appropriate starting materials to afford the (R) isomer of the title compound as a white solid: mp 278-280°C (dec); [α]²⁵_{D}= +28.76° (9.8 mg/mL, DMSO); ¹H NMR (DMSO-d₆) δ 7.21 (t, 1H), 7.17 (m, 1H), 7.15 (m, 1H), 6.90 ( d, 1H), 6.82 (d, 1H), 4.78 (m, 2H), 3.89 (m, 1H), 3.81 (s, 3H), 2.32 (s, 3H), 1.08 (d, 3H), 0.84 (s, 9H) ppm. IR (KBr): 3150, 2970, 1795, 1645 cm⁻¹; MS (m/z) 330 (M⁺).

| Elemental analysis for C₁₉H₂₆N₂O₃ | |
|---|---|
| Calc'd | C, 69.06; H, 7.93; N, 8.48. |
| Found | C, 69.16; H, 7.99; N, 8.47. |

### EXAMPLE 101 3-tert-Butylamino-4-(2-ethyl-6-methyl-benzylamino)-cyclobut-3-ene-1,2-dione

This compound was prepared in a manner similar to Example 94 using appropriate starting materials to afford the title compound as a white solid: mp melts and resolidifies at 183-186°C, then melts at 240-244°C; ¹H NMR (DMSO-d₆) δ 7.39 (br s, 1H), 7.29 (br t, 1H), 7.22-7.06 (m, 3H), 4.80 (d, 2H), 2.67 (q, 2H), 2.35 (s, 3H), 1.34 (s, 9H), 1.13 (t, 3H) ppm. MS (m/z) 300 (M⁺).

| Elemental analysis for C₁₈H₂₄N₂O₂ | |
|---|---|
| Calc'd | C, 71.97; H, 8.05; N, 9.33. |
| Found | C, 71.32; H, 8.20; N, 9.17. |

### EXAMPLE 102 3-tert-Butylamino-4-(4-fluoro-2-trifluoromethyl-benzylamino)-cyclobut-3-ene-1,2-dione

To a room temperature solution of (0.7 g, 3.55 mmol) of 3-ethoxy-4-(tert-butylamino)-cyclobut-3-ene-1,2-dione and THF (30 mL) was added (0.73 g, 3.78 mmol) of the 4-fluoro-2-trifluoromethylbenzylamine via a syringe. The resulting mixture was stirred at room temperature for 12 hours. After concentration in vacuo, the resulting white solid was recrystallized from hot acetonitrile to give 0.55 gm (52%) of 3-tert-butylamino-4-(4-fluoro-2-trifluoromethyl-benzylamino)-cyclobut-3-ene-1,2-dione as a white solid: mp 192-194 °C; ¹H NMR (DMSO-d₆) δ 1.36 (s, 9H), 4.88 (d, 2H) 7.67 (m, 5H) ppm; IR (KBr) 3250, 2900, 1800, 1580, 1530 cm⁻¹; MS (m/z) 345 ([M+H]⁺).

| Elemental analysis for C₁₆H₁₆F₄N₂O₂ | |
|---|---|
| Calc'd | C, 55.82; H, 4.68; N, 8.14. |
| Found | C, 55.68; H, 8.02; N, 8.02. |

### EXAMPLE 103 3-tert-Butylamino-4-(2-chloro-4-fluoro-benzylamino)-cyclobut-3-ene-1,2-dione

This compound was prepared according the procedure for Example 102. From 2-chloro-4-fluorobenzylamine and 3-ethoxy-4-(tert-butylamino)-cyclobut-3-ene-1,2-dione there was obtained a white solid (59%): mp 202-204 °C; ¹H NMR (DMSO-d₆) δ 1.36 (s, 9H), 3.34 (m, 1H), 4.80 (d, 2H), 7.28 (m, 1H), 7.53 (m, 2H), 7.67 (m, 1H) ppm; IR (KBr) 3260, 2950, 1800, 1660, 1570, 1530, 1500 cm⁻¹; MS (m/z) 311 ([M+H]⁺).

| Elemental analysis for C₁₅H₁₆ClFN₂O₂ | |
|---|---|
| Calc'd | C, 57.98; H, 5.19; N, 9.01. |
| Found | C, 58.13; H, 5.33; N, 8.46. |

### EXAMPLE 104 3-(4-Fluoro-2-trifluoromethyl-benzylamino)-4-(1,2,2-trimethyl-propylamino)-cyclobut-3-ene-1,2-dione

This compound was prepared according to the procedure for Example 102. From (R)-3-ethoxy-4-(1,2,2-triimethyl-propylamino)-cyclobut-3-ene-1,2-dione and 4-fluoro-2-trifluoromethylbenzylamine in acetonitrile. After recrystallization from diethyl ether the (R) isomer of the title acompound was obtained as a white solid (54%); mp 208-210°C; IR (KBr) 3260, 2950, 1800, 1660, 1570, 1530, 1500 cm⁻¹; MS (m/z) 373 ([M+H]⁺).

| Elemental analysis for C₁₈H₂₀F₄N₂O₂ | |
|---|---|
| Calc'd | C, 58.06; H, 5.41; N, 7.52. |
| Found | C, 58.42; H, 5.62; N, 7.51. |

### EXAMPLE 105 3-(2,4-Difluoro-benzylamino)-4-(1,1-dimethyl-propylamino)-cyclobut-3-ene-1,2-dione

This compound was prepared according to the procedure for Example 102. From 3-ethoxy-4-(1,1-dimethyl-propylamino)-cyclobut-3-ene-1,2-dione and 2,4-difluorobenzylamine in acetonitrile. The title compound was obtained, after recrystallization from diethyl ether, as a white solid (80%): mp 242-244 °C; ¹H NMR (DMSO-d₆) δ 0.82 (t, 3H), 1.30 (s, 6H), 1.68 (q, 2H), 4.76 (d, 2H), 7.09 (m, 1H), 7.27 (m, 1H), 7.50 (m, 2H), 7.80 (m, 1H) ppm; IR (KBr) 3250, 3150, 2970, 1790, 1652, 1580, 1538, 1510 cm⁻¹; MS (m/z) 309 ((M+H]⁺).

| Elemental analysis for C₁₆H₁₈F₂N₂O₂ | |
|---|---|
| Calc'd | C, 62.33; H, 5.88; N, 9.09. |
| Found | C, 62.29; H, 5.93; N, 8.90. |

### EXAMPLE 106 3-(2-Chloro-4-fluoro-benzylamino)-4-(1,1-dimethyl-propylamino)-cyclobut-3-ene-1,2-dione

This compound was prepared according to the procedure for Example 102. From 2-chloro-4-fluorobenzylamine and 3-ethoxy-4-(1,1-dimethyl-propylamino)-cyclobut-3-ene-1,2-dione in acetonitrile. The title compound was obtained, after recrystallization from petroleum ether, as a white solid (60%); mp 201-203 °C, ¹H NMR (DMSO-d₆) δ 0.81 (t, 3H), 1.30 (s, 6H), 1.68 (q, 2H), 4.76 (d, 2H), 7.25 (m, 1H), 7.50 (m, 3H), 7.78 (m, 1H) ppm; IR (KBr) 3250, 3040, 1790, 1660, 1580, 1530, 1490 cm⁻¹; MS (m/z) 325 ([M+H]⁺).

| Elemental analysis for C₁₆H₁₈ClFN₂O₂ | |
|---|---|
| Calc'd | C, 59.17; H, 5.59; N, 8.63. |
| Found | C, 59.17; H, 5.50; N, 8.51. |

### EXAMPLE 107 3-tert-Butylamino-4-(2-fluoro-5-trifluoromethyl-benzylamino)-cyclobut-3-ene-1,2-dione

This compound was prepared according to the procedure for Example 102. From 2-fluoro-5-trifluoromethylbenzylamine and 3-ethoxy-4-(tert-butylamino)-cyclobut-3-ene-1,2-dione the title compound was obtained as a white solid (66%): mp 225-227 °C; ¹H NMR (DMSO-d₆) δ 1.35 (s, 9H), 4.75 (d, 2H), 7.51 (m, 2H), 7.83 (m, 3H) ppm; IR (KBr) 3233, 3045, 1794, 1658, 1571, 1537, 1471 cm⁻¹; MS (m/z) 345 ([M+H]⁺).

| Elemental analysis for C₁₆H₁₆F₄N₂O₂ | |
|---|---|
| Calc'd | C, 55.82; H, 4.68; N, 8.14. |
| Found | C,55.74; H, 4.46; N, 7.95. |

### EXAMPLE 108 3-(2-Chloro-5-fluoro-benzylamino)-4-(1,2,2-trimethyl-propylamino)-cyclobut-3-ene-1,2-dione

This compound was prepared according to the procedure for Example 102. From (R)-3-ethoxy-4-(1,2,2-trimethyl-propylamino)-cyclobut-3-ene-1,2-dione and 2-chloro-5-fluoro-benzylamine there was obtained after recrystallization from diethyl ether, the (R) isomer of the title compound as a white solid (80%): mp 218-220 °C; ¹H NMR (DMSO-d₆) δ 0.87 (s, 9H), 1.11 (d, Hz, 3H), 3.91 (m, 1H), 4.80 (q, 2H), 7.30 (m, 2H), 7.50 (m, 2H), 7.60 (m, 1H) ppm; IR (KBr) 3194, 3063, 1797, 1654, 1578, 1541, 1492 cm⁻¹; MS (m/z) 339 ([M+H]⁺).

| Elemental analysis for C₁₇H₂₀ ClFN₂O₂ | |
|---|---|
| Calc'd | C, 60.27; H, 5.95; N, 8.27. |
| Found | C, 60.44; H, 6.01; N, 8.11. |

### EXAMPLE 109 3-(2,5-Difluoro-benzylamino)-4-(1,2,2-trimethyl-propylamino)-cyclobut-3-ene-1,2-dione

This compound was prepared according to the procedure for Example 102. From (R)-3-ethoxy-4-(1,2,2-trimethyl-propylamino)-cyclobut-3-ene-1,2-dione and 2,5-difluorobenzylamine there was obtained after recrystallization from diethyl ether, the (R) isomer of the title compound as a white solid (85%): mp 265-267 °C; ¹H NMR (DMSO-d₆) δ 0.87 (s, 9H), 1.11 (d, 3H), 3.91 (m, 1H), 4.80 (q, 2H), 7.25 (m, 4H), 7.60 (m, 1H) ppm; IR (KBr) 3195, 3068, 1798, 1653, 1577, 1543, 1494 cm⁻¹; MS: 323 ([M+H]⁺).

| Elemental analysis for C₁₇H₂₀F₂N₂O₂ | |
|---|---|
| Calc'd | C, 63.34; H, 6.25; N, 8.69. |
| Found | C, 63.55; H, 6.30; N, 8.59. |

### EXAMPLE 110 3-tert-Butylamino-4-(3-chloro-4-fluoro-benzylamino)-cyclobut-3-ene-1,2-dione

This compound was prepared according to the procedure for Example 102. From 3-chloro-4-fluorobenzylamine and 3-ethoxy-4-(tert-butylamine)-cyclobut-3-ene-1,2-dione, the title compound was obtained as a white solid (59%): mp 288-290 °C; ¹H NMR (DMSO-d₆) δ 1.36 (s, 9H), 4.80 (d, 2H) 7.44 (m, 2H), 7.60 (m, 2H), 7.75 (m, 1H) ppm; IR (KBr) 3233, 3039, 1792, 1652, 1571, 1537, 1501 cm⁻¹; MS (m/z) 311 ([M+H]⁺).

| Elemental analysis for C₁₅H₁₆ClFN₂O₂ | |
|---|---|
| Calc'd | C, 57.98; H, 5.19; N, 9.01. |
| Found | C, 58.02; H, 5.24; N, 8.82. |

### EXAMPLE 111 3-tert-Butylamino-4-(3,4-difluoro-benzylamino)-cyclobut-3-ene-1,2-dione

This compound was prepared according to the procedure for Example 102. From 3,4-difluorobenzylamine and 3-ethoxy-4-(tert-butylamino)-cyclobut-3-ene-1,2-dione, the title compound was obtained as a white solid (77%): mp 296-298 °C; ¹H NMR (DMSO-d₆) δ 1.36 (s, 9H), 4.72 (d, 2H) 7.22 (m, 1H), 7.56 (m, 3H), 7.77 (m, 1H) ppm; IR (KBr) 3235, 2970, 1790, 1650, 1570, 1540, 1490 cm⁻¹; MS (m/z) 295 ([M+H]⁺).

| Elemental analysis for C₁₅H₁₆F₂N₂O₂ | |
|---|---|
| Calc'd | C, 61.22; H, 5.48; N, 9.52. |
| Found | C, 61.05; H, 5.40; N, 9.31. |

### EXAMPLE 112 3-(3-Chloro-4-fluoro-benzylamino)-4-(1,2,2-trimethyl-propylamino)-cyclobut-3-ene-1,2-dione

This compound was prepared according to the procedure for Example 102. From (R)-3-ethoxy-4-(1,2,2-trimethyl-propylamino)-cyclobut-3-ene-1,2-dione and 3-chloro-4-fluorobenzylamine, the (R) isomer of the title compound was obtained after recrystallization from diethyl ether, as a white solid (73%): mp 270- 272 °C; ¹H NMR (DMSO-d₆) δ 0.87 (s, 9H), 1.11 (d, 3H), 3.91 (m, 1H), 4.80 (q, 2H), 7.45 (m, 3H), 7.60 (m, 2H) ppm; IR (KBr) 3201, 3062, 1798, 1650, 1575, 1492 cm⁻¹; MS (m/z) 339 ([M+H]⁺).

| Elemental analysis for C₁₇H₂₀ClFN₂O₂ | |
|---|---|
| Calc'd | C, 60.27; H, 5.95; N, 8.27. |
| Found | C, 60.27; H, 6.32; N, 8.18. |

### EXAMPLE 113 3-(3,4-Difluoro-benzylamino)-4-(1,1-dimethyl-propylamino)-cyclobut-3-ene-1,2-dione

This compound was prepared according to the procedure for Example 102. From 3-ethoxy-4-(1,1-dimethyl-propylamino)-cyclobut-3-ene-1,2-dione and 3,4-difluorobenzylamine in acetonitrile there was obtained after recrystallization from diethyl ether, the title compound as a white solid (62%): mp 273-275 °C; ¹H NMR (DMSO-d₆) d 0.82 (t, 3H), 1.30 (s, 6H), 1.68 (q, 2H) 4.71 (d, 2H), 7.21 (m, 1H), 7.44 (m, 3H), 7.81 (m, 1H) ppm; IR (KBr) 3290, 2970, 1790, 1652, 1570, 1538, 1510 cm⁻¹; MS (m/z) 309 ([M+H]⁺).

| Elemental analysis for C₁₆H₁₈F₂N₂O₂ | |
|---|---|
| Calc'd | C, 62.33; H, 5.88; N, 9.09. |
| Found | C, 62.36; H, 5.83; N, 8.91. |

### EXAMPLE 114 3-tert-Butylamino-4-(3,5-difluoro-benzylamino)-cyclobut-3-ene-1,2-dione

This compound was prepared according the procedure for Example 102. From 3,5-difluorobenzylamine and 3-ethoxy-4-(tert-butylamino)-cyclobut-3-ene-1,2-dione there was obtained the title compound as a white solid (77%): mp 287-289 °C; ¹H NMR (DMSO-d₆) δ 1.37 (s, 9H), 4.74 (d, 2H), 7.08 (m, 2H), 7.12 (m, 1H), 7.60 (m, 1H), 7:80 (m, 1H) ppm; IR (KBr) 3280, 3080, 1793, 1656, 1571, 1538, 1470 cm⁻¹; MS (m/z) 294 ([M+H]⁺).

| Elemental analysis for C₁₅H₁₆F₂N₂O₂ | |
|---|---|
| Calc'd | C, 61.22; H, 5.48; N, 9.52. |
| Found | C, 61.29; H, 5.63; N, 9.29. |

### EXAMPLE 115 3-tert-Butylamino-4-(3-fluoro-5-trifluoromethyl-benzylamino)-cyclobut-3-ene-1,2-dione

This compound was prepared according to the procedure for Example 102. From 3-fluero-5-trifluoromethylbenzylamine and 3-ethoxy-4-(tert-butylamino)-cyclobut-3-ene-1,2-dione there was obtained after recrystallization from diethyl ether, the title compound as a white solid (53%): mp 246-248 °C; ¹H NMR (DMSO-d₆) δ 1.37 (s, 9H), 4.75 (d, 2H), 7.63 (m, 3H), 7.82 (m, 1H) ppm; IR (KBr): 3230, 3040, 1794, 1658, 1571, 1537, 1470 cm⁻¹; MS (m/z) 344 ([M+H]⁺).

| Elemental analysis for C₁₆H₁₆F₄N₂O₂ | |
|---|---|
| Calc'd | C, 55.82; H, 4.68; N, 8.14. |
| Found | C, 55.81; H, 4.55; N, 8.13. |

### EXAMPLE 116 3-(3,5-Bis-trifluoromethyl-benzylamino)-4-tert-butylamino-cyclobut-3-ene-1,2-dione

This compound was prepared according to the procedure for Example 102. From 3,5-bis-trifluoromethylbenzylamine and 3-ethoxy-4-(tert-butylamino)-cyclobut-3-ene-1,2-dione the title compound was obtained as a white solid (58%): mp 253-255 °C; ¹H NMR (DMSO-d₆) δ 1.36 (s, 9H), 4.74 (d, 2H), 7.61 (s, 3H), 7.85 (s, 1H), 7.81 (m, 1H) ppm; IR (KBr) 3243, 3055, 1794, 1661, 1575, 1537, 1470 cm⁻¹; MS (m/z) 394 ([M+H]⁺).

| Elemental analysis for C₁₇H₁₆F₆N₂O₂ | |
|---|---|
| Calc'd | C, 51.78; H, 4.09; N, 7.10. |
| Found | C, 52.70; H, 4.10; N, 7.15. |

### EXAMPLE 117 3-(3,5-Difluoro-benzylamino)-4-(1,1-dimethyl-propylamino)-cyclobut-3-ene-1,2-dione

This compound was prepared according to the procedure for Example 102. From 3-ethoxy-4-(1,1,-dimethyl-propylamino)-cyclobut-3-ene-1,2-dione and 3,5-difluorobenzylamine the title compound was obtained after recrystallization from diethyl ether, as a white solid (81%): mp 264-266 °C; ¹H NMR (DMSO-d₆) δ 0.83 (t, 3H), 1.31 (s, 6H), 1.68 (q, 2H), 4.75 (d, 2H), 7.07 (m, 2H), 7.18 (m, 1H), 7.43 (s, 1H), 7.82 (m, 1H) ppm. IR (KBr): 3294, 2977, 1796, 1655, 1574, 1538, 1465 cm⁻¹; MS (m/z) 309 ([M+H]⁺).

| Elemental analysis for C₁₆H₁₈F₂N₂O₂ | |
|---|---|
| Calc'd | C, 62.33; H, 5.88; N, 9.09. |
| Found | C, 62.37; H, 5.97; N, 8.95. |

### EXAMPLE 118 3-Chloro-4-{[2-(2-fluoro-1,2-dimethyl-propylamino)-3,4-dioxo-cyclobut-1-enylamino]-methyl}-benzonitrile

### Step 1) 3-Fluorovalinol

To a solution of lithium borohydride (1.61 g, 74 mmol) in tetrahydrofuran (40 mL) under a nitrogen atmosphere was added trimethylsilyl chloride (18.8 mL, 148 mmol) via pipet. A precipitate quickly formed. After 3 minutes, 3-fluorovaline (5 g, 37 mmol) was added in three portions. This mixture was stirred for 24 hours. The reaction was quenched by the dropwise addition of methanol. The methanol and tetrahydrofuran were removed on a rotary evaporator (30 degree water bath) and water (25 mL) was added. The aqueous mixture was made basic with 2.5N aqueous sodium hydroxide and was then extracted with dichloromethane (4 x 50 mL). The combined organics were dried (Na₂SO₄), filtered and evaporated to give 3.83 g of 3-fluorovalinol: ¹H NMR (CDCl₃) δ 3.71 (dd, 1H), 3.36 (m, 1H), 2.90 (m,1H), 2.10 (br, 2H), 1.38 (d, 3H), 1.33 (d, 3H) ppm.

### Step 2) N-Butoxycarbonyl-3-fluorovalinol

To a solution of 3-fluorovalinol (3.79 g, 31.4 mmol) in chloroform (35 mL) under a nitrogen atmosphere was added a solution of di-t-butyl dicarbonate (6.84 g, 31.4 mmol) in chloroform (15 mL). The mixture was stirred at room temperature for four hours, then the solvent was removed on a rotary evaporator. The residue was dissolved in diethyl ether (100 mL), washed with 20% phosphoric acid (1 x50 mL), brine (1 x 50 mL), saturated aqueous sodium bicarbonate (1 x 50 mL), brine (1 x 50 mL), and then dried (MgSO₄). Filtration and concentration under reduced pressure gave 6.34 g of N-butoxycarbonyl-3-fluorovalinol as a white solid: ¹H NMR (CDCl₃) δ 5.08 (br, 1H), 3.82 (m, 1H), 3.68 (m, 1H), 1.46 (s, 9H), 1.46 (d, 3H), 1.39 (d, 3H) ppm.

### Step 3) N-Butoxycarbonyl-1-iodo-2-amino-3-fluoro-3-methyl-n-butane

To a well-stirred mixture of polystyryl supported triphenylphosphine (29.3 mmol) in dry dichloromethane (40 mL) under a nitrogen atmosphere was added iodine (7.44 g, 29.3 mmol). After ten minutes, imidazole (2.0 g, 29.3 mmol) was added, followed in ten minutes by a solution of N-butoxycarbonyl-3-fluorovalinol (13.3 mmol) in dichloromethane (200 mL). The mixture was heated to reflux for two hours. The cooled mixture was filtered through celite and the filtrate was evaporated. The residue was dissolved in diethyl ether (150 mL), and this solution was washed with dilute aqueous sodium thiosulfate (1 x 75 mL) and water (2 x 75 mL). The organic layer was dried (Na₂SO₄), filtered through a pad of silica gel and evaporated to afford 3.46 g of N-butoxycarbonyl-1-iodo-2-amino-3-fluoro-3-methyl-n-butane: ¹H NMR (CDCl₃) δ 4.72 (br d, 1H), 3.86 (br m, 1H), 3.56 (dd, 1H), 1.47 (s, 9H), 1.43 (m, 6H) ppm.

### Step 4) N-Butoxycarbonyl-2-amino-3-fluoro-3-methyl-n-butane

A Parr bottle was charged with palladium (II) hydroxide (800 mg), a solution of N-butoxycarbonyl-1-iodo-2-amino-3-fluoro-3-methyl-n-butane (3.26 g, 9.8 mmol) in ethanol (80 mL) and triethylamine (0.99 g, 9.8 mmol). The reaction mixture was placed under hydrogen gas (50 psig) and shaken for 20 hours. The mixture was filtered through Celite® and evaporated. The residue was dissolved in diethyl ether (100 mL) and washed with 1N aqueous HCl (2 x 50 mL), water (2 x 50 mL), and then dried (MgSO₄). Filtration and evaporation gave a residue that was chromatographed (silica gel, diethyl ether/hexane (3/1)) to afford 1.80 g of N-butoxycarbonyl-2-amino-3-fluoro-3-methyl-n-butane: ¹H NMR (CDCl₃) δ 4.65 (br, 1H), 3.70 (br m, 1H), 1.45 (s, 9H), 1.39 (d, 3H), 1.32 (d, 3H), 1.18 (d, 3H) ppm.

### Step 5) 3-Ethoxy-4-(N-3-fluoro-3-methyl-n-butyl-2-amine)-3-cyclobutene-1,2-dione

A mixture of N-butoxycarbonyl-2-amino-3-fluoro-3-methyl-n-butane (1.75 g, 8.5 mmol), dichloromethane (5 mL), trifluoroacetic acid (4 mL), and methanol (0.75 mL) were warmed to 45°C for five hours. The volatile components were removed on a rotary evaporator and the syrupy residue was used without further purification. To a solution of 3-fluoro-3-methyl-n-butyl-2-amine trifluoroacetate salt (8.5 mmol) in ethanol (42.5 mL) was added 3,4-diethoxy-3-cyclobutene-1,2-dione (1.44 g, 8.5 mmol) followed by triethylamine (2.58 g, 25.5 mmol). The reaction mixture was stirred under a nitrogen atmosphere at- room temperature for two hours then the temperature was raised to 50°C for five hours. The mixture was cooled and the solvents removed on a rotary evaporator. The residue was dissolved in diethyl ether (90 mL) and washed with water (1 x 60 mL), 1 N aqueous HCl (1 x 60 mL), water 1 x 60 mL). The organic layer was dried (MgSO₄), filtered, and evaporated. The residue was chromatographed (silica gel, diethyl ether) to afford 1.65 g of 3-ethoxy-4-(N-3-fluoro-3-methyl-n-butyl-2-amine)-3-cyclobutene-1,2-dione as a white solid: ¹H NMR (CDCl₃) δ 6.21 (br, 1H), 4.77 (br m, 2H), 3.80 (br, 1H), 1.47 (t, 3H,), 1.43 (d, 3H), 1.36 (d, 3H), 1.32 (d, 3H) ppm.

### Step 6) 3-Chloro-4-{[2-(2-fluoro-1,2-dimethyl-propylamino)-3,4-dioxo-cyclobut-1-enylamino]-methyl}-benzonitrile

To a solution of 3-ethoxy-4-(N-3-fluoro-3-methyl-n-butyl-2-amine)-3-cyclobutene-1,2-dione (0.573 g, 2.5 mmole) in dry tetrahydrofuran(8 mL) was added 2-chloro-4-cyanobenzylamine (0.458 g, 2.75 mmole). The mixture was heated to 70°C under a nitrogen atmosphere for 18 hours. The mixture was cooled to room temperature with stirring and vacuum filtered through a fritted glass filter. The solid was washed well with several portions of an ethanol/diethyl ether (1/1) solvent mixture. The solid was air dried then heated to 77°C under high vacuum for 16 hours. This afforded 0.49 g of the title compound as a white solid: ¹H NMR (DMSO-d₆) δ 8.10 (d, 1H), 7.88 (dd, 1H), 7.75 (br, 1H), 7.60 (d, 1H), 7.60 (br, 1H), 4.87 (m, 2H), 4.17 (br, 1H), 1.35 (s, 3H), 1.29 (s, 3H), 1.20 (d, 3H) ppm. IR (KBr): 2220, 1850 cm⁻¹; MS (m/z) 350 ([M+H]⁺).

### EXAMPLE 119 3-Chloro-4-{[2-(1,1-dimethyl-propylamino)-3,4-dioxo-cyclobut-1-enylamino]-methyl}-benzonitrile

Tetrahydrofuran (10 mL), 3-butoxy-4-(1,1-dimethylpropylamino)-cyclobut-3-ene-1,2-dione (0.957 g, 4 mmol, Example 46) and 2-chloro-4-cyanobenzylamine (0.666 g, 4 mmol, Example 45, Step 3) were stirred together at room temperature for 138 hours. Removal of solvent, through trituration of the residue with diethyl ether and drying provided a white solid. Recrystallization of this solid from acetonitrile (charcoal) followed by a second recrystallization from acetonitrile gave 0.863 g of title compound as a white solid: mp 215.5-219.5 °C (softens 212.5 °C); ¹H NMR (DMSO-d₆) δ 8.11 (d, 1H), 7.89 (m, 1H), 7.86 (m, 1H), 7.61 (d, 1H), 7.53 (s, 1H), 4.90 (d, 2H), 1.68 (q, 2H), 1.30 (s, 6H), 0.82 (t, 3H) ppm. IR (KBr): 3300, 2230, 1790, 1660 cm⁻¹; MS (m/z) 331/333 (M⁺). HPLC indicates a major component (99.6%).

| Elemental analysis for C₁₂H₁₈ClN₃O₂ | |
|---|---|
| Calc'd | C, 61.54; H, 5.47; N, 12.66. |
| Found | C, 60.81; H, 5.40; N, 12.52. |
| | C, 60.81; H, 5.35; N, 12.86. |

### EXAMPLE 120 N-(2-Chloro-4-cyano-benzyl)-N-[2-(1,1-dimethyl-propylamino)-3,4-dioxo-cyclobut-1-enyl)-acetamide

A solution of 3-chloro-4-{[2-(1,1-dimethyl-propylamino)-3,4-dioxo-cyclobut-1-enylamino]-methyl}-benzonitrile (0.829 g, 2.50 mmol, Example 119) in tetrahydrofuran (10 mL) and N,N-dimethylformamide (5 mL) was treated with sodium hydride (0.066 g, 2.75 mmol) and stirring was continued for 15 minutes. Acetic anhydride (0.281 g, 2.75 mmol) was added. After stirring 40 minutes at room temperature, solvent was removed and the residue was partitioned between ethyl acetate and brine, and the brine fraction was extracted with ethyl acetate. The combined ethyl acetate fractions were washed with dilute, aqueous sodium carbonate solution, with brine and dried (anhydrous sodium sulfate). Removal of solvent gave a yellow solid that was chromatographed (gravity, chloroform) on silica. The appropriate fractions were freed of solvent and the foamy residue was solidified by addition-rotoevaporation of hexane (twice). Recrystallization (twice) of the solid from acetone-hexane gave 513 mg of the title compound as a white solid: mp 150.5-151.0 °C (softens 149.5 °C); ¹H NMR (DMSO-d₆) δ 7.90-9.22 (broad hump, 1H), 8.10 (s, 1H), 7.84 (m, 1H), 7.51 (d, 1H), 5.29 (br s, 2H), 2.16 (s, 3H), 1.71 (q, 2H), 1.35 (s, 6H), 0.85 (t, 3H) ppm. IR (KBr): 3300, 3080, 2230, 1790, 1720, 1680 cm⁻¹. MS (m/z) 373/375 (M⁺). HPLC indicates a major component (> 99%).

| Elemental analysis for C₁₉H₂₀ClN₃O₃ | |
|---|---|
| Calc'd | C, 61.04; H, 5.39; N, 11.24. |
| Found | C, 60.67; H, 5.26; N, 11.17. |

### EXAMPLE 121 N-(2-Chloro-4-cyano-benzyl)-N-[2-(1,1-dimethyl-propylamino)-3,4-dioxo-cyclobut-1-enyl]-butyramide

Employing the procedure of Example 120, 3-chloro-4-{[2-(1,1-dimethyl-propylamino)-3,4-dioxo-cyclobut-1-enylamino]-methyl}-benzonitrile (0.929 g, 2.80 mmol, Example 119) was reacted with sodium hydride (0.074 g, 3.08 mmol) and then with butyric anhydride (0.487 gm 3.08 mmol) to yield, after chromatographic purification (gravity, chloroform) on silica, 928 mg of a pale yellow solid. Two recrystallizations of the crude product from diisopropyl ether afforded 0.646 g of the title compound as a white solid: mp 105.5-111.0 °C (softens 104.0 °C). MS (m/z) 402 [M+H]⁺.

| Elemental analysis for C₂₁H₂₄ClN₃O₃ | |
|---|---|
| Calc'd | C, 62.76; H, 6.02; N, 10.46. |
| Found | C, 62.56; H, 5.65. N, 10.46. |

### EXAMPLE 122 3-(2,4-Dimethyl-benzylamino)-4-(1,1-dimethyl-propylamino)-cyclobut-3-ene-1,2-dione

Employing a procedure similar to that of Example 51, 3-butoxy-4-(1,1-dimethyl-propylamino)-cyclobut-3-ene-1,2-dione (1.20 g, 5 mmol, Example 46) and 2,4-dimethylbenzylamine (0.68 g, 5.0 mmol, a mixture of 2,4- and 2,6-dimethylbenzylamine isomers) in tetrahydrofuran (8 mL) was reacted to give 1.09 g of a cream-colored solid. Three recrystallizations of this material gave 0.677 g of a white solid. A 0.496 g portion of this solid was purified by HPLC and the solid isolated from the appropriate eluates was recrystallized from acetonitrile to yield 0.278 g of the title compound as a white solid: mp 177-178 °C (softens 160 °C); ¹H NMR (DMSO-d₆) δ 7.61 (br m, 1H), 7.35 (br s, 1H), 7.17 (d, 1H), 7.03 (s, 1H), 7.00 (m, 1H), 4.70 (d, 2H), 2.27 (s, 3H), 2.25 (s, 3H), 1.65 (q, 2H), 1.29 (s, 6H), 0.80 (t, 3H) ppm. IR (KBr): 3210, 1790, 1640 cm⁻¹. M (m/z) 300 (M⁺). HPLC indicates a major component (99.1%).

| Elemental analysis for C₁₈H₂₄N₂O₂ | |
|---|---|
| Calc'd | C, 71.97; H, 8.05; N, 9.32. |
| Found | C, 71.87; H, 7.99; N, 9.32. |

### EXAMPLE 123 3-(2,3-Dimethyl-benzylamino)-4-(1,1-dimethyl-propylamino)-cyclobut-3-ene-1,2-dione

Tetrahydrofuran (15 mL), 3-butoxy-4-(1,1-dimethyl-propylamino)-cyclobut-3-ene-1,2-dione (1.20 g, 5 mmol), 2,3-dimethylbenzylamine hydrochloride (0.858g, 5 mmol, prepared according to the procedure of R. Fuller et al., J. Med. Chem., 16 (2), 101 (1973)) and triethylamine (0.506 g, 5 mmol) were refluxed 22.5 hours and then processed in a manner similar to that of Example 47, Step 3, to provide 1.337 g of crude product. Recrystallization (twice) of this material from methanol afforded 0.777 g of the title compound as a white solid: mp 196-198 °C. MS (m/z) 300 (M⁺).

| Elemental analysis for C₁₈H₂₄N₂O₂ | |
|---|---|
| Calc'd | C, 71.97; H, 8.05; N, 9.33. |
| Found | C, 71.80; H, 8.23; N, 9.23. |

### EXAMPLE 124 3-(1,1-Dimethyl-propylamino)-4-(2,4,6-trimethyl-benzylamino)-cyclobut-3-ene-1,2-dione

Tetrahydrofuran (15 mL), 3-butoxy-4-(1,1-dimethyl-propylamino)-cyclobut-3-ene-1,2-dione (1.20 g, 5 mmol), 2,4,6-trimethylbenzylamine hydrochloride (0.928 g, 5 mmol, Example 52, Step 2) and triethylamine (0.506 g, 5 mmol) were stirred together at room temperature for 3 days. The mixture was then prepared as in Example 52, Step 3. Three recrystallization of the crude product from acetonitrile provided 0.664 g of the title compound as a white solid: mp 284-5 °C (dec) (softens 282 °C). MS (m/z) 314 (M⁺).

| Elemental analysis for C₁₉H₂₆N₂O₂ | |
|---|---|
| Calc'd | C, 72.58; H, 8.33; N, 8.91. |
| Found | C, 72.61; H, 8.46; N, 9.01. |

### EXAMPLE 125 3-tert-Butylamino-4-(2,5-dichloro-benzylamino)-cyclobut-3-ene-1,2-dione

Employing the procedure of Example 45, Step 4, and a reaction period of 23 hours, 2,5-dichlorobenzylamine (0.88 g, 5 mmol) and 3-butoxy-4-tert-butylamino-cyclobut-3-ene-1,2-dione (1.13 g, 5 mmol) in tetrahydrofuran (15 mL) were converted to 1.52 g of a cream-colored solid. Recrystallization of this material from acetonitrile (twice) and from methanol provided 0.853 g of the title compound as a white solid: mp 236 °C (softens 216 °C). MS (m/z) 326/328/330 (M⁺).

| Elemental analysis for C₁₅H₁₆Cl₂N₂O₂ | |
|---|---|
| Calc'd | C, 55.06; H, 4.93; N, 8.56. |
| Found | C, 55.29; H, 4.76; N, *8.55.* |

### EXAMPLE 126 3-(2,5-Dichloro-benzylamino)-4-(1,1-dimethyl-propylamino)-cyclobut-3-ene-1,2-dione

Tetrahydrofuran (13 mL), 3-butoxy-4-(1,1-dimethyl-propylamino)-cyclobut-3-ene-1,2-dione (1.20 g, 5 mmol) and 2,5-dichlorobenzylamine (0.88 g, 5 mmol) were stirred together for approximately 64.5 hours. Removal of solvent and thorough trituration of the residue with diethyl ether gave 1.51 g of a light yellow solid. As purification of this material by three recrystallizations from acetonitrile was unrewarding, the recovered crude product was subjected to HPLC. The solid isolated from the appropriate fractions was recrystallized from acetonitrile to afford 0.598 g of the title compound as a white solid: mp 195-196 °C MS (m/z) 340/342/344 (M⁺). HPLC indicates a major component (> 99%).

| Elemental analysis for C₁₆H₁₈Cl₂N₂O₂ | |
|---|---|
| Calc'd | C, 56.32; H, 5.32; N, 8.21. |
| Found | C, 56.31; H, 5.26; N, 8.22. |

### EXAMPLE 127 3-tert-Butylamino-4-(3,4-dimethyl-benzylamino)-cyclobut-3-ene-1,2-dione

Tetrahydrofuran (17 mL), 3-butoxy-4-tert-butylamino-cyclobut-3-ene-1,2-dione (1.13 g, 5 mmol, Example 44) and 3,4-dimethylbenzylamine (0.68 g, 5 mmol) were stirred together at room temperature for 2 days. Removal of solvent and trituration of the residue with diethyl ether gave 1.06 g of a light yellow solid. Four recrystallizations of this material from acetonitrile provided 0.394 g of the title compound as a white solid: mp 264-264 °C (softens 260 °C). MS (m/z) 286 (M⁺). HPLC indicates a major component (96%).

| Elemental analysis for C₁₇H₂₂N₂O₂ | |
|---|---|
| Calc'd | C, 71.30; N, 7.74; N, 9.78. |
| Found | C, 71.28; H, 7.71; N, 9.61. |

### EXAMPLE 128 3-(3,4-Dimethyl-benzylamino)-4-(1,1-dimethyl-propylamino)-cyclobut-3-ene-1,2-dione

Tetrahydrofuran (12 mL), 3-butoxy-4-(1,1-dimethyl-propylamino)-cyclobut-3-ene-1,2-dione (1.20 g, 5 mmol), and 3,4-dimethylbenzylamine (0.68 g, 5 mmol) were stirred together at room temperature for 17 hours. Removal of solvent and trituration of the residue with diethyl ether provided 1.04 g of a cream-colored solid. Recrystallization of the crude product from acetonitrile (twice) and from methanol (twice) gave 0.45 g of the title compound as a fluffy, white soid: mp 248 °C (softens 246 °C). MS (m/z) 300 (M⁺). HPLC indicates a major component (97%).

| Elemental analysis for C₁₈H₂₄N₂O₂ | |
|---|---|
| Calc'd | C, 71.97; H, 8.05; N, 9.33. |
| Found | C, 71.91; H, 8.13; N, 9.37. |

### EXAMPLE 129 3-tert-Butylamino-4-(3,5-dimethyl-benzylamino)-cyclobut-3-ene-1,2-dione

### Step 1) Preparation of 3,5-dimethylbenzylamine hydrochloride

The reduction of 3,5-dimethylbenzonitrile with diborane was conducted according to the procedure of R. Fuller et al., J. Med. Chem., 16 (2), 101 (1973), and the amine was converted to the hydrochloride salt with isopropanolic HCl. Recrystallization of the crude salt from methanol-acetonitrile in the presence of several drops of isopropanolic HCl, provided the title compound as a white solid: mp 251-255 °C. MS (m/z) 135 (M⁺, base).

M. Konawalow, Chem. Ber., 28, 1852 (1895) reported mp 245-246°C (dec) for this hydrochloride.

### Step 2) 3-tert-Butylamino-4-(3,5-dimethyl-benzylamino)-cyclobut-3-ene-1,2-dione

The product from Example 129, Step 1 (0.858 g, 5 mmol), 3-butoxy-4-tert-butylamino-cyclobut-3-ene-1,2-dione (1.13 g, 5 mmol, Example 44), triethylamine (0.506 g, 5 mmol), and tetrahydrofuran (15 mL) were refluxed 17 hours and then processed in a manner similar to that of Example 47, Step 3 to give 0.833 g of crude product. Recrystallization (twice) of this material from methanol gave 0.715 g of the title compound as a white solid: mp 246-248 °C. MS (m/z) 286 (M⁺).

| Elemental analysis for C₁₇H₂₂N₂O₂ | |
|---|---|
| Calc'd | C, 71.30; H, 7.74; N, 9.78. |
| Found | C, 71.23; H, 7.79; N, 9.81. |

### EXAMPLE 130 3-(3,5-Dimethyl-benzylamino)-4-(1,1-dimethyl-propylamino)-cyclobut-3-ene-1,2-dione

The product from Example 129, Step 1 (0.858 g, 5 mmol), triethylamine (0.506 g, 5 mmol), 3-butoxy-4-(1,1-dimethyl-propylamino)-cyclobut-3-ene-1,2-dione (1.20 g, 5 mmol, Example 46), and tetrahydrofuran was refluxed approximately 19 hours in a manner similar to that of Example 47, Step 3. The crude product was recrystallized (twice) from methanol to yield 0.81 g of the title compound as a white solid: mp 221.5-224 °C. MS (m/z) 300 (M⁺).

| Elemental analysis for C₁₈H₂₄N₂O₂ | |
|---|---|
| Calc'd | C, 71.97; H, 8.05; N, 9.33. |
| Found | C, 71.60; H, 7.95; N, 9.31. |

### EXAMPLE 131 3-(3,5-Dichloro-benzylamino)-4-(1,1-dimethyl-propylamino)-cyclobut-3-ene-1,2-dione

A solution of 3-butoxy-4-(1,1-dimethyl-propylamino)-cyclobut-3-ene-1,2-dione (1.20 g, 5 mmol, Example 46), 3,5-dichlorobenzylamine (0.88 g, 5 mmol) and tetrahydrofuran (15 mL) was stirred at room temperature for 3 days. The reaction mixture was processed as in Example 45, Step 4. The crude product was recrystallized (twice) from methanol to provide 1.309 g of the title compound as a white solid: mp 257-258 °C (dec). MS (m/z) 340/342/344 (M⁺). HPLC indicates a major component (97%).

| Elemental analysis for C₁₆R₁₈Cl₂N₂O₂ | |
|---|---|
| Calc'd | C, 56.32; H, 5.32; N, 8.21. |
| Found | C, 56.26; H, 5.13; N, 8.06. |

### EXAMPLE 132 3-tert-Butylamino-4-(3,5-dichloro-benzylamino)-cyclobut-3-ene-1,2-dione

A mixture of 3-butoxy-4-tert-butylamino-cyclobut-3-ene-1,2-dione (1.13 g, 5 mmol, Example 44), 3.5-dichlorobenzylamine (0.88 g, 5 mmol) and tetrahydrofuran (15 mL) was stirred at room temperature overnight, and then was processed as in Example 45, Step 4. Recrystallization (twice) of the crude product from methanol gave 1.095 g of the title compound as a whte solid: mp 281-283°C (dec). MS (m/z) 326/328/330 (M⁺). HPLC indicates a major component (99.2%).

| Elemental analysis for C₁₅H₁₆Cl₂N₆O₂ | |
|---|---|
| Calc'd | C, 55.06; H, 4.93; N, 8.56. |
| Found | C, 55.01; H, 4.77; N, 8.57. |

### EXAMPLE 133 3-tert-Butylamino-4-(3-chloro-4-methyl-benzylamino)-cyclobut-3-ene-1,2-dione

### Step 1) Preparation of 3-Chioro-4-methylbenzylamine hydrochloride

The reduction of 3-chloro-4-methylbenzonitrile with diborane was conducted according to the procedure of R. Fuller et al., J. Med. Chem., 16 (2), 101 (1973), and the amine was converted to the hydrochloride salt with isopropanolic HCl. Recrystallization of the crude salt from ethanol, in the presence of a small amount of isopropanolic HCl, gave the title compound as a white solid: mp 260-264°C (softens 254°C). MS (m/z) 155/157 (M⁺ base).

### Step 2) 3-tert-Butylamino-4-(3-chloro-4-methyl-benzylamino)-cyclobut-3-ene-1,3-dione.

The product from Example 133, Step 1 (0.96 g, 5 mmol), 3-butoxy-4-tert-butylamino-cyclobut-3-ene-1,2-dione (1.12 g, 5 mmol. Example 44), triethylamine (0.506 g, 5 mmol), and tetrahydrofuran (25 mL) were refluxed overnight and then was processed in a manner similar to that of Example 47, Step 3. The crude product (1.221 g) was recrystallized (twice) from ethanol to yield 1.06 g of the title compound as a white solid: mp 291-292°C (dec). MS (m/z) 306/308 (M⁺).

| Elemental analysis for C₁₆H₁₉ClN₂O₂ | |
|---|---|
| Calc'd | C, 62.64; H, 6.24; N, 9.13. |
| Found | C, 62.30; H, 6.38; N, 9.04. |

### EXAMPLE 134 3-(3-Chloro-4-methyl-benzylamino)-4-(1,1-dimethyl-propylamino)-cyclobut-3-ene-1,2-dione

The product from Example 133, Step 1 (0.96 g, 5 mmol), 3-butoxy-4-(1,1-dimethyl-propylamino)-cyclobut-3-ene-1,2-dione (1.20 g, 5 mmol, Example 46), triethylamine (0.56 g, 5 mmol), and tetrahydrofuran (15 mL) were refluxed for approximately 22 hours. Processing the reaction mixture in a manner similar to that of Example 47, Step 3 afforded 1.234 g of a white solid. Recrystallization (twice) of the crude product from methanol provided 0.963 g of the title compound as a white solid: mp 261-263°C. MS (m/z) 320/322 (M⁺).

| Elemental analysis for C₁₇H₂₁ClN₂O₂ | |
|---|---|
| Calc'd | C, 63.65; H, 6.60; N, 8.73. |
| Found | C, 63.67; H, 6.63; N, 8.70. |

### EXAMPLE 135 4-[(2-Butoxy-3,4-dioxo-cyclobut-1-enylamino)-methyl]-3-chloro-benzonitrile

A solution of 3,4-dibutoxy-3-cyclobutene-1,2-dione (2.263 g, 10 mmol) and 2-chloro-4-cyanobenzylamine (1.666 g, 10 mmol, Example 45, Step 3) in tetrahydrofuran (25 mL) was stirred at room temperature for 22 hours. The residue remaining after removal of solvent was dissolved in chloroform and was chromatographed (flash, ethyl acetate/hexane) on silica. The solid isolated from the appropriate fractions was recrystallized from ethyl acetate to provide 2.427 g of a white solid: mp 147.5-149°C. MS (m/z) 318 (M⁺). HPLC indicates a major component (> 99%).

| Elemental analysis for C₁₆H₁₅ClN₂O₃ | |
|---|---|
| Calc'd | C, 60.29; H, 4.74; N, 8.79. |
| Found | C, 60.05; H, 4.54; N, 8.69. |

### EXAMPLE 136 3-Chloro-4-{[2-(2-hydroxy-1,1-dimethyl-ethylamino)-3,4-dioxo-cyclobut-1-enylamino]-methyl}-benzonitrile

### Step 1) Preparation of 3-butoxy-4-(2-hydroxy-1,1-dimethyl-ethylamino)-cyclobut-3-ene-1,2-dione

With ice-bath cooling, a solution of 3,4-dibutoxy-3-cyclobutene-1,2-dione (4.526 g, 20 mmol) in tetrahydrofuran (35 mL) was stirred and treated dropwise with a solution of 2-amino-2-methylpropanol (1.783 g, 20 mmol) in tetrahydrofuran (20 mL). The solution then was stirred at room temperature overnight. The brown oil isolated after removal of solvent was chromatographed (flash, ethyl acetate/hexane) on silica. The appropriate fractions were freed of solvent and the residue dried to give 4.122 g of the title compound as a colorless oil: ¹H NMR (DMSO-d₆) δ 8.43 and 8.33 (two m, 1H, rotamers), 4.90 (t, 1H), 4.65 (m, 2H), 3.37 (m, 2H), 1.72 (m, 2H), 1.39 (m, 2H), 1.23 (m, 6H), 0.91 (t, 3H) ppm. IR (film): 3440, 3250, 1790, 1690 cm⁻¹; MS (m/z) 242 ([M+H]⁺).

### Step 2) 3-Chloro-4-{[2-(2-hydroxy-1,1-dimethyl-ethylamino)-3,4-dioxo-cyclobut-1-enylamino)-methyl}-benzonitrile

Tetrahydrofuran (10 mL), the product from Example 136, Step 1 (1.031 g, 5 mmol), and 2-chloro-4-cyanobenzylamine (0.833 g, 5 mmol, Example 45, Step 3) were stirred together at room temperature for approximately 67 hours. Removal of solvent and trituation of the residue with diethyl eher gave 1.18 g of a white solid. mp 220-223°C; ¹H NMR (DMSO-d₆) δ 8.11 (d, 1H), 7.99 (t, 1H), 7.88 (m, 1H), 7.62 (d, 1H), 7.58 (s, 1H), 5.10 (t, 1H), 4.86 (d, 2H), 3.40 (d, 2H), 1.28 (s, 6H) ppm. IR (Kbr): 3320, 3200, 2240, 1790, 1665 cm⁻¹; MS (m/z) 334 ([M+H]⁺).

| Elemental analysis for C₁₆H₁₆ClN₃O₃ | |
|---|---|
| Calc'd | C, 57.58; H, 4.83; N, 12.59. |
| Found | C, 57.51; H, 4.71; N, 12.74. |

### EXAMPLE 137 3-(2,5-Dimethyl-benzylamino)-4-(1,1-dimethyl-propylamino)-cyclobut-3-ene-1,2-dione

### Step 1) Preparation of N-(2,5-dimethylbenzyl)phthalimide

N,N-Dimethylformamide (200 mL), 2,5-dimethylbenzyl chloride (15.5 g, 0.10 mol), and potassium phthalimide (20.38 g, 0.11 mol) were stirred together for 140 minutes. During this period, the reaction temperature rose from 22°C to 31°C and then receded to 27°C. Following removal of solvent in vacuo, the residue was thoroughly triturated with water and dried to give 25.69 g of a white solid. This solid was recrystallized from ethyl acetate and, prior to drying, a 0.99 g portion (wet weight) was further recrystallized from ethyl acetate to provide 0.68 g of N-(2,5-dimethylbenzyl)-phthalimide as a white solid: mp 148-150°C (softens 145°C). MS (m/z) 265 (M⁺).

| Elemental analysis for C₁₇H₁₅NO₂ | |
|---|---|
| Calc'd | C, 76.96; H, 5.70; N, 5.28. |
| Found | C, 76.87; H, 5.57; N, 5.29. |

The amount of dried, once recrystallized N-(2,5-dimethylbenzyl)phthalimide, also exhibiting mp 148-150°C (softens145°C), was 22.75 g.

### Step 2) Preparation of 2,5-dimethylbenzylamine hydrochloride

With mechanical stirring N-(2,5-dimethylphthalimide (22.67 g, 85.5 mmol, Example 137, Step 1), hydrazine hydrate (8.6 g, 170 mmol), and absolute ethanol (200 mL) were refluxed for one hour. After cooling to room temperature, the stirred mixture was treated carefully with 2N hydrochloric acid (123 mL) and stirred forty minutes longer. The mixture was filtered and the insolubles were rinsed with ethanol and with water. Removal of solvent from the combined filtrate and rinsings gave a residue that was treated with ice-water (300 mL). The mixture was basified with 2.5 N sodium hydroxide (123 mL) and then was extracted with chloroform. The combined extracts were washed successively with 1N sodium hydroxide, with water, with brine, and then were dried (anhydrous sodium sulfate).

The brownish-yellow oil remaining after removal of solvent was dissolved in diethyl ether and treated with isopropanolc hydrogen chloride. The solid was collected, rinsed with diethyl ether and dried to yield 12.05 g of an off-white solid.

The crude salt was recrystallized from absolute ethanol and, prior to drying, a 1 g (wet weight) portion was further recrystallized from absolute ethanol to afford 0.464 g of 2,5-dimethylbenzylamine hydrochloride as a white solid: mp 234°C (softens 231°C).

| Elemental analysis for C₉H₁₃N · HCl | |
|---|---|
| Calc'd | C, 62.97; H, 8.22; N, 8.16. |
| Found | C, 63.06; H, 8.28; N, 8.06. |

The yield of dried, once recrystallized 2,5-dimethylbenzylamine hydrochloride, mp 234°C (softens 230°C) was 5.66 g (H. Mix, et al., Z. Physiol. Chem., 343, 52 (1965) reported mp 233°C for 2,5-dimethylbenzylamine hydrochloride).

### Step 3) 3-(2,5-Dimethylbenzylamino)-4-(1,1-dimethyl-propylamino)-cyclobut-3-ene-1,2-dione

Tetrahydrofuran (10 mL), 2,5-dimethylbenzylamine hydrochloride (0.858 g, 5.0 mmol, Example 137, Step 2), triethylamine (0.51 g, 5.0 mmol), and 3-butoxy-4-(1,1-dimethylpropylamino)-cyclobut-3-ene-1,2-dione (1.20 g, 5.0 mmol, Example 46) were stirred together at room temperature for approximately 18 hours. After removal of solvent the residue was triturated with water, with diethyl ether, and dried to give 1.31 g of a light yellow solid. Three recrystallizations of the crude product from acetonitrile provided 0.812 g of the title compound as a fluffy, white solid: mp 199-200°C (softens 195°C). MS (m/z) 301 ([M+H]⁺). HPLC indicates a major component (>99%).

| Elemental analysis for C₁₈H₂₄N₂O₂ | |
|---|---|
| Calc'd | C, 71.97; H, 8.05; N, 9.33. |
| Found | C, 71.89; H, 8.01; N, 9.30. |

### EXAMPLE 138 3-(t-Butylamino)-4-(2,5-dimethyl-benzylamino)-cyclobut-3-ene-1,2-dione

Tetrahydrofuran (10 mL), 3-butoxy-4-tert-butylamino-cyclobut-3-ene-1,2-dione (1.13 g, 5.0 mmol, Example 44), 2,5-dimethylbenzylamine (0.858 g, 5.0 mmol), and triethylamine (0.51 g, 5.0 mmol) were stirred together at room temperature for approximately 18 hours. Following removal of solvent, the residue was triturated with water, with diethyl ether, and dried to give 0.698 g of a cream-colored solid. Three recrystallizations of this material from methanol afforded 0.444 g of the title compound as a white solid: mp 246-247°C (softens 242°C). MS (m/z) 287 ((M+H]⁺). HPLC indicates a major component (>99%).

| Elemental analysis for C₁₇H₂₂N₂O₂ | |
|---|---|
| Calc'd | C, 71.30; H, 7.74; N, 9.78. |
| Found | C, 71.34; H, 7.76; N, 9.77. |

### EXAMPLE 139 3-tert-Butylamino-4-(3-chloro-2-methyl-benzylamino)-cyclobut-3-ene-1,2-dione

### Step 1) Preparation of 3-Chloro-2-methylbenzylamine hydrochloride

A solution of 3-chloro-2-methylbenzonitrile (7.58 g, 50 mmol) in tetrahydrofuran (50 mL) was added slowly with stirring to 1.0 M boranetetrahydrofuran complex (150 mL, 150 mmol) and the mixture was refluxed for 21 hours. With cooling (ice-water bath) and stirring 2N hydrochloric acid (50 mL) was added cautiously and stirring was continued for 40 minutes longer. The reaction mixture was freed of solvent and the residue was partitioned between water and chloroform. As both phases contained solids, they were filtered. The aqueous filtrate was basified with dilute aqueous sodium hydroxide and was extracted with diethyl ether. The extracts were washed with water, with brine, dried (anhydrous sodium sulfate), and concentrated to give 3.21 g of the crude amine.

The solids collected from the original aqueous acidic and chloroform fractions were partially dissolved in water. The mixture was basified with dilute aqueous sodium hydroxide and were extracted thoroughly with diethyl ether. After drying (anhydrous sodium sulfate), the extracts were concentrated to give an additional 2.23 g of crude amine.

Dissolution of the two lots of crude amine in diethyl ether, and addition of isopropanolic hydrogen chloride gave a solid that was rinsed with diethyl ether and dried to yield 5.57 g of a light beige salt.

The crude salt was recrystallized from absolute ethanol in the presence of a small amount of isopropanolic hydrogen chloride. Prior to drying, a 0.98 g (wet weight) portion was recrystallized in the same manner and dried to afford 0.63 g of 3-chloro-2-methylbenzylamine hydrochloride as a white solid: mp 265-266°C (softens 263°C). MS (m/z) 155/157 ([M+H]⁺).

| Elemental analysis for C₈H₁₀ClN · HCl | |
|---|---|
| Calc'd | C, 50.02; H, 5.77; N, 7.29. |
| Found | C, 49.86; H, 5.75; N, 7.23, |

The amount of dried, once recrystallized 3-chloro-2-methylbenzylamine hydrochloride, mp 266-267°C (softens 263°C) obtained was 3.76 g.

### Step 2) 3-tert-Butylamino-4-(3-chloro-2-methyl-benzylamino)-cyclobut-3-ene-1,2-dione

Tetrahydrofuran (10 mL), 3-chloro-2-methylbenzylamine hydrochloride (0.96 g, 5.0 mmol, Example 139, Step 1), triethylamine (0.51 g, 5.0 mmol), and 3-butoxy-4-tert-butylamino-cyclobut-3-ene-1,2-dione (1.13 g, 5.0 mmol) were stirred together at room temperature for 18.5 hours. The residue remaining after removal of solvent was triturated with water, with diethyl ether, and was dried to give 1.11 g of a cream-colored solid. Three recrystallizations of this material from acetonitrile provided 0.80 g of the title compound as a white, fluffy solid: mp 258-259°C (dec) (softens 242°C). MS (m/z) 307 ([M+H]⁺). HPLC indicates a major component (>99%).

| Elemental analysis for C₁₆H₁₉ClN₂O₂ | |
|---|---|
| Calc'd | C, 62.64; H, 6.24; N, 9.13. |
| Found | C, 62.80; H, 6.18; N, 9.11. |

### EXAMPLE 140 3-(3-Chloro-2-methyl-benzylamino)-4-(1,1-dimethyl-propylamino)-cyclobut-3-ene-1,2-dione

Tetrahydrofuran (10 mL), 3-butoxy-4-(1,1-dimethyl-propylamino)-cyclobut-3-ene-1,2-dione (1.20 g, 5.0 mmol, Example 46), 3-chloro-2-methylbenzylamine hydrochloride (0.96 g, 5.0 mmol, Example 139, Step 1), and triethylamine (0.51 g, 5.0 mmol) were stirred together at room temperature for 65 hours. Processing the reaction mixture as in Example 139, Step 2 provided 1.44 g of a white solid. Two recrystallizations of the crude product from methanol gave 1.12 g of the title compound as a fluffy, white solid: mp 236-237°C (softens 233°C). MS (m/z) 321/323 ([M+H]⁺). HPLC indicates a major component (99.6%).

| Elemental analysis for C₁₇H₂₁ClN₂O₂ | |
|---|---|
| Calc'd | C, 63.65; H, 6.60; N, 8.73. |
| Found | C, 63.63; H, 6.59; N, 8.78. |

### EXAMPLE 141 N-(2-tert-Butylamino-3,4-dioxo-cyclobut-1-enyl)-N-(2-chloro-4-cyano-benzyl)-acetamide

Employing the procedure of Example 120, 4-[(tert-butylamino-3,4-dioxo-cyclobut-1-enylamino)-methyl]-3-chlorobenzonitrile (0.731 g, 2.30 mmol), sodium hydride (0.062 g, 2.6 mmol), and acetic anhydride (0.27 g, 2.6 mmol) were reacted in tetrahydrofuran (10 mL) and N,N-dimethylformamide (5 mL) to provide 0.592 g of a yellow solid. This material was chromatographed (flash, chloroform) on silica and the appropriate fractions were concentrated. The residue was solidified by addition-rotoevaporation of hexane (twice) and dried to give 0.353 g of crude product. Recrystallization (twice) of this material from dichloromethane-hexane afforded 0.125 g of the title compound as a pale yellow solid: mp 184-185°C (softens 181°C); ¹H NMR (DMSO-d₆) δ 9.10-8.15 (broad hump, 1H), 8.10 (s 1H), 7.84 (m, 1H), 7.50 (d, 1H), 5.27 (br s, 2H), 2.16 (s, 3H), 1.40 (s, 9H) ppm. IR (KBr): 3270, 2230, 1780, 1720, 1680 cm⁻¹; MS (m/z) 359/361 (M⁺). HPLC indicates a major component (>99.9%).

| Elemental analysis for C₁₈H₁₈ClN₃O₃ | |
|---|---|
| Calc'd | C, 60.09; H, 5.04; N, 11.68. |
| Found | C, 59.61; H, 4.72; N, 11.51. |
| | C, 59.93; H, 4.86; N, 11.50. |

### EXAMPLE 142 3-(2,3-Dichloro-benzylamino)-4-(1,1-dimethyl-propylamino)-cyclobut-3-ene-1,2-dione

Tetrahydrofuran (12 mL), 3-butoxy-4-(1,1-dimethyl-propylamino)-cyclobut-3-ene-1,2-dione (1.20 g, 5.0 mmol) and 2,3-dichlorobenzylamine (0.88 g, 5.0 mmol) were stirred together at room temperature for 64 hours. The residue remaining after removal of solvent was trituated thoroughly with diethyl ether and dried to give 1.55 g of a white solid. Recrystallization (four times) of the crude product from acetonitrile provided 0.796 g of the title compound as a white solid: mp 222-223°C (softens 218°C). MS (m/z) 340/342/344 (M⁺). HPLC indicates a major component (96.2%).

| Elemental analysis for C₁₆H₁₈C₁₂N₂O₂ | |
|---|---|
| Calc | C, 56.32; H, 5.32; N, 8.21. |
| Found | C, 56.39; H, 5.28; N, 8.18. |

### EXAMPLE 143 3-(t-Butylamino)-4-(2,3-dichloro-benzylamino)-cyclobut-3-ene-1,2-dione

Tetrahydrofuran (12 mL), 3-butoxy-4-teit-butylamino-cyclobut-3-ene-1,2-dione (1.13 g, 5.0 mmol, Example 44), and 2,3-dichlorobenzylamine (0.88 g, 5.0 mmol) were stirred together at room temperature for 64 hours. After removal of solvent, trituation of the residue with diethyl ether and drying gave 1.46 g of a white solid. Four recrystallizations of this crude product from acetonitrile provided 0.927 g of the title compound as a white solid: mp 237-243°C (dec). MS (m/z) 327 ([M+H]⁺).

| Elemental analysis for C₁₅H₁₆Cl₂N₂O₂ | |
|---|---|
| Calc'd | C, 55.06; H, 4.93; N, 8.56. |
| Found | C, 54.77; H, 4.81; N, 8.51. |

### EXAMPLE 144 N-(2-tert-Butylamino-3,4-dioxo-cyclobut-1-enyl)-N-(2-chloro-4-cyano-benzyl)-butyramide

Employing the procedure of Example 120, 4-[(tert-butylamino-3,4-dioxo-cyclobut-1-enylamino)-methyl]-3-chlorobenzonitrile (0.887 g, 2.79 mmol), sodium hydride (0.076 g, 3.07 mmol), and butyric anhydride (0.486 g, 3.07 mmol) were reacted in tetrahydrofuran (10 mL) and N,N-dimethylformamide (5 mL) to provide 0.996 g of a golden yellow solid. This material was dissolved in chloroform and was chromatographed (flash, ethyl acetate-hexane) on silica and the appropriate fractions were concentrated. The residue was solidified by addition-rotoevaporation of hexane (twice). Recrystallization (twice) of the solid from t-butyl methyl ether provided 0.539 g of the title compound as a white solid: mp 133-134°C (softens 131°C). MS (m/z) 388/390 ([M+H]⁺).

| Elemental analysis for C₂₀H₂₂ClN₃O₃ | |
|---|---|
| Calc'd | C, 61.93; H, 5.72; N, 10.83. |
| Found | C, 61.59; H, 5.50; N, 10.68. |

### EXAMPLE 145 3-(4-Fluoro-benzylamino)-4-(1,2,2-trimethyl-propylamino)-cyclobut-3-ene-1,2-dione

### Step 1 (R)-3-Ethoxy-4-(1,2,2-trimethyl-propylamino)-cyclobut-3-ene-1,2-dione

A solution of 3,4-diethoxy-3-cyclobutene-1,2-dione (10 g, 59 mmol) and (R)-2-amino-3,3-dimethylbutane (353 mL of a 0.2 M solution in absolute ethanol, 71 mmol) was stirred at room temperature for 24 hours. Another portion of (R)-2-amino-3,3-dimethylbutane (150 mL of a 0.2 M solution in absolute ethanol, 30 mmol) was added, and the resulting solution was stirred at room temperature for 24 hours. The slurry was filtered, and the filtrate concentrated under reduced pressure. The resulting solid was triturated with hexane:ethyl acetate (150:5 mL), and washed with hexane to give 9.78 g (74%) of a white solid: 'H NMR: (DMSO-d₆): δ 8.72 and 8.50 (two d, 1H, rotamers), 4.65 (m, 2H), 3.90 and 3.42 (two m, 1H, rotamers), 1.37 and 1.35 (two overlapping t, 2H, rotamers), 1.10 (two overlapping d, 3H, rotamers), 0.85 and 0.84 (two s, 9H, rotamers) ppm. IR(KBr): 3150, 2950, 1800, 1700 cm⁻¹; MS (m/z): 225 (M⁺); Anal. Calcd. for C₁₂H₁₉NO₃: C, 63.98; H, 8.50; N, 6.22. Found: C, 64.33; H, 8.54; N, 6.52.

### Step 2 (R)-3-(4-Fluoro-benzylamino)-4-(1,2,2-trimethyl-propylamino)-cyclobut-3-ene-1,2-dione

To a solution of 100 mg (0.44 mmol) (R)-3-ethoxy-4-(1,2,2-trimethyl-propylamino)-cyclobut-3-ene-1,2-dione and 10 mL of CH₃CN at room temperature, was added 55 mg (0.44 mmol) of 4-fluoro-benzylamine. The reaction mixture was heated to 80 °C for 12 hours. After cooling to room temperature and evaporation to dryness, 10 mL of Et₂O was added and the mixture was filtered to give 100 mg (0.33 mmol, a 75 % yield) of the (R) isomer of the title compound as a white solid. mp: 301-303 °C; ¹H NMR (300 MHz, DMSO-d₆): δ 0.83-0.90 (m, 9H), 1.09-1.15 (m, 3H), 3.21-3.90 (brs, 1H), 4.71 (brm, 2H), 7.08-7.30 (m, 3H), 7.39-7.48 (m, 3H), 7.61 (brs, 1H); IR (KBr, cm⁻¹): 3195m, 3059w, 2967m, 2874w, 1651m, 1572s, 1511s, 1478m, 1223w; MS (ES) *m/z* (relative intensity): 327 (M⁺+Na, 20), 305 (M⁺+H, 100); Anal. Calcd. for C₁₇H₂₁FN₂O₂: C, 67.09; H, 6.95; N, 9.20. Found: C, 66.82; H, 7.23; N, 9.03.

### EXAMPLE 146 3-(4-Trifluoromethyl-benzylamino)-4-(1,2,2-trimethyl-propylamino)-cyclobut-3-ene-1,2-dione

The (R) isomer of the title compound was prepared according to the procedure for Example 145 - step 2 except 4-trifluoromethyl-benzylamine was used in place of 4-fluoro-benzylamine. Yield: 67%; mp: 316-318 °C; ¹H NMR (300 MHz, DMSO-d₆): δ 0.82-0.90 (m, 9H), 1.07-1.14 (m, 3H), 3.92 (brs, 1H), 4.82 (brm, 2H), 7.05-7.19 (m, 1H), 7.57 (d, J = 8.2 Hz, 2H), 7.76 (d, J = 8.2 Hz, 2H), 7.61 (brs, 1H); IR (KBr, cm⁻¹): 3196m, 3064w, 2969m, 2875w, 1649m, 1570s, 1480m, 1328m, 1127m; MS (ES) *m/z* (relative intensity): 377 (M⁺+Na, 25), 355 (M⁺+H, 100); Anal. Calcd. for C₁₈H₂₁F₃N₂O₂: C, 61.01; H, 5.97; N, 7.99. Found: C, 61.25; H, 6.00; N, 7.92.

### EXAMPLE 147 3-tert-Butylamino-4-(4-trifluoromethoxy-benzylamino)-cyclobut-3-ene-1,2-dione

### Step 1 - 3-Ethoxy-4-(tert-butylamino)-cyclobut-3-ene-1,2-dione

To a room temperature solution of 5.81 g (34 mmol) of 3,4-diethoxy-3-cyclobutene-1,2-dione and 50 mL of THF was added 2.9 g (34 mmol) of tert-butyl amine. After stirring at room temperature for 12 hours, the reaction mixture was evaporated to a yellow oil. Flash chromatography on silica gel, eluting with 0 to 5 % MeOH/CH₂Cl₂ gave 6.2 g (34 mmol, a 100 % yield) of the title compound as a white solid: mp: 80-82°C; ¹H NMR: (300 MHz, DMSO-d₆): δ 1.30-1.62 (m, 12H), 4.70-4.93 (m, 2H), 5.50-6.42 (brm, 1H); IR (KBr, cm⁻¹): 3214w, 3144m, 3071w, 2973m, 2941m, 1794m, 1702s, 1606s, 1577s, 1500-1442brs; MS (CI) *m/z* (relative intensity): 198 (M⁺+H, 100); Anal. Calcd. for C₁₀H₁₅NO₃: C, 60.90; H, 7.67; N, 7.10. Found: C, 61.03; H, 7.66; N, 6.98.

### Step 2 - 3-tert-Butylamino-4-(4-trifluoromethoxy-benzylamino)-cyclobut-3-ene-1,2-dione

The title compound was prepared according to the procedure for Example 145 - step 2 except that 3-ethoxy-4-(tert-butylamino)-cyclobut-3-ene-1,2-dione was used in place of (R)-3-ethoxy-4-(1,2,2-trimethyl-propylamino)-cyclobut-3-ene-1,2-dione and 4-trifluoromethoxy-benzylamine was used in place of 4-fluoro-benzylamine. Yield: 66 %; mp: 292-294 °C; ¹H NMR (300 MHz, DMSO-d₆): δ 1.36 (m, 9H), 4.73-4.79 (m 2H), 7.39 (d, J=8.0 Hz, 2H), 7.76 (d, J=8.2 Hz, 2H), 7.81 (brs, 1H), one N-H proton not seen; IR (KBr, cm⁻¹): 3293s, 3242s, 3061w, 2980m, 1794m, 1655s, 1569s, 1542s, 1472s, 1447s, 1280-1160brs; MS (ES) *m/z* (relative intensity): 365 (M⁺+Na, 100), 343 (M⁺+H, 75); Anal. Calcd. for C₁₆H₁₇F₃N₂O₃: C, 56.14; H, 5.01; N, 8.18. Found: C, 56.28; H, 5.05; N, 7.97.

### EXAMPLE 148 3-tert-Butylamino-4-(4-trifluoromethyl-benzylamino)-cyclobut-3-ene-1,2-dione

The title compound was prepared according to the procedure for Example 145 - step 2 except that 3-ethoxy-4-(tert-butylamino)-cyclobut-3-ene-1,2-dione was used in place of (R)-3-ethoxy-4-(1,2,2-trimethyl-propylamino)-cyclobut-3-ene-1,2-dione and 4-trifluoromethyl-benzylamine was used in place of 4-fluoro-benzylamine. Yield: 63 %; mp: 306-308 °C; ¹H NMR (300 MHz, DMSO-d₆): δ 1.37 (m, 9H), 4.81-4.87 (m 2H), 7.57 (d, J = 7.9 Hz, 2H), 7.76 (d, J = 8.0 Hz, 2H), 7.81 (brm, 1H), one N-H proton not seen; IR (KBr, cm⁻¹): 3291s, 3244s, 3061w, 2980m, 1794m, 1656s, 1567s, 1540s, 1471s, 1327s, 1162s, 1127s; MS (ES) *m/z* (relative intensity): 349 (M⁺+Na, 20), 327 (M⁺+H, 100); Anal. Calcd. for C₁₆H₁₇F₃N₂O₂: C, 58.89; H, 5.25; N, 8.58. Found: C, 59.07; H, 5.34; N, 8.31.

### EXAMPLE 149 3-tert-Butylamino-4-(4-fluoro-benzylamino)-cyclobut-3-ene-1,2-dione

The title compound was prepared according to the procedure for Example 145 - step 2 except that 3-ethoxy-4-(tert-butylamino)-cyclobut-3-ene-1,2-dione was used in place of (R)-3-ethoxy-4-(1,2,2-trimethyl-propylamino)-cyclobut-3-ene-1,2-dione. Yield: 86 %; mp: 296-298 °C; ¹H NMR (300 MHz, DMSO-d₆): δ 1.36 (m, 9H), 4.77-4.83 (m 2H), 7.18-7.29 (m, 2H), 7.40-7.50 (m, 2H), 7.53 (s, 1H), 7.78 (brm, 1H); IR (KBr, cm⁻¹): 3291s, 3240s, 3069w, 2970m, 2973m, 1793m, 1654s, 1540s, 1511s, 1471s, 1455s, 1219s; MS (CI) *m/z* (relative intensity): 277 (M⁺+H, 100); Anal. Calcd. for C₁₅H₁₇FN₂O₂: C, 65.20; H, 6.20; N, 10.14. Found: C, 65.19; H, 6.25; N, 10.06.

### EXAMPLE 150 3-(4-Trifluoromethoxy-benzylamino)-4-(1,2,2-trimethyl-propylamino) cyclobut-3-ene-1,2-dione

The (R) isomer of the title compound was prepared according to the procedure for Example 145 - step 2 except that 4-trifluromethyl-benzylamine was used in place of 4-fluoro-benzylamine. Yield: 61 %; mp: 289-291 °C; ¹H NMR (300 MHz, DMSO-d₆): δ 0.83-0.88 (m, 9H), 1.08-1.14 (m, 3H), 3.91 (brs, 1H), 4.71-4.81 (m, 2H), 7.39 (d, J = 8.1 Hz, 2H), 7.48 (d, J = 8.1 Hz, 2H), 7.65 (brs, 1H), one NH proton not seen; IR (KBr, cm⁻¹): 3196m, 3064w, 2969m, 1796w, 1648m, 1575s, 1511s, 1480s, 1347m, 1264m, 1162s; MS (ES) *m/z* (relative intensity): 393 (M⁺+Na, 20), 371 (M⁺+H, 100); Anal. Calcd. for C₁₈H₂₁F₃N₂O₃: C, 58.37; H, 5.72; N, 7.56. Found: C, 59.20; H, 6.01; N, 7.56.

### EXAMPLE 151 3-(1,1-Dimethyl-propylamino)-4-(4-fluoro-benzylamino)-cyclobut-3-ene-1,2 dione

### Step 1 3-Ethoxy-4-(1,1-dimethyl-propylamino)-cyclobut-3-ene-1,2-dione

To a room temperature solution of 4.9 g (29 mmol) of 3,4-diethoxy-3-cyclobutene-1,2-dione and 50 mL of THF was added 2.5 g (34 mmol) of 1,1-dimethylpropylamine. After stirring at room temperature overnight, the reaction mixture was evaporated to a yellow oil. Trituration with Et₂O and filtration gave 6.2 g (20 mmol, a 100% yield) of the title compound as a white solid: mp: 78-80 °C; ¹H NMR: (300 MHz, DMSO-d₆): δ 0.91 and 1.47 (t, J = 7.5 Hz, 3H, rotamers), 1.36-1.97 (m, 6H), 4.70-4.92 (m, 2H), 5.41 and 5.99 (brs, 1H, rotamers); IR (KBr, cm⁻¹): 3252w, 3181w, 3067w, 2968m, 2887m, 1788m, 1695s, 1603s, 1578s, 1500-1434brs, 1343s; MS (CI) *m/z* (relative intensity): 212 (M⁺+H, 100); Anal. Calcd. for C₁₀H₁₅NO₃: C 62.54; H 8.11; N 6.63. Found: C, 62.54; H, 8.12; N, 6.52.

### Step 2 3-(1,1-Dimethyl-propylamino)-4-(4-fluoro-benzylamino)-cyclobut-3-ene-1,2 dione

The title compound was prepared according to the procedure for Example 145 - step 2 except that 3-ethoxy-4-(1,1-dimethyl-propylamino)-cyclobut-3-ene-1,2-dione was used in place of (R)-3-ethoxy-4-(1,2,2-trimethyl-propylamino)-cyclobut-3-ene-1,2-dione. Yield: 72 %; mp: 255-257 °C; ¹H NMR (300 MHz, DMSO-d₆): δ 0.82 (t, J = 7.5 Hz, 3H), 1.30 (s, 6H), 1.67 (q, J = 7.5 Hz, 2H), 4.69-4.74 (m 2H), 7.19-7.31 (m, 2H), 7.37-7.55 (m, 3H), 7.80 (brs, 1H); IR (KBr, cm⁻¹): 3239m, 3069w, 2972w, 2882w, 1792w, 1652m, 1571s, 1537s, 1511m, 1468w; MS (ES) *m/z* (relative intensity): 313 (M⁺+Na, 30), 291 (M⁺+H, 100); Anal. Calcd. for C₁₆H₁₉FN₂O₂: C, 66.19; H, 6.60; N, 9.65. Found: C, 66.25; H, 6.72; N, 9.51.

### EXAMPLE 152 3-tert-Butylamino-4-(3-fluoro-benzylamino)-cyclobut-3-ene-1,2-dione

The title compound was prepared according to the procedure for Example 145 - step 2 except that 3-ethoxy-4-(tert-butylamino)-cyclobut-3-ene-1,2-dione was used in place of (R)-3-ethoxy-4-(1,2,2-trimethyl-propylamino)-cyclobut-3-ene-1,2-dione and 3-trifluoro-benzylamine was used in place of 4-fluoro-benzylamine. Yield: 75 %; mp: 295-297 °C; ¹H NMR (300 MHz, DMSO-d₆): δ 1.37 (m, 9H), 4.72-4.79 (m 2H), 7.11-7.26 (m, 3H), 7.40-7.49 (m, 1H), 7.54 (brs, 1H), 7.75-7.84 (brm, 1H); IR (KBr, cm⁻¹): 3236s, 3091w, 2971m, 2937m, 1794m, 1654s, 1571s, 1536s, 1470s, 1444s, 1370m, 1248m, 1216m; MS (ES) *m/z* (relative intensity): 299 (M⁺+Na, 15), 277 (M⁺+H, 100); Anal. Calcd. for C₁₅H₁₇FN₂O: C, 65.20; H, 6.20; N, 10.14. Found: C, 65.09; H, 6.28; N, 10.19.

### EXAMPLE 153 3-tert-Butylamino-4-(2-fluoro-benzylamino)-cyclobut-3-ene-1,2-dione

The title compound was prepared according to the procedure for Example 145 - step 2 except that 3-ethoxy-4-(tert-butylamino)-cyclobut-3-ene-1,2-dione was used in place of (R)-3-ethoxy-4-(1,2,2-trimethyl-propylamino)-cyclobut-3-ene-1,2-dione and 2-trifluromethoxy-benzylamine was used in place of 4-fluoro-benzylamine. Yield: 73 %; mp: 260-262 °C; ¹H NMR (300 MHz, DMSO-d₆): δ 1.36 (m, 9H), 4.75-4.81 (m 2H), 7.20-7.30 (m, 2H), 7.35-7.46 (m, 2H), 7.53 (brs, 1H), 7.72-7.80 (brm, 1H); IR (KBr, cm⁻¹): 3221m, 3048w, 2973m, 1793m, 1653s, 1570s, 1537s, 1470-1445brs, 1231m, 1217m; MS (ES) *m/z* (relative intensity): 299 (M⁺+Na, 10), 277 (M⁺+H, 100); Anal. Calcd. for C₁₅H₁₇FN₂O₂: C, 65.20; H, 6.20; N, 10.14. Found: C, 64.99; H, 6.09; N, 9.99.

### EXAMPLE 154 3-tert-Butylamino-4-(3-trifluoromethyl-benzylamino)-cyclobut-3-ene-1,2-dione

The title compound was prepared according to the procedure for Example 145 - step 2 except that 3-ethoxy-4-(tert-butylamino)-cyclobut-3-ene-1,2-dione was used in place of (R)-3-ethoxy-4-(1,2,2-trimethyl-propylamino)-cyclobut-3-ene-1,2-dione and 3-trifluoromethyl-benzylamine was used in place of 4-fluoro-benzylamine. Yield: 73 %; mp: 237-239 °C; ¹H NMR (300 MHz, DMSO-d₆) δ 1.36 (s, 9H), 4.81-4.87 (m, 2H), 7.57 (s, 1H), 7.60-7.77 (m, 4H), 7.82 (brm, 1H); IR (KBr, cm⁻¹): 3233m, 3059w, 2973w, 1793w, 1653s, 1571s, 1540s, 1471m, 1447m, 1354m, 1166m, 1127m; MS (CI) *m/z* (relative intensity): 327 (M⁺+H, 80); Anal. Calcd. for C₁₆H₁₇F₃N₂O₂: C, 58.89; H, 5.25; N, 8.58. Found: C, 58.99; H, 5.23; N, 8.47.

### EXAMPLE 155 3-tert-Butylamino-4-(2-trifluoromethyl-benzylamino)-cyclobut-3-ene-1,2-dione

The title compound was prepared according to the procedure for Example 145 - step 2 except that 3-ethoxy-4-(tert-butylamino)-cyclobut-3-ene-1,2-dione was used in place of (R)-3-ethoxy-4-(1,2,2-trimethyl-propylamino)-cyclobut-3-ene-1,2-dione and 2-trifluoromethyl-benzylamine was used in place of 4-fluoro-benzylamine. Yield: 73 %; mp: 237-239 °C; ¹H NMR (300 MHz, DMSO-d₆): δ 1.37 (m, 9H), 4.81-4.88 (m 2H), 7.56-7.61 (m, 2H), 7.63-7.83 (m, 4H); IR (KBr, cm⁻¹): 3299m, 3224m, 3040w, 2976w, 2940w, 1796m, 1670m, 1650m, 1576s, 1534s, 1468s, 1315s, 1216-1127brm; MS (ES) *m/z* (relative intensity): 349 (M⁺+Na, 20), 327 (M⁺+H, 100); Anal. Calcd. for C₁₆H₁₇F₃N₂O₂: C, 58.89; H, 5.25; N, 8.58. Found: C, 58.84; H, 5.28; N, 8.54.

### EXAMPLE 156 3-tert-Butylamino-4-(3-trifluoromethoxy-benzylamino)-cyclobut-3-ene-1,2-dione

The title compound was prepared according to the procedure for Example 145 - step 2 except that 3-ethoxy-4-(tert-butylamino)-cyclobut-3-ene-1,2-dione was used in place of using (R)-3-ethoxy-4-(1,2,2-trimethyl-propylamino)-cyclobut-3-ene-1,2-dione and 3-trifluoromethoxy-benzylamine was used in place of 4-fluoro-benzylamine. Yield: 66 %; mp: 210-212 °C; ¹H NMR (300 MHz, DMSO-d₆): δ 1.36 (m, 9H), 4.76-4.81 (m 2H), 7.32-7.43 (m, 3H), 7.50-7.60 (m, 2H), 7.76-7.84 (brm, 1H); IR (KBr, cm⁻¹): 3295m, 3239m, 3063w, 2973m, 1794m, 1655s, 1569s, 1542s, 1471s, 1446s, 1259-1168brs; MS (ES) *m/z* (relative intensity): 365 (M⁺+Na, 50), 343 (M⁺+H, 100); Anal. Calcd. for C₁₆H₁₇F₃N₂O₃: C, 56.14; H, 5.01; N, 8.18. Found: C, 55.91; H, 4.70; N, 7.99.

### EXAMPLE 157 3-(3-Trifluoromethyl-benzylamino)-4-(1,2,2-trimethyl-propylamino)-cyclobut-3-ene-1,2-dione

The (R) isomer of the title compound was prepared according to the procedure for Example 145 - step 2 except that 3-trifluromethyl-benzylamine was used in place of 4-fluoro-benzylamine. Yield: 76 %; mp: 236-238 °C; ¹H NMR (300 MHz, DMSO-d₆): δ 0.83-0.90 (m, 9H), 1.09-1.15 (m, 3H), 3.88-3.96 (m, 1H), 4.75-4.90 (m, 2H), 7.15-7.38 (m, 1H), 7.59-7.82 (m, 5H); IR (KBr, cm⁻¹): 3201m, 3061w, 2969m, 2876w, 1790w, 1649m, 1569brs, 1480m, 1329s, 1167m, 1126m; MS (CI) *m/z* (relative intensity): 355 (M⁺+H, 20); Anal. Calcd. for C₁₈H₂₁F₃N₂O₂: C, 61.01; H, 5.97; N, 87.90. Found: C, 61.41; H, 5.94; N, 7.96.

### EXAMPLE 158 3-(2-Trifluoromethyl-benzylamino)-4-(1,2,2-trimethyl-propylamino)-cyclobut-3-ene-1,2-dione

The (R) isomer of the title compound was prepared according to the procedure for Example 145 - step 2 except that 2-trifluromethyl-benzylamine was used in place of 4-fluoro-benzylamine. Yield: 76 %; mp: 216-218 °C; ¹H NMR (300 MHz, DMSO-d₆): δ 0.83-0.90 (m, 9H), 1.10-1.16 (m, 3H), 3.88-3.97 (m, 1H), 4.80-4.91 (m, 2H), 7.17-7.40 (m, 1H), 7.59-7.82 (m, 5H); IR (KBr, cm⁻¹): 3210m, 3066w, 2967m, 2875w, 1664m, 1586s, 1541s, 1476m, 1315s, 1165m, 1122m; MS (CI) *m/z* (relative intensity): 355 (M⁺+H, 100); Anal. Calcd. for C₁₈H₂₁F₃N₂O₂: C, 61.01; H, 5.97; N, 7.90. Found: C, 61.23; H, 5.69; N, 7.91.

### EXAMPLE 159 3-(2-Fluoro-benzylamino)-4-(1,2,2-trimethyl-propylamino)-cyclobut-3-ene-1,2-dione

The (R) isomer of the title compound was prepared according to the procedure for Example 145 - step 2 except that 2-fluoro-benzylamine was used in place of 4-fluoro-benzylamine. Yield: 66 %; mp: 275-277 °C; ¹H NMR (300 MHz, DMSO-d₆): δ 0.84-0.9 (m, 9H), 1.08-1.14 (m, 3H), 3.85-3.96 (m, 1H), 4.70-4.90 (m, 2H), 7.15-7.32 (m, 3H), 7.37-7.55 (m, 2H), 7.60-7.76 (m, 1H); IR (KBr, cm⁻¹): 3193m, 3065w, 2967m, 2874m, 1648m, 1574s, 1545s, 1489-1421brm, 1368w; MS (ES) *m/z* (relative intensity): 327 (M⁺+Na, 20), 305 (M⁺+H, 100); Anal. Calcd. for C₁₇H₂₁FN₂O₂: C, 67.09; H, 6.95; N, 9.20. Found: C, 66.79; H, 7.16; N, 9.08.

### EXAMPLE 160 3-(3-Trifluoromethoxy-benzylamino)-4-(1,2,2-trimethyl-propylamino)-cyclobut-3-ene-1,2-dione

The (R) isomer of the title compound was prepared according to the procedure for Example 145 - step 2 except that 3-trifluoromethoxy-benzylamine was used in place of 4-fluoro-benzylamine. Yield: 60 %; mp: 236-238 °C; ¹H NMR (300 MHz, DMSO-d₆): δ 0.84-0.90 (m, 9H), 1.10-1.15 (m, 3H), 3.88-3.97 (m, 1H), 4.73-4.85 (m, 2H), 7.15-7.45 (m, 4H), 7.50-7.58 (m, 1H), 7.62-7.77 (m, 1H); IR (KBr, cm⁻¹): 3201m, 3065w, 2968m, 2876w, 1648m, 1567s, 1481m, 1449m, 1262m, 1216m; MS (ES) *m/z* (relative intensity): 393 (M⁺+Na, 25), 371 (M⁺+H, 100); Anal. Calcd. for C₁₈H₂₁F₃N₂O₃: C, 58.37; H, 5.72; N, 7.56. Found: C, 59.02; H, 5.99; N, 7.57.

### EXAMPLE 161 (2,4-Dichloro-6-methyl-benzyl)-[2-(1,1-dimethyl-propylamino)-3,4-dioxo-cyclobut-1-enyl]-carbamic acid tert-butyl ester

### Step 1) Preparation of (2,4-dichloro-6-methyl-benzyl)-[2-butoxy-3,4-dioxo-cyclobut-1-enyl]-carbamic acid tert-butyl ester

To 2,4-dichloro-6-methylbenzylamine (2.0 g, 10.5 mmol) in tetrahydrofuran (15 mL) was added 3,4-dibutoxy-3-cyclobutene-1,2-dione (2.85 g, 12.6 mmol). The mixture was stirred at room temperature under nitrogen for 24 h. The reaction mixture was concentrated under reduced pressure and the resulting residue was recrystallized from a mixture of ethyl acetate and hexane. The solid was filtered and dried. This gave 3.15 g (84%) of a solid, which was used without further purification. A portion of this solid, 3-(2,4-dichloro-6-methyl-benzylamino)-4-butoxy-cyclobut-3-ene-1,2-dione, (1.12 g, 3.1 mmol) in dichloromethane (20 mL) was mixed with triethylamine (0.44 mL, 3.1 mmol), di-*tert*-butyl dicarbonate (1.45 g, 6.6 mmol), and 4-dimethylaminopyridine (0.38 g, 3.1 mmol). The reaction mixture was stirred at room temperature under nitrogen for 24 h. The resulting solution was filtered through a plug of silica gel, which was subsequently washed with a solution of 1:1 hexane:ethyl acetate (100 mL). The filtrate was concentrated under reduced pressure to give 1.25 g (86%) of a solid: ¹H NMR (DMSO-d₆) δ 7.43 (s, 1H), 7.32 (s, 1H), 5.10 (s, 2H), 4.74 (t, 2H), 2.38 (s, 3H), 1.71 (m, 2H), 1.40 - 1.29 (m overlappping a singlet at δ 1.31, 11H), 0.89 (t, 3H).

### Step 2) (2,4-Dichloro-6-methyl-benzyl)-[2-(1,1-dimethyl-propylamino)-3,4-dioxo-cyclobut-1-enyl]-carbamic acid tert-butyl ester

The product from Example 161, Step 1 (1.20 g, 2.63 mmol) was dissolved in tetrahydrofuran 30 mL). 1,1-Dimethylpropylamine (0.62 mL, 5.3 mmol) was added. The reaction mixture was stirred at room temperature under nitrogen for 3 days. The solvent was removed under reduced pressure, and the resulting yellow oil was triturated with diethyl ether and hexane to give 1.15 g (95%) of the title compound as a white solid: mp 141.2-142.5°C; 'H NMR (DMSO-d₆) δ 7.94 (br s, 1H), 7.46 (d, 1H), 7.34 (d, 1H), 5.25 (s, 2H), 2.36 (s, 3H), 1.71 (q, 2H), 1.35 (s, 6H), 1.25 (s, 9H), 0.85 (t, 3H). MS (m/z) 454/456/458 (M⁺).

| Elemental analysis for C₂₂H₂₈Cl₂N₂O₄ | |
|---|---|
| Calc'd | C, 58.03; H, 6.20; N, 6.15. |
| Found | C, 58.09; H, 6.11; N, 6.09. |

The smooth muscle relaxing activity of the compounds of this invention was established in accordance with standard pharmacologically accepted test procedures with representative compounds as follows:

Sprague-Dawley rats (150-200 g) are rendered unconscious by CO₂ asphyxiation and then euthanized by cervical dislocation. The bladder is removed into warm (37 deg.C) physiological salt solution (PSS) of the following composition (mM): NaCl, 118.4; KCl, 4.7; CaCl₂, 2.5; MgSO₄, 4.7; H₂O, 1.2; NaHCO₃, 24.9; KH₂PO₄, 1.2; glucose, 11.1; EDTA, 0.023; gassed with 95% O₂; 2/5% CO₂; pH 7.4. The bladder is opened and then cut into strips 1-2 mm in width and 7-10 mm in length. The strips are subsequently suspended in a 10 mL tissue bath under an initial resting tension of 1.5 g. The strips are held in place by two surgical clips, one of which is attached to a fixed hook while the other is attached to an isometric force transducer. The preparations, which usually exhibit small spontaneous contractions, are allowed to recover for a period of 1 hour prior to a challenge with 0.1 µM carbachol. The carbachol is then washed out and the tissue allowed to relax to its resting level of activity. Following a further 30 minute period of recovery, an additional 15 mM of KCl is introduced into the tissue bath. This increase in KCl concentration results in a large increase in the amplitude of spontaneous contractions (and initiation of contractions in previously quiescent strips) superimposed upon a small increase in basal tone. Following stabilization of this enhanced level of contractile activity, incremental increases in the concentration of test compound or vehicle are introduced into the tissue bath. Contractile activity is measured for each compound or vehicle concentration during the last minute of a 30 minute challenge.

The isometric force developed by the bladder strips is measured using a concentration required to elicit 50% inhibition of pre-drug contractile activity (IC₅₀ concentration) and is calculated from this concentration-response curve. The maximum percentage inhibition of contractile activity evoked by a test compound is also recorded for concentrations of test compound less than or equal to 30 µM.

The results of this study are shown in Table I.

**Table I**

| Inhibition of Contractions in Isolated Rat Bladder Strips | | |
|---|---|---|
| Compound | n | IC₅₀ (µM) |
| Example 1 | 2 | 0.29±0.04 |
| Example 2 | 3 | 1.1±0.4 |
| Example 3 | 7 | 14.0±4.7 |
| Example 4 | 2 | 1.3±0.2 |
| Example 5 | 4 | 0.30±0.01 |
| Example 6 | 3 | 21.9±7.2 |
| | 2 | I^{a}=25±7% |
| Example 7 | 4 | 21.3±7.2 |
| Example 8 | 2 | I^{a}=29±1% |
| | 1 | C^{b}=3% |
| Example 9 | 4 | 9.4±2.7 |
| Example 10 | 3 | 10.2±2.2 |
| | 1 | I^{a}=25% |
| Example 11 | 4 | I^{a}=15.6±3.3% |
| Example 12 | 4 | 4.0±1.1 |
| Example 13 | 4 | 2.2±0.5 |
| | 2 | I^{a}=37.0±1.8% |
| Example 14 | 1 | 14.4 |
| | 1 | C^{b}=22% |
| Example 15 | 2 | 12.2±10.2 |
| | 4 | I^{a}=38±3.9% |
| | 4 | C^{b}=32.1±13.2% |
| Example 16 | 4 | 18.7±6.8 |
| | 1 | I^{a}=43% |
| | 1 | C^{b}=6% |
| Example 17 | 4 | I^{a}=20±6.4% |
| Example 18 | 2 | I^{a}=34.7±8.7% |
| | 1 | C^{b}=10% |
| Example 20 | 4 | 2.0±1.0 |
| Example 22 | 4 | 9.8±5.1 |
| Example 24 | 2 | 0.11±0.002 |
| Example 25 | 2 | 15.8±0.05 |
| Example 26 | 2 | C^{b}=82.8±26.0% |
| | 1 | I^{a}=10.2% |
| Example 27 | 8 | 0.20±0.06 |
| Example 29 | 4 | 1.3±0.6 |
| Example 30 | 2 | 0.22±0.08 |
| Example 32 | 4 | I^{a}=22±5.9% |
| Example 33 | 6 | 2.0±0.8 |
| Example 34 | 2 | 9.35±0.46 |
| | 4 | I^{a}=36±3.2% |
| Example 35 | 2 | C^{b}=245±15% |
| Example 36 | 3 | I^{a}=26±13.8% |
| Example 37 | 2 | C^{b}=92.5±44.2% |
| Example 38 | 2 | 2.53±0.19 |
| Example 40 | 2 | 24.0±9.3 |
| Example 41 | 2 | 0.34±0.22 |
| Example 42 | 4 | 1.48±0.57 |
| | 3 | I^{a}=32.5±3.1% |
| | 1 | C^{b}=30% |
| Example 43 | 4 | 8.9±4.0 |
| | 2 | C^{b}=57.9±4.2% |
| Example 45 | 3 | 0.14±0.02 |
| Example 47 | 3 | 0.28±0.03 |
| Example 49 | 3 | 0.66±0.20 |
| | 1 | I^{a}=41.2% |
| Example 50 | 4 | 0.86±0.27 |
| Example 51 | 4 | 0.63±0.15 |
| Example 52 | 3 | 1.8±0.7 |
| Example 53 | 4 | 1.11±0.41 |
| Example 55 | 2 | 6.6±1.6 |
| | 2 | I^{a}=37±0.7% |
| Example 56 | 4 | I^{a}=19.1±1.7% |
| Example 57 | 3 | I^{a}=27.2±2.4% |
| | 1 | C^{b}=54% |
| Example 58 | 4 | 11.1±0.4 |
| Example 59 | 4 | 2.9±0.1 |
| Example 60 | 4 | 9.6±5.7 |
| Example 61 | 4 | 2.1±0.9 |
| Example 62 | 4 | 7.5±4.5 |
| Example 63 | 4 | 8.6±3.5 |
| Example 64 | 4 | 3.9±1.1 |
| Example 65 | 3 | 25±10.0 |
| Example 66 | 4 | 2.7±0.3 |
| Example 67 | 4 | 27.1±4.0 |
| Example 68 | 4 | 31±4.5 |
| Example 69 | 4 | 5.2±0.6 |
| Example 70 | 4 | 0.24±0.03 |
| Example 71 | 4 | 2.0±0.2 |
| Example 72 | 4 | I^{a}=38.6±3.6% |
| Example 73 | 4 | 0.18±0.03 |
| | 1 | I^{a}=23% |
| Example 74 | 4 | 1.1±0.3 |
| Example 75 | 4 | 4.35±4.1 |
| Example 76 | 2 | 0.16±0.01 |
| Example 77 | 4 | 1.9±1.1 |
| Example 78 | 3 | 8.9±3.0 |
| Example 79 | 2 | 0.29±0.02 |
| Example 80 | 3 | 1.2±1.1 |
| Example 81 | 2 | C^{b}=200% |
| Example 82 | 3 | 0.71±0.50 |
| Example 83 | 2 | 0.2±0.08 |
| Example 84 | 2 | I^{a}=41±2.9% |
| Example 85 | 4 | 4.7±1.2 |
| Example 86 | 4 | 1.1±0.1 |
| Example 87 | 2 | 0.26±0.015 |
| Example 88 | 4 | I^{a}=25±2.04% |
| Example 89 | 4 | 6.1±3.8 |
| Example 90 | 3 | 16.7±6.2 |
| Example 91 | 3 | 0.27±0.08 |
| Example 92 | 2 | 1.0±0.60 |
| Example 93 | 2 | 0.105±0.005 |
| Example 94 | 1 | 5.6 |
| | 1 | I^{a}=44% |
| | 3 | C^{b}=22±3.2% |
| Example 95 | 4 | 5.9±1.8 |
| Example 96 | 2 | 0.83±0.66 |
| Example 97 | 1 | 17.8 |
| | | I^{a}=46% |
| Example 98 | 4 | 1.5±0.4 |
| Example 99 | 4 | 0.95±0.75 |
| Example 100 | 3 | I^{a}=29.3±2.3% |
| | 1 | C^{b}=160% |
| Example 101 | 6 | 2.3±0.24 |
| | 1 | I^{a}=25% |
| | | |
| Example 102 | 3 | 1.8±0.6 |
| Example 103 | 4 | 1.4±0.4 |
| Example 104 | 3 | 1.7±1.0 |
| Example 105 | 2 | 0.22±0.04 |
| Example 106 | 3 | 1.5±1.0 |
| Example 107 | 5 | 4.9±2.7 |
| Example 108 | 2 | 0.85±0.41 |
| Example 109 | 2 | 0.98±0.40 |
| Example 110 | 3 | 5.3±1.2 |
| | 1 | I^{a}=47.7% |
| Example 111 | 5 | 2.2±0.8 |
| Example 112 | 3 | 5.4±2.1 |
| Example 113 | 2 | 1.6±0.77 |
| Example 114 | 4 | 2.6±0.4 |
| Example 115 | 5 | 6.5±3.6 |
| Example 116 | 2 | 23.5±8.1 |
| | 2 | I^{a}=35.8±4.9% |
| Example 117 | 2 | 0.75±0.45 |
| Example 118 | 2 | 1.59±0.27 |
| Example 119 | 4 | 0.06±0.014^{c} |
| Example 120 | 3 | 2.2±0.95 |
| | 1 | I^{a}=25% |
| Example 121 | 3 | 2.8±1.3 |
| | 2 | I^{a}=18.0±6.5% |
| | 2 | C^{b}=28.5±8.7% |
| Example 122 | 3 | 0.53±0.23 |
| Example 123 | 2 | 5.0±3.5 |
| Example 124 | 3 | 0.31±0.08 |
| Example 125 | 2 | 0.68±0.24 |
| Example 126 | 2 | 0.81±0.32 |
| Example 127 | 2 | 1.6±0.18 |
| Example 128 | 2 | I^{a}=35.1±4.4% |
| Example 129 | 4 | 5.1±0.96 |
| Example 130 | 2 | I^{a}=13.6±8.4% |
| Example 131 | 4 | 7.3±6.0 |
| Example 132 | 2 | 2.3±0.85 |
| Example 133 | 4 | I^{a}=16.6±3.6% |
| Example 134 | 4 | I^{a}=30.7±6.7% |
| Example 136 | 3 | 8.1±2 |
| | 1 | I^{a}=44% |
| Example 137 | 6 | 1.91±0.84 |
| Example 138 | 2 | 1.5±0.3 |
| Example 139 | 2 | 1.3±0.3 |
| Example 140 | 2 | 0.28±0.025 |
| Example 141 | 4 | 20.3±0.79 |
| Example 142 | 4 | 0.79±0.47 |
| Example 143 | 2 | 1.24±0.17 |
| Ecample 144 | 2 | 2.36±1.6 |
| Example 145 | 3 | 0.76±0.46 |
| Example 146 | 4 | 6.5+1.9 |
| Example 147 | 1 | 16.2^{a} |
| Example 148 | 3 | 6.5±0.87 |
| Example 149 | 4 | 1.5±0.32 |
| Example 150 | 1 | 20.4^{a} |
| Example 151 | 4 | 0.36±0.14 |
| Example 152 | 3 | 2.4±0.36 |
| Example 153 | 4 | 2.08±0.25 |
| Example 154 | 4 | 7.9±2.7 |
| Example 155 | 4 | 2.3±0.51 |
| Example 156 | 3 | 13.6±3.3 |
| Example 157 | 4 | 40.1^{a} |
| Example 158 | 2 | 20.4±7.8 |
| Example 159 | 4 | 1.23±0.22 |

| Compound | n | IC₅₀(µM) |
|---|---|---|
| Example 160 | 1 | 10^{a} |
| Example 161 | 3 | 4.5±4.2 |

| | | |
|---|---|---|
| ^{a} Percent inhibition at 30 µM | | |
| ^{b} Percent contraction at 30 µM | | |
| ^{c} A second lot (mp 215.5-216.0°C (softens 211.5°C)) exhibited an IC₅₀=0.13±0.03µM (n=6). | | |

In addition, we tested the ability of compounds to inhibit the hyperactivity of hypertrophied bladder (detrussor) smooth muscle in conscious female rats with hypertrophied bladders and thereby alleviate urinary incontinence in rats according to the following protocol described by Malmgren et al., J. Urol. 142:1134, 1989:

Female Sprague-Dawley rats, ranging in weight from 190-210 g are used. Up to 25 animals are prepared each time. After development of bladder hypertrophy 4-8 animals are used per test.

Compounds are dissolved in PEG-200 and administered by gastric gavage or intravenously in a volume of 5 ml/kg. For primary screening all drugs are administered at the arbitrary dose of 10 mg/kg p.o. to groups of 4 rats.

The animals are anesthetized with halothane. Through a midline incision the bladder and urethra are exposed and a ligature of 4-0 silk is tied around the proximal urethra in the presence of a stainless steel rod (1 mm diameter) to produce a partial occlusion. The rod is then removed. The abdominal region is closed using surgical staples and each rat receives 150,000 units of bicillin C-R. The animals are allowed six weeks to develop sufficient bladder hypertrophy. After six weeks, the ligature is removed under halothane anesthesia and a catheter (PE 60) with a cuff is placed in the dome of the bladder and secured with a purse string suture. The catheter is tunneled under the skin and exteriorized through an opening in the back of the neck. The abdominal incision is sutured and the free end of the catheter sealed. In order to prevent infections the rats receive an injection of bieillin C-R (150000 units/rat). Two days later the animals are used in cystometrical evaluations, The animals are placed in the metabolic cages and the catheter is attached (using a "T" connector) to a Statham pressure transducer (Model P23Db) and to a Harvard infusion pump. A plastic beaker attached to a force displacement transducer (Grass FT03) is placed under the rat's cage to collect and record urine volume. Animals are allowed 15-30 minutes to rest before the saline infusion (20 ml/hr for 20 minutes) is started for the first cystometry period. Two hours after the first cystometry period, the rats are dosed with the vehicle or the test compound and one hour later a second cystometry is performed.

The following urodynamic variables are recorded:
- Basal bladder pressure =: the lowest bladder pressure during cystometry
- Threshold pressure =: bladder pressure immediately prior to micturition
- Micturition volume =: volume expelled
- Micturition pressure =: peak pressure during voiding
- Spontaneous activity =: mean amplitude of bladder pressure fluctuations during filling

### Presentation of results:

The mean value of each variable is calculated before and after compound administration. For each compound the changes in the variables measured are compared to the values obtained before treatment and expressed as percent inhibition. The data are also subjected to 2-way analysis of variance to determine significant (p<0.05) changes in the variable measured. The most characteristic finding in this rat model is spontaneous bladder contractions which develop during filling.

The results of this study are shown in Table II.

**Table II**

| **Inhibition of Spontaneous Contractions In Vivo** | | | |
|---|---|---|---|
| Compound | # of animals | dose mg/kg (p.o.) | % Red (F)^{d} |
| Example 1 | 3 | 10 mg/kg | -53±2 |
| Example 2 | 4 | 3 mg/kg | -26±7 |
| Example 4 | 4 | 10 mg/kg | -30±8 |
| Example 5 | 6 | 10 mg/kg | -27±7 |
| Example 15 | 5 | 10 mg/kg | -52±12 |
| Example 16 | 3 | 10 mg/kg | -23±9 |
| Example 24 | 7 | 1 mg/kg | -40±13^{f} |
| | 10 | 3 mg/kg | -58±9^{f} |
| Example 27 | 6 | 1 mg/kg | -63±13 |
| | 4 | 3 mg/kg | -82±5 |
| Example 30 | 3 | 3 mg/kg | -53±18 |
| Example 35 | 2 | 10 mg/kg | -87±2 |
| Example 45 | 3 | 3 mg/kg | -52±17^{e} |
| | 4 | 10 mg/kg | -93±3^{e} |
| Example 49 | 4 | 3 mg/kg | -51±6 |
| | 3 | 10 mg/kg | -71±3 |
| Example 51 | 3 | 3 mg/kg | -38±7 |
| Example 70 | 4 | 10 mg/kg | -20±22 |
| Example 73 | 3 | 3 mg/kg | -68±10 |
| Example 74 | 4 | 10 mg/kg | -31±13 |
| Example 79 | 4 | 3 mg/kg | -83±9 |
| Example 119 | 3 | 3 mg/kg | -57±12 |
| Example 145 | 4 | 3 mg/kg | 3±25 |
| Example 151 | 4 | 3 mg/kg | 67±2 |
| Compound | # of animals | dose mg/kg (p.o.) | %Ampl^{g} |
| Example 145 | 4 | 3 mg/kg | 3±25 |
| Example 151 | 4 | 3 mg/kg | 67±2 |

| | | | |
|---|---|---|---|
| ^{d} Percent reduction in the total number of spontaneous contractions in the hypertrophied rat bladder model | | | |
| ^{e} Determined on a prior lot of this compound (mp 234-236°C (softens 225°C)) exhibiting an IC₅₀=0.57±0.34 µM (n=4) | | | |
| ^{f}Findings obtained on a prior lot of this compound, mp. 195-196°C, IC₅₀=0.21±0.04 µM (n=4) | | | |
| ^{g} Percent change in the amplitude of spontaneious contractions in the hypertrophied rat bladder model | | | |

Hence, the compounds of this invention have a pronounced effect on smooth muscle contractility and are useful in the treatment of urinary incontinence, irritable bladder and bowel disease, asthma, hypertension, stroke, and similar diseases as mentioned above, which are amenable to treatment with potassium channel activating compounds by administration, orally parenterally, or by aspiration to a patient in need thereof.

## Claims

1. A compound of the formula (I): 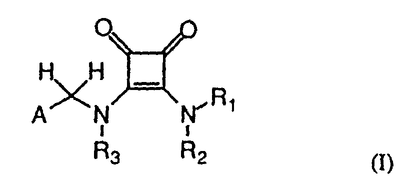 wherein:
R₁ is straight chain alkyl of I to 10 carbon atoms, branched chain alkyl of 3 to 10 carbon atoms, cycloalkyl of 3 to 10 carbon atoms, hydroxyalkyl of 2 to 10 carbon atoms or fluoroalkyl of 1 to 10 carbon atoms;
R₂ and R₃ are, independently, hydrogen or an acyl substituent selected from the group consisting of formyl, alkanoyl of 2 to 7 carbon atoms, alkenoyl of 3 to 7 carbon atoms, straight chain alkoxycarbonyl of 2 to 11 carbon atoms, branched chain alkoxycarbonyl of 4 to 11 carbon atoms, cycloalkoxycarbonyl of 4 to 11 carbon atoms, alkenoxycarbonyl of 2 to 11 carbon atoms, aralkoxycarbonyl of 6 to 12 carbon atoms, alkylsulfonyl of 1 to 7 carbon atoms, aroyl of 7 to 12 carbon atoms, arylalkenoyl of 9 to 20 carbon atoms, arylsulfonyl of 6 to 12 carbon atoms, arylalkanoyl of 8 to 12 carbon atoms or arylalkylsulfonyl of 7 to 12 carbon atoms; with the proviso that when R₃ is straight chain alkoxycarbonyl of 2 to 11 carbon atoms, branched chain alkoxycarbonyl of 4 to 11 carbon atoms, cycloalkoxycarbonyl of 4 to 11 carbon atoms, alkenoxycarbonyl of 2 to 11 carbon atoms or aralkoxycarbonyl of 6 to 12 carbon atoms, R₂ must be hydrogen;
A is a substituted phenyl group of the following formula: 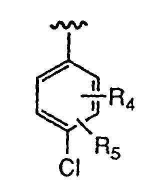 wherein:
R₄ and R₅ are, independently, cyano, nitro, amino, alkyl of 1 to 6 carbon atoms, fluoroalkyl of 1 to 6 carbon atoms, alkoxy of 1 to 6 carbon atoms, fluoroalkoxy of 1 to 6 carbon atoms, amino, alkylamino of 1 to 6 carbon atoms, dialkylamino of 2 to 12 carbon atoms, sulfamyl, alkylsulfonamido of 1 to 6 carbon atoms, arylsulfonamido of 6 to 12 carbon atoms, carbamoyl, alkylcarbamoyl of 2 to 7 carbon atoms, dialkylcarbamoyl of 4 to 14 carbon atoms, alkylcarboxamido containing 2 to 7 carbon atoms, arylcarboxamido containing 7 to 13 carbon atoms, alkylsulfonyl of 1 to 6 carbon atoms, perfluoroalkylsulfonyl of 1 to 6 carbon atoms, arylsulfonyl of 6 to 12 carbon atoms, chloro, bromo, fluoro, iodo, 1-imidazolyl, carboxyl or hydrogen, with the proviso that R₄ and R₅ cannot both be hydrogen;
or A is a substitued phenyl group of the following formula : 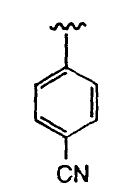 or A is a phenyl group with either two or three substituents of the following formula: 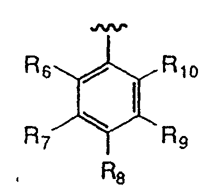 wherein:
the positions of substitution are R₆,R₇-, R₆,R₈-, R₆,R₉-, R₆,R₁₀-, R₇,R₈-, R₇,R₉-, and
R₆,R₈,R₁₀- and R₆ is methyl, ethyl, fluoro, chloro, methoxy or trifluoromethyl;
R₇ is methyl, ethyl, fluoro, chloro, methoxy or trifluoromethyl;
R₈ is methyl, fluoro, bromo, methoxy or cyano;
R₉ is methyl, fluoro, chloro, methoxy, cyano or trifluoromethyl;
R₁₀ is methyl, fluoro, chloro, or methoxy;
or A is a substituted phenyl group of the following formula: 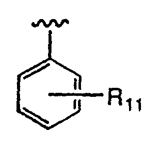 wherein:
R₁₁ is F, perfluoroalkyl group of 1 to 10 carbon atoms or perfluoroalkoxy of 1 to 10 carbon atoms; or a pharmaceurically acceptable salt thereof.

2. A compound according to claim 1 in which R₁ is straight chain alkyl of 1 to 10 carbon atoms, branched chain alkyl of 3 to 10 carbon atoms or fluoroalkyl of 1 to 10 carbon atoms.

3. A compound according to claim 1 or 2 in which R₁ is branched alkyl of 3 to 10 carbon atoms or polyfluoroalkyl of 1 to 10 carbon atoms.

4. A compound according to any of the preceding claims in which R₂ and R₃ are, independently, hydrogen, alkanoyl of 2 to 7 carbon atoms, alkenoyl of 3 to 7 carbon atoms, aroyl of 7 to 12 carbon atoms, arylalkenoyl of 9 to 20 carbon atoms, straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms; with the proviso that when R₃ is straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms, R₂ must be hydrogen; or a pharmaceutically acceptable salt thereof.

5. A compound according to any of the preceding claims in which R₂ and R₃ are, independently, hydrogen, alkanoyl of 2 to 7 carbon atoms, alkenoyl of 3 to 7 carbon atoms, straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms; with the proviso that when R₃ is straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms, R₂ must be hydrogen, or a pharmaceutically acceptable salt thereof.

6. A compound according to any of the preceding claims in which R₂ and R₃ are, independently, hydrogen, alkanoyl of 2 to 7 carbon atoms, alkenoyl of 3 to 7 carbon atoms, aroyl of 7 to 12 carbon atoms, or arylalkenoyl of 9 to 20 carbon atoms; or a pharmaceutically acceptable salt thereof.

7. A compound according to any of the preceding claims in which R₂ and R₃ are, independently, hydrogen, alkanoyl of 2 to 7 carbon atoms or alkenoyl of 3 to 7 carbon atoms; or a pharmaceutically acceptable salt thereof.

8. A compound according to any of the preceding claims in which A is a phenyl group with either two or three substituents of the following formula: 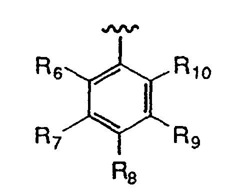 wherein:
the positions of substitution are R₆,R₇-, R₆,R₈-, R₆,R₉-, R₆,R₁₀-,
R₇,R₈₋, R₇,R₉-, and R₆,R₈,R₁₀- and
R₆ is methyl, ethyl, fluoro, chloro, methoxy or trifluoromethyl;
R₇ is methyl, ethyl, fluoro, chloro, methoxy or trifluoromethyl;
R₈ is methyl, fluoro, bromo, methoxy or cyano;
R₉ is methyl, fluoro, chloro, methoxy, cyano or trifluoromethyl;
R₁₀ is methyl, fluoro, chloro, or methoxy;
or a pharmaceutically acceptable salt thereof.

9. A compound according to claim 8 in which
R₆ is methyl, ethyl or chloro;
R₇ is methyl ethyl or chloro;
R₈ is methyl, bromo or cyano;
R₉ is cyano, chloro, or methyl;
R₁₀ is methyl or choro;
or a pharmaceutically acceptable salt thereof;

10. A compound according to claim 8 in which
the substitutional variations are at position combinations R₆,R₈- or R₇,R₈- and
R₆ is trifluoromethyl, fluoro or chloro;
R₇ is fluoro or chloro;
R₈ is fluoro;
or a pharmaceutically acceptable salt thereof;

11. A compound according to claim 8 in which the substitutional variations are at position combinations R₆,R₉-, R₆,R₁₀- or R₇,R₉- and
R₆ is methyl, fluoro or chloro;
R₇ is fluoro;
R₉ is fluoro or trifluoromethyl;
R₁₀ is fluoro;
or a pharmaceutically acceptable salt thereof;

12. A compound according to claim 8 in which the positions of substitution are R₆,R₇-, R₆,R₈- or R₆,R₈,R₁₀-:
where
R₆ is methyl, ethyl or chloro;
R₇ is methyl or chloro;
R₈ is methyl, bromo or cyano;
R₁₀ is methyl or chloro;
or a pharmaceutically acceptable salt thereof

13. A compound according to claim 8 in which the positions of substitution are R₇,R₈-, R₇,R₉-, R₆,R₉-, or R₆,R₁₀-:
where
R₆ is methyl or chloro;
R₇ is methyl, ethyl or chloro;
R₈ is cyano or methyl;
R₉ is cyano, chloro, or methyl;
R₁₀ is methyl or chloro;
or a pharmaceutically acceptable salt thereof

14. A compound according to any of claims 1 to 7 in which A is a substituted phenyl group of the following formula: 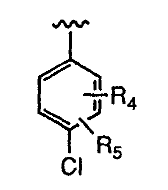 wherein:
R₄ and R₅ are, independently, cyano, nitro, amino, alkyl of 1 to 6 carbon atoms, fluoroalkyl of 1 to 6 carbon atoms, alkoxy of 1 to 6 carbon atoms, fluoroalkoxy of 1 to 6 carbon atoms, amino, alkylamino of 1 to 6 carbon atoms, dialkylamino of 2 to 12 carbon atoms, sulfamyl, alkylsulfonamido of 1 to 6 carbon atoms, arylsulfonamido of 6 to 12 carbon atoms, carbamoyl, alkylcarbamoyl of 2 to 7 carbon atoms, dialkylcarbamoyl of 4 to 14 carbon atoms, alkylcarboxamido containing 2 to 7 carbon atoms, arylcarboxamido containing 7 to 13 carbon atoms, alkylsulfonyl of 1 to 6 carbon atoms, perfluoroalkylsulfonyl of 1 to 6 carbon atoms, arylsulfonyl of 6 to 12 carbon atoms, chloro, bromo, fluoro, iodo, 1-imidazolyl, carboxyl or hydrogen, with the proviso that R₄ and R₅ cannot both be hydrogen.

15. A compound according to claim 14 wherein:
R₄ and R₅ are, independently, cyano, alkyl of 1 to 6 carbon atoms, fluoroalkyl of 1 to 6 carbon atoms, alkoxy of 1 to 6 carbon atoms, fluoroalkoxy of 1 to 6 carbon atoms, chloro, bromo, fluoro, iodo or hydrogen, with the proviso that R₄ and R₅ cannot both be hydrogen; or a pharmaceutically acceptable salt thereof.

16. A compound according to claim 14 or 15 wherein:
R₄ and R₅ are, independently, cyano, methyl, ethyl, trifluoromethyl, fluoroalkyl of 1 to 2 carbon atoms, methoxy, ethoxy, trifluoromethoxy, fluoroalkoxy of 1 to 2 carbon atoms, chloro, bromo, fluoro or hydrogen, with the proviso that R₄ and R₅ cannot both be hydrogen; or a pharmaceutically acceptable salt thereof.

17. A compound according to any of claims 1 to 7 in which A is a substituted phenyl group of the following formula :

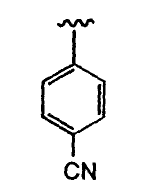

18. A compound according to any of claims 1 to 7 in which A is a substituted phenyl group of the following formula: 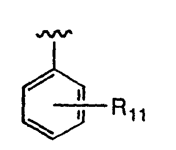
wherein R₁₁ is F, perfluoroalkyl group of 1 to 10 carbon atoms or perfluoroalkoxy of 1 to 10 carbon atoms;
or a pharmaceutically acceptable salt thereof.

19. The compound of Claim 1 which is:
4- {[3,4-dioxo-2-(1,2,2-trimethyl-propylamino)-cyclobut-1-enylamino]-methyl}-benzonitrile or a pharmaceutically acceptable salt thereof;
4-{[2-(1,2-dimethyl-propylamino)-3,4-dioxo-cyclobut-1-enylamino]-methyl}-benzonitrile or a pharmaceutically acceptable salt thereof;
4-[(2-isopropylamino-3,4-dioxo-cyclobut-1-enylamino)-methyl]-benzonitrile or a pharmaceutically acceptable salt thereof;
4-{[2-(1-ethyl-propylamino)-3,4-dioxo-cyclobut-1-enylamino]-methyl]-benzonitrile or a pharmaceutically acceptable salt thereof;
4-[(2-tert-butylamino-3,4-dioxo-cyclobut-1-enylamino)-methyl]-benzonitrile or a pharmaceutically acceptable salt thereof;
4-[(3,4-dioxo-2-propylamino-cyclobut-1-enylamino)-methyl]-benzonitrile or a pharmaceutically acceptable salt thereof;
4-{[2-(2-hydroxy-1,1-dimethyl-ethylamino)-3,4-dioxo-cyclobut-1-enylamino]-methyl)-benzonitrile or a pharmaceutically acceptable salt thereof;
4-[(2-butylamino-3,4-dioxo-cyclobut-1-enylamino)-methyl]-benzonitrile or a pharmaceutically acceptable salt thereof;
4-[(2-cyclopentylamino-3,4-dioxo-cyclobut-1-enylamino)-methyl)-benzonitrile or a pharmaceutically acceptable salt thereof;
4-[(2-isobptylamino-3,4-dioxo-cyclobut-1-enylamino)-methyl]-benzonitrile or a pharmaceutically acceptable salt thereof;
4-[(2-methylamino-3,4-dioxo-cyclobut-1-enylamino)-methyl]-benzonitrile or a pharmaceutically acceptable salt thereof;
(S)-4-[2-sec-butylamino-3,4-dioxo-cyclobut- 1-enylamino)-methyl)-benzonitrile or a pharmaceutically acceptable salt thereof;
(R)-4-[(2-sec-butylamino-3,4-dioxo-cyclobut-1-enylamino)-methyl]-benzonitrile or a pharmaceutically acceptable salt thereof;
4-{[3,4-dioxo-2-(2,2,3,3,3-pentafluoro-propylamino)-cyclobut-1-enylamino]-methyl}-benzonitrile or a pharmaceutically acceptable salt thereof;
N-[2-(4-cyano-benzylamino)-3,4-dioxo-cyclobut-1-enyl]-N-(1,2,2-trimethyl-propyl)-butyramide or a pharmaceutically acceptable salt thereof;
N-(4-cyano-benzyl)-N-[3,4-dioxo-2-(1,2,2-trimethyl-propylamino)-cyclobut-1-enyl]-but-2-enamide or a pharmaceutically acceptable salt thereof;
pentanoic acid (4-cyano-benzyl)-[3,4-dioxo-2-(1,2,2-trimethyl-propylamino)-cyclobut-1-enyl]-amide or a pharmaceutically acceptable salt thereof;
hexanoic acid (4-cyano-benzyl)-[3,4-dioxo-2-(1,2,2-trimethyl-propylamino)-cyclobut-1-enyl]-amide or a pharmaceutically acceptable salt thereof;
3-(2,4-dichlorobenzylamino)-4-(1,2,2-trimethylpropylamino)-cyclobut-3-ene-1,2-dione or a pharmaceutically acceptable salt thereof;
3-(2,4-dichloro-6-methyl-benzylamino)-4-(1,2,2-trimethyl-propylamino)-cyclobut-3-ene-1,2-dione or a pharmaceutically acceptable salt thereof;
3-(2,4-dichlorobenzylamino)-4-(1,1-dimethylpropylamino)-cyclobut-3-ene-1,2-dione or a pharmaceutically acceptable salt thereof;
N-(2,4-dichlorobenzyl)-N-[2-(1,1-dimethyl-propylamino)-3,4-dioxo-cyclobut-1-enyl]-acetamide or a pharmaceutically acceptable salt thereof;
N-(2,4-dichlorobenzyl)-N-[2-(1,1-dimethyl-propylamino)-3,4-dioxo-cyclobut-1-enyl]-butyramide or a pharmaceutically acceptable salt thereof;
3-(2,4-dichloro-6-methyl-benzylamino)-4-(1,1-dimethyl-propylamino)-cyclobut-3-ene-1,2-dione or a pharmaceutically acceptable salt thereof;
3-(t-butylamino)-4-(2,4-dichlorobenzylamino)-cyclobut-3-ene-1,2-dione or a pharmaceutically acceptable salt thereof;
3-tert-butylamino-4-(2,4-dichloro-6-methyl-benzylamino)-cyclobut-3-ene-1,2-dione or a pharmaceutically acceptable salt thereof;
3-(3,4-dichlorobenzylamino)-4-(1-ethyl-propylamino)-cyclobut-3-ene-1,2-dione or a pharmaceutically acceptable salt thereof;
3-(2,4-dichlorobenzylamino)-4-(1-ethyl-propylamino)-cyclobut-3-ene-1,2-dione or a pharmaceutically acceptable salt thereof;
3-(2,4-dichloro-6-methyl-benzylamino)-4-(2,2,3,3,3-pentafluoro-propylamino)-cyclobut-3-ene-1,2-dione or a pharmaceutically acceptable salt thereof;
N-(2,4-dichloro-6-methyl-benzyl)-N-[2-(1,1-dimethyl-propylamino)-3,4-dioxo-cyclobut-1-enyl]-butyramide or a pharmaceutically acceptable salt thereof;
N-(2,4-dichloro-6-methyl-benzyl)-N-[2-(1,1-dimethyl-propylamino)-3,4-dioxo-cyclobut-1-enyl]-acetamide or a pharmaceutically acceptable salt thereof;
N-(tert-butyl)-N-[2-(2,4-dichloro-6-methyl-benzylamino)-3,4-dioxo-cyclobut-1-enyl]-propionamide or a pharmaceutically acceptable salt thereof;
3-(2,4-dichloro-6-methyl-benzylamino)-4-(1,2-dimethyl-2-fluoro-propylamino)-cyclobut-3-ene-1,2-dione or a pharmaceutically acceptable salt thereof;
3-(2,4-dichlorobenzylamino)-4-(2-hydroxy-1,1-dimethylethylamino)-cyclobut-3-ene-1,2-dione or a pharmaceutically acceptable salt thereof;
(R)-3-(2,4-dichlorobenzylamino)-4-(1,2,2-trimethylpropylamino)-cyclobut-3-ene-1,2-dione or a pharmaceutically acceptable salt thereof;
3-tert-butylamino-4-(3,4-dichlorobenzylamino)-cyclobut-3-ene-1,2-dione or a pharmaceutically acceptable salt thereof;
3-(3,4-dichlorobenzylamino)-4-(1,1-dimethylpropylamino)-cyclobut-3-ene-1,2-dione or a pharmaceutically acceptable salt thereof;
(2,4-Dichloro-6-methyl-benzyl)-[2-(1,1-dimethyl-propylamino)-3,4-dioxo-cyclobut-1-enyl]-carbamic acid tert-butyl ester or a pharmaceutically acceptable salt thereof;
4-[(2-tert-butylamino-3,4-dioxo-cyclobut-1-enylamino)-methyl]-3-chloro-benzonitrile or a pharmaceutically acceptable salt thereof;
3-(2,6-dichloro-benzylamino)-4-(1,1-dimethyl-propylamino)-cyclobut-3-ene-1,2-dione or a pharmaceutically acceptable salt thereof;
3-chloro-4- {[3,4-dioxo-2-(1,2,2-trimethylpropylamino)-cyclobut-1-enylamino]-methyl}-benzonitrile or a pharmaceutically acceptable salt thereof;
3-{[2-(1,1-dimethyl-propylamino)-3,4-dioxocyclobut-1-enylamino]-methyl}-5-methyl-benzonitrile or a pharmaceutically acceptable salt thereof;
3-tert-butylamino-4-(2,4-dimethyl-benzylamino)-cyclobut-3-ene-1,2-dione or a pharmaceutically acceptable salt thereof;
4-[(2-tert-butylamino-3,4-dioxo-cyclobut-1-enylamino)-methyl]-3-ethyl-benzonitrile or or a pharmaceutically acceptable salt thereof;
(R)-3-chloro-4-{[3,4-dioxo-2-(1,2,2-trimethyl-propylamino)-cyclobut-1-enylamino]-methyl}-benzonitrile or a pharmaceutically acceptable salt thereof;
3-chloro-4-{[2-(1,1-dimethyl-propylamino)-3,4-dioxo-cyclobut-1-enylamino]-methyl}-5-methyl-benzonitrile or a pharmaceutically acceptable salt thereof;
3-(4-bromo-2,6-dimethyl-benzylamino)-4-tert-butylamino-cyclobut-3-ene-1,2-dione or a pharmaceutically acceptable salt thereof;
3-tert-butylamino-4-(2-chloro-4,6-dimethyl-benzylamino)-cyclobut-3-ene-1,2-dione or a pharmaceutically acceptable salt thereof;
3-(2-chloro-4,6-dimethyl-benzylamino)-4-(1,1-dimethyl-propylamino)-cyclobut-3-ene-1,2-dione or a pharmaceutically acceptable salt thereof;
2-chloro-4-{[2-(1,1-dimethyl-propylamino]-3,4-dioxo-cyclobut-1-enylamino)-methyl}-benzonitrile or a pharmaceutically acceptable salt thereof;
3-(4-bromo-2-ethyl-benzylamino)-4-tert-butylamino-cyclobut-3-ene-1,2-dione or a pharmaceutically acceptable salt thereof;
4-[(1,1-dimethyl-propylamino-3,4-dioxo-cyclobut-1-enylamino)-methyl]-3-ethyl-benzonitrile or a pharmaceutically acceptable salt thereof;
3-(2,6-dimethyl-benzylamino)-4-(1,1-dimethyl-propylamino)-cyclobut-3-ene-1,2-dione or a pharmaceutically acceptable salt thereof;
3-chloro-4-([2-(1,1-dimethyl-propylamino)-3,4-dioxo-cyclobut-1-enylamino)-methyl}-benzonitrile or a pharmaceutically acceptable salt thereof;
3-(2,4-dimethyl-benzylamino)-4-(1,1-dimethyl-propylamino)-cyclobut-3-ene-1,2-dione or a pharmaceutically acceptable salt thereof;
3-(1,1-dimethyl-propylamino)-4-(2,4,6-trimethyl-benzylamino)-cyclobut-3-ene-1,2-dione or a pharmaceutically acceptable salt thereof;
3-tert-butylamino-4-(2,5-dichloro-benzylamino)-cyclobut-3-ene-1,2-dione or a pharmaceutically acceptable salt thereof;
3-(2,5-dichloro-benzylamino)-4-(1,1-dimethyl-propylamino)-cyclobut-3-ene-1,2-dione or a pharmaceutically acceptable salt thereof;
3-(3-chloro-2-methyl-benzylamino)-4-(1,1-dimethyl-propylamino)-cyclobut-3-ene-1,2-dione or a pharmaceutically acceptable salt thereof;
3-(2,4-difluoro-benzylamino)-4-(1,1-dimethyl-propylamino)-cyclobut-3-ene-1,2-dione or a pharmaceutically acceptable salt thereof;
3-(2-chloro-5-fluoro-benzylamino)-4-(1,2,2-trimethyl-propylamino)-cyclobut-3-ene-1,2-dione or a pharmaceutically acceptable salt thereof;
3-(2,5-difluoro-benzylamino)-4-(1,2,2-trimethyl-propylamino)-cyclobut-3-ene-1,2-dione or a pharmaceutically acceptable salt thereof;
3-(3,5-difluoro-benzylamino)-4-(1,1-dimethyl-propylamino)-cyclobut-3-ene-1,2-dione or a pharmaceutically acceptable salt thereof;
4-[(2-tert-butylamino-3,4-dioxo-cyclobut-1-enylamino)-methyl]-3-chloro-benzonitrile or a pharmaceutically acceptable salt thereof;
3-(2,6-dichloro-benzylamino)-4-(1,1-dimethyl-propylamino)-cyclobut-3-ene-1,2-dione or a pharmaceutically acceptable salt thereof;
3-chloro-4-{[3,4-dioxo-2-(1,2,2-trimethylpropylamino)-cyclobut-1-enylamino]-methyl}-benzonitrile or a pharmaceutically acceptable salt thereof;
3-{[2-(1,1-dimethyl-propylamino)-3,4-dioxo-cyclobut-1-enylamino]-methyl}-5-methyl-benzonitrile or a pharmaceutically acceptable salt thereof;
3-tert-butylamino-4-(2,4-dimethyl-benzylamino)-cyclobut-3-ene-1,2-dione or a pharmaceutically acceptable salt thereof;
3-tert-butylamino-4-(2,4,6-trimethyl-benzylamino)-cyclobut-3-ene-1,2-dione or a pharmaceutically acceptable salt thereof;
3-tert-butylamino-4-(2,6-dichloro-benzylamino)-cyclobut-3-ene-1,2-dione or a pharmaceutically acceptable salt thereof;
3-{[3,4-dioxo-2-(1,2,2-trimethyl-propylamino)-cyclobut-1-enylamino]-methyl}-5-methyl-benzonitrile or a pharmaceutically acceptable salt thereof;
3-{[2-(1-ethyl-propylamino)-3,4-dioxo-cyclobut-1-enylamino]-methyl)-5-methyl-benzonitrile or a pharmaceutically acceptable salt thereof;
3-(3,4-dimethoxy-benzylamino)-4-(1-ethyl-propylamino)-cyclobut-3-ene-1,2-dione or a pharmaceutically acceptable salt thereof;
3-tert-butylamino-4-(2,4,6-trimethoxy-benzylamino)-cyclobut-3-ene-1,2-dione or a pharmaceutically acceptable salt thereof;
3-[(2-tert-butylamino-3,4-dioxo-cyclobut-1-enylamino)-methyl]-5-methyl-benzonitrile or a pharmaceutically acceptable salt thereof;
3-tert-butylamino-4-(2,4-dimethoxy-benzylamino)-cyclobut-3-ene-1,2 -dione or a pharmaceutically acceptable salt thereof;
3-tert-butylamino-4-(2,4-difluoro-benzylamino)-cyclobut-3-ene-1,2-dione or a pharmaceutically acceptable salt thereof;
3-tert-butylamino-4-(2,6-difluoro-benzylamino)-cyclobut-3-ene-1,2-dione or a pharmaceutically acceptable salt thereof;
3-tert-butylamino-4-(2,3-dimethoxy-benzylamino)-cyclobut-3-ene-1,2-dione or a pharmaceutically acceptable salt thereof;
3-tert-butylamino-4-(2,5-difluoro-benzylamino)-cyclobut-3-ene-1,2-dione or a pharmaceutically acceptable salt thereof;
3-tert-butylamino-4-(3,5-dimethoxy-benzylamino)-cyclobut-3-ene-1,2-dione or a pharmaceutically acceptable salt thereof;
3-tert-butylamino-4-(2,3-dimethyl-benzylamino)-cyclobut-3-ene-1,2-dione or a pharmaceutically acceptable salt thereof;
4-[(2-butylamino-3,4-dioxo-cyclobut-1-enylamino)-methyl]-3-ethyl-benzonitrile or a pharmaceutically acceptable salt thereof;
3-ethyl-4-[(2-isopropylamino-3,4-dioxo-cyclobut-1-enylamino)-methyl-benzonitrile or a pharmaceutically acceptable salt thereof;
3-ethyl-4-{[(2-(1-ethyl-propylamino)-3,4-dioxo-cyclobut-1-enylamino]-methyl}-benzonitrile or a pharmaceutically acceptable salt thereof;
4-[(2-tert-butylamino-3,4-dioxo-cyclobut-1-enylamino)-methyl]-3-ethyl-benzonitrile or a pharmaceutically acceptable salt thereof;
4-{[3,4-dioxo-2-(1,2,2-trimethyl-propylamino)-cyclobut-1-enylamino]-methyl}-3-ethyl-benzonitrile or a pharmaceutically acceptable salt thereof;
3-(2-chloro-6-methyl-benzylamino)-4-(1,2,2-trimethyl-propylamino)-cyclobut-3-ene-1,2-dione or a pharmaceutically acceptable salt thereof;
(R)-3-chloro-4-{[3,4-dioxo-2-(1,2,2-trimethyl-propylamino)-cyclobut-1-enylaminol-methyl)-benzonitrile or a pharmaceutically acceptable salt thereof;
3-tert-butylamino-4-(2-chloro-6-methyl-benzylamino)-cyclobut-3-ene-1,2-dione or a pharmaceutically acceptable salt thereof;
3-(2-chloro-6-methyl-benzylamino)-4-(1,1-dimethyl-propylamino)-cyclobut-3-ene-1,2-dione or a pharmaceutically acceptable salt thereof;
3-chloro-4-{[2-(1,1-dimethyl-propylamino)-3,4-dioxo-cyclobut-1-enylamino]-methyl}-5-methyl-benzonitrile or a pharmaceutically acceptable salt thereof;
3-chloro-4-{[3,4-dioxo-2-(1,2,2-trimethyl-propylamino)-cyclobut-1-enylamino]-methyl}-5-methyl-benzonitrile or a pharmaceutically acceptable salt thereof;
3-(4-bromo-2,6-dimethyl-benzylamino)-4-(1,2,2-trimethyl-propylamino)-cyclobut-3-ene-1,2-drone or a pharmaceutically acceptable salt thereof;
3-(4-bromo-2,6-dimethyl-benzylamino)-4-tert-butylamino-cyclobut-3-ene-1,2-dione or a pharmaceutically acceptable salt thereof;
3-(4-bramo-2,6-dimethyl-benzylamino)-4-(1,1-dimethyl-propylamino)-cyclobut-3-ene-1,2-dione or a pharmaceutically acceptable salt thereof;
3-(2-chloro-4,6-dimethyl-benzylamino)-4-(1,2,2-trimethyl-propylamino)-cyclobut-3-ene-1,2-dione or a phannaceutically acceptable salt thereof;
3-tert-butylamino-4-(2-chloro-4,6-dimethyl-benzylamino)-cyclobut-3-ene-1,2-dione or a pharmaceutically acceptable salt thereof;
3-(2-chloro-4,6-dimethyl-benzylamino)-4-(1,1-dimethyl-propylamino)-cyclobut-3-ene-1,2-dione or a pharmaceutically acceptable salt thereof;
3-(2-chloro-4,6-dimethyl-benzylamino)-4-(2,2,3,3,3-pentafluoro-propylamino)-cyclobut-3-ene-1,2-dione or a pharmaceutically acceptable salt thereof;
2-chloro-4-{[3,4-dioxo-2-(1,2,2-trimethyl-propylamino)-cyclobut-1-enylamino]-methyl}-benzonitrile or a pharmaceutically acceptable salt thereof;
4-[(2-tert-butylamino-3,4-dioxo-cyclobut-1-enylamino)-methyl]-2-chloro-benzonitrile or a pharmaceutically acceptable salt thereof;
2-chloro-4-{[2-(1,1-dimethyl-propylamino)-3,4-dioxo-cyclobut-1-enylamino]-methyl}-benzonitrile or a pharmaceutically acceptable salt thereof;
4-{[3,4-dioxo-2-(1,2,2-trimethyl-propylamino)-cyclobut-1-enylamino]-methyl}-2-ethyl-benzonitrile or a pharmaceutically acceptable salt thereof;
4-{(2-tert-butylamino-3,4-dioxo-cyclobut-1-enylamino)-methyl]-2-ethyl-benzonitrile or a pharmaceutically acceptable salt thereof;
3-(4-bromo-2-ethyl-benzylamino)-4-(1,2,2-trimethyl-propylamino)-cyclobut-3-ene-1,2-dione or a pharmaceutically acceptable salt thereof;
3-(4-bromo-2-ethyl-benzylamino)-4-tert-butylamino-cyclobut-3-ene-1,2-dione or a pharmaceutically acceptable salt thereof;
3-(4-bromo-2-ethyl-benzylamino)-4-(1,1-dimethyl-propylamino)-cyclobut-3-ene-1,2-dione or a pharmaceutically acceptable salt thereof;
4-[(1,1-dimethyl-propylamino-3,4-dioxo-cyclobut-1-enylamino)-methyl]-3-ethyl-benzonitrile or a pharmaceutically acceptable salt thereof;
3-(2,6-dimethyl-benzylamino)-4-(1,2,2-trimethyl-propylamino)-cyclobut-3-ene-1,2-dione or a pharmaceutically acceptable salt thereof;
3-tert-butylamino-4-(2,6-dimethyl-benzylamino)-cyclobut-3-ene-1,2-dione or a pharmaceutically acceptable salt thereof;
3-(2,6-dimethyl-benzylamino)-4-(1,1-dimethyl-propylamino)-cyclobut-3-ene-1,2-dione or a pharmaceutically acceptable salt thereof;
3-(2,6-dimethyl-benzylamino)-4-(2,2,3,3,3-pentafluoro-propylamino)-cyclobut-3-ene-1,2-dione or a pharmaceutically acceptable salt thereof;
4-{[3,4-dioxo-2-(1,2,2-trimethyl-propylamino)-cyclobut- -enylamino]-methyl)-3-methyl-benzonitrile or a pharmaceutically acceptable salt thereof;
4-{[3,4-dioxo-2-(1,2,2-trimethyl-propylamino)-cyclobut-1-enylamino]-methyl}-3-methoxy-benzonitrile or a pharmaceutically acceptable salt thereof;
3-(2-methoxy-6-methyl-benzylamino)-4-(1,2,2-trimethyl-propylamino)-cyclobut-3-ene-1,2-dione or a pharmaceutically acceptable salt thereof;
3-tert-butylamino-4-(2-ethyl-6-methyl-benzylamino)-cyclobut-3-ene-1,2-dione or a pharmaceutically acceptable salt thereof;
3-tert-butylamino-4-(4-fluoro-2-trifluoromethyl-benzylamino)-cyclobut-3-ene-1,2-dione or a pharmaceutically acceptable salt thereof;
3-tert-butylamino-4-(2-chloro-4-fluoro-benzylamino)-cyclobut-3-ene-1,2-dione or a pharmaceutically acceptable salt thereof;
3-(4-fluoro-2-trifluoromethyl-benzylamino)-4-(1,2,2-trimethyl-propylamino)-cyclobut-3-ene-1,2-dione or a pharmaceutically acceptable salt thereof;
3-(2,4-difluoro-benzylamino)-4-(1,1-dimethyl-propylamino)-cyclobut-3-ene-1,2-dione or a pharmaceutically acceptable salt thereof;
3-(2-chloro-4-fluoro-benzylamino)-4-(1,1-dimethyl-propylamino)-cyclobut-3-ene-1,2-dione or a pharmaceutically acceptable salt thereof;
3-tert-butylamino-4-(2-fluoro-5-trifluoromethyl-benzylamino)-cyclobut-3-ene-1,2-dione or a pharmaceutically acceptable salt thereof;
3-(2-chloro-5-fluoro-benzylamino)-4-(1,2,2-trimethyl-propylamino)-cyclobut-3-ene-1,2-dione or a pharmaceutically acceptable salt thereof;
3-(2,5-difluoro-benzylamino)-4-(1,2,2-trimethyl-propylamino)-cyclobut-3-ene-1,2-dione or a pharmaceutically acceptable salt thereof;
3-tert-butylamino-4-(3-chloro-4-fluoro-benzylamino)-cyclobut-3-ene-1,2-dione or a pharmaceutically acceptable salt thereof;
3-tert-butylamino-4-(3,4-difluoro-benzylamino)-cyclobut-3-ene-1,2-dione or a pharmaceutically acceptable salt thereof;
3-(3-chloro-4-fluoro-benzylamino)-4-(1,2,2-trimethyl-propylamino)-cyclobut-3-ene-1,2-dione or a pharmaceutically acceptable salt thereof;
3-(3,4-difluoro-benzylamino)-4-(1,1-dimethyl-propylamino)-cyclobut-3-ene-1,2-dione or a pharmaceutically acceptable salt thereof;
3-tert-butylamino-4-(3,5-difluoro-benzylamino)-cyclobut-3-ene-1,2-dione or a pharmaceutically acceptable salt thereof;
3-tert-butylamino-4-(3-fluoro-5-trifluoromethyl-benzylamino)-cyclobut-3-ene-1,2-dione or a pharmaceutically acceptable salt thereof;
3-(3,5-bis-trifluoromethyl-benzylamino)-4-tert-butylamino-cyclobut-3-ene-1,2-dione or a pharmaceutically acceptable salt thereof;
3-(3,5-difluoro-benzylamino)-4-(1,1-dimethyl-propylamino)-cyclobut-3-ene-1,2-dione or a pharmaceutically acceptable salt thereof;
3-chloro-4-([2-(2-fluoro-1,2-dimethyl-propylamino)-3,4-dioxo-cyclobut-1-enylamino]-methyl}-benzonitrile or a pharmaceutically acceptable salt thereof;
3-chloro-4-{[2-(1,1-dimethyl-propylamino)-3,4-dioxo-cyclobut-1-enylamino]-methyl}-benzonitrile or a pharmaceutically acceptable salt thereof;
N-(2-chloro-4-cyano-benzyl)-N-[2-(1,1-dimethyl-propylamino)-3,4-dioxo-cyclobut-1-enyl)-acetamide or a pharmaceutically acceptable salt thereof;
N-(2-chloro-4-cyano-benzyl)-N-[2-(1,1-dimethyl-propylamino)-3,4-dioxo-cyclobut-1-enyl]-butyramide or a pharmaceutically acceptable salt thereof;
3-(2,4-dimethyl-benzylamino)-4-(1,1-dimethyl-propylamino)-cyclobut-3-ene-1,2-dione or a pharmaceutically acceptable salt thereof;
3-(2,3-dimethyl-benzylamino)-4-(1,1-dimethyl-propylamino)-cyclobut-3-ene-1,2-dione or a pharmaceutically acceptable salt thereof;
3-(1,1-dimethyl-propylamino)-4-(2,4,6-trimethyl-benzylamino)-cyclobut-3-ene-1,2-dione or a pharmaceutically acceptable salt thereof;
3-tert-butylamino-4-(2,5-dichloro-benzylamino)-cyclobut-3-ene-1,2-dione or a pharmaceutically acceptable salt thereof;
3-(2,5-dichloro-benzylamino)-4-(1,1-dimethyl-propylamino)-cyclobut-3-ene-1,2-dione or a pharmaceutically acceptable salt thereof;
3-tert-butylamino-4-(3,4-dimethyl-benzylamino)-cyclobut-3-ene-1,2-dione or a pharmaceutically acceptable salt thereof;
3-(3,4-dimethyl-benzylamino)-4-(1,1-dimethyl-proylamino)-cyclobut-3-ene-1,2-dione or a pharmaceutically acceptable salt thereof;
3-tert-butylamino-4-(3,5-dimethyl-benzylamino)-cyclobut-3-ene-1,2-dione or a pharmaceutically acceptable salt thereof;
3-(3,5-dimethyl-benzylamino)-4-(1,1-dimethyl-propylamino)-cyclobut-3-ene-1,2-dione or a pharmaceutically acceptable salt thereof;
3-(3,5-dichloro-benzylamino)-4-(1,1-dimethyl-propylamino)-cyclobut-3-ene-1,2-dione or a pharmaceutically acceptable salt thereof;
3-tert-butylamino-4-(3,5-dichloro-benzylamino)-cyclobut-3-ene-1,2-dione or a pharmaceutically acceptable salt thereof;
3-tert-butylamino-4-(3-chloro-4-methyl-benzylamino)-cyclobut-3-ene-1,2-dione or a pharmaceutically acceptable salt thereof;
3-(3-chloro-4-methyl-benzylamino)-4-(1,1-dimethyl-propylamino)-cyclobut-3-ene-1,2-dione or a pharmaceutically acceptable salt thereof;
3-chloro-4-{[2-(2-hydroxy-1,1-dimethyl-ethylamino)-3,4-dioxo-cyclobut-1-enylamino]-methyl}-benzonitrile or a pharmaceutically acceptable salt thereof;
3-(2,5-dimethyl-benzylamino)-4-(1,1-dimethyl-propylamino)-cyclobut-3-ene-1,2-dione or a pharmaceutically acceptable salt thereof;
3-tert-butylamino-4-(2,5-dimethyl-benzylamino)-cyclobut-3-ene-1,2-dione or a pharmaceutically acceptable salt thereof;
3-tert-butylamino-4-(3-chloro-2-methyl-benzylamino)-cyclobut-3-ene-1,2-dione or a pharmaceutically acceptable salt thereof;
3-(3-chloro-2-methyl-benzylamino)-4-(1,1-dimethyl-propylamino-cyclobut-3-ene-1,2-dione or a pharmaceutically acceptable salt thereof;
N-(2-tert-butylamino-3,4-dioxo-cyclobut-1-enyl)-N-(2-chloro-4-cyano-benzyl)-acetamide or a pharmaceutically acceptable salt thereof;
3-(2,3-dichloro-benzylamino)-4-(1,1-dimethyl-propylamino)-cyclobut-3-ene-1,2-dione or a pharmaceutically acceptable salt thereof;
3-(t-butylamino)-4-(2,3-dichloro-benzylamino)-cyclobut-3-ene-1,2-dione or a pharmaceutically acceptable salt thereof;
N-(2-tert-butylamino-3,4-dioxo-cyclobut-1-enyl)- N-(2-chloro-4-cyano-benzyl)-butyramide or a pharmaceutically acceptable salt thereof;
3-(4-fluoro-benzylamino)-4-(1,2,2-trimethyl-propylamino)-cyclobut-3-ene-1,2-dione or a pharmaceutically acceptable salt thereof;
3-(4-trifluoromethyl-benzylamino)-4-(1,2,2-trimethyl-propylamino)-cyclobut-3-ene-1,2-dione or a pharmaceutically acceptable salt thereof;
3-tert-butylamino-4-(4-trifluoromethoxy-benzylamino)-cyclobut-3-ene-1,2-dione or a pharmaceutically acceptable salt thereof;
3-tert-butylamino-4-(4-trifluoromethyl-benzylamino)-cyclobut-3-ene-1,2-dione or a pharmaceutically acceptable salt thereof;
3-tert-butylamino-4-(4-fluoro-benzylamino)-cyclobut-3-enc-1,2-dione or a pharmaceutically acceptable salt thereof;
3-(4-trifluoromethoxy-benzylamino)-4-(1,2,2-trimethyl-propylamino)-cyclobut-3-ene-1,2-dione or a pharmaceutically acceptable salt thereof;
3-(1,1-dimethyl-propylamino)-4-(4-fluoro-benzylamino)-cyclobut-3-ene-1,2-dione or a pharmaceutically acceptable salt thereof;
3-tert-butylamino-4-(3-fluorobenzyl-amino)-cyclobut-3-ene-1,2-dione or a pharmaceutically acceptable salt thereof;
3-tert-butylamino-4-(2-fluoro-benzylamino)-cyclobut-3-ene-1,2-dione or a pharmaceutically acceptable salt thereof;
3-tert-butylamino-4-(3-trifluoromethyl-benzylamino)-cyclobut-3-ene-1,2-dione or a pharmaceutically acceptable salt thereof;
3-tert-butylamino-4-(2-trifluoromethyl-benzylamino)-cyclobut-3-ene-1,2-dione or a pharmaceutically acceptable salt thereof;
3-tert-butylamino-4-(3-trifluoromethoxy-benzylamino)-cyclobut-3-ene-1,2-dione or a pharmaceutically acceptable salt thereof;
3-(3-trifluoromethyl-benzylamino)-4-(1,2,2-trimethyl-propylamino) cyclobut-3-ene-1,2-dione or a pharmaceutically acceptable salt thereof;
3-(2-trifluoromethyl-benzylamino)-4-(1,2,2-trimethyl-propylamino) cyclobut-3-ene-1,2-dione or a pharmaceutically acceptable salt thereof;
3-(2-fluoro-benzylamino)-4-(1,2,2-trimethyl-propylamino) cyclobut-3-ene-1,2-dione or a pharmaceutically acceptable salt thereof;
3-(3-trifluoromethoxy-benzylamino)-4-(1,2,2-trimethyl-propylamino)-cyclobut-3-ene-1,2-dione or a pharmaceutically acceptable salt thereof.

20. A pharmaceutical composition of matter comprising a compound of formula (I) as defined in any of claims 1 to 19 or a pharmaceutically acceptable salt thereof and a pharmaceutically acceptable carrier therefor.

21. A method for reducing the adverse effects of smooth muscle contractions which comprises administering, orally or parenterally, to a patient in need thereof, a compound of formula (I) as defined in any of claims 1 to 19 or a pharmaceutically acceptable salt thereof, or a pharmaceutical composition as defined in claim 20.

22. The method according to claim 21 in which the smooth muscle adversely contracting causes urinary incontinence.

23. The method according to claim 21 in which the smooth muscle adversely contracting causes irritable bowel syndrome.

24. A compound as claimed in any of claims 1 to 19 for use as a pharmaceutical or therapeutic substance.

25. A compound as claimed in any of claims 1 to 19 for use in the treatment of diseases or conditions related to smooth muscle contractions

26. A compound according to claim 25 in which the smooth muscle adversely contracting causes urinary incontinence.

27. The method according to claim 25 in which the smooth muscle adversely contracting causes irritable bowel syndrome.

28. A process for the preparation of the compounds of formula I as defined in any of claims 1 to 19 which comprises reacting a compound of formula (II): 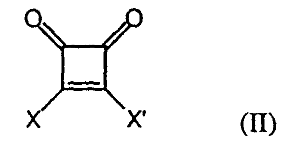 wherein X and X' are leaving groups with a compound of formula (III):
A₁-CH₂NH₂ (III)
wherein A₁ is A as defined hereinbefore or a group of atoms convertible thereto, followed by treatment with a compound of formula (IV): 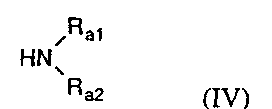 wherein Rₐ₁ and Rₐ₂ are R₁ and R₂, respectively, as defined hereinbefore or a group of atoms convertible thereto in a solvent, and optionally reacting the product with an appropriate anhydride in pyridine with or without protection of the benzyl nitrogen thereby attaching R₂ and/or R₃, and optionally thereafter forming a pharmaceutically acceptable salt thereof.

29. Process according to claim 28 in which the solvent is ethanol, acetonitrile or the appropriate amine (IV)

30. Process according to claim 28 or 29 in which dichloromethane is used as a cosolvent.

31. Process according to any of claims 28 to 30 which comprises reacting a sodium, potassium, or lithium salt of compound of formula (II), where X is methoxy, ethoxy, butoxy, isopropoxy, or similar leaving group and X' is NHR₁, with the appropriate anhydride in dichloromethane, tetrahydrofuran and/or N,N-dimethylformamide or any other suitable solvent, followed by treatment with a compound of formula (III) as defined above in a solvent such as acetonitrile at room temperature.
